(19) **Europäisches Patentamt European Patent Office Office européen des brevets**

(11) **EP 3 615 689 B1**

(12) # EUROPEAN PATENT SPECIFICATION

(45) Date of publication and mention
of the grant of the patent:
**23.02.2022 Bulletin 2022/08**

(21) Application number: **18719856.9**

(22) Date of filing: **23.04.2018**

(51) International Patent Classification (IPC):
***C12Q 1/6883*** *(2018.01)*

(52) Cooperative Patent Classification (CPC):
**C12Q 1/6883;** C12Q 2600/142; C12Q 2600/158

(86) International application number:
**PCT/EP2018/060386**

(87) International publication number:
**WO 2018/197438 (01.11.2018 Gazette 2018/44)**

(54) **ANALYTICAL METHODS AND ARRAYS FOR USE IN THE SAME**

ANALYTISCHE VERFAHREN UND ARRAYS ZUR VERWENDUNG DARIN

MÉTHODES D'ANALYSE ET RÉSEAUX DESTINÉS À ÊTRE UTILISÉS DANS LESDITES
MÉTHODES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.04.2017 GB 201706464**

(43) Date of publication of application:
**04.03.2020 Bulletin 2020/10**

(73) Proprietor: **SenzaGen AB
223 81 Lund (SE)**

(72) Inventors:
• **LINDSTEDT, Malin Marie
24791 Södra Sandby (SE)**
• **JOHANSSON, Henrik
21158 Malmö (SE)**
• **ZELLER WALTHER, Kathrin
22647 Lund (SE)**

(74) Representative: **Potter Clarkson
The Belgrave Centre
Talbot Street
Nottingham NG1 5GG (GB)**

(56) References cited:
**WO-A1-2013/034569**

• **MUSSOTTER FRANZ ET AL: "Proteomics
analysis of dendritic cell activation by contact
allergens reveals possible biomarkers regulated
by Nrf2", TOXICOLOGY AND APPLIED
PHARMACOLOGY, vol. 313, 3 November 2016
(2016-11-03), pages 170-179, XP029821404, ISSN:
0041-008X, DOI: 10.1016/J.TAAP.2016.11.001**
• **MANUEL HITZLER ET AL: "Evaluation of selected
biomarkers for the detection of chemical
sensitization in human skin: A comparative study
applying THP-1, MUTZ-3 and primary dendritic
cells in culture", TOXICOLOGY IN VITRO., vol. 27,
no. 6, 1 September 2013 (2013-09-01), pages
1659-1669, XP055480493, GB ISSN: 0887-2333,
DOI: 10.1016/j.tiv.2013.04.009**
• **ALINE ZIMMER ET AL: "A regulatory dendritic cell
signature correlates with the clinical efficacy of
allergen-specific sublingual immunotherapy",
JOURNAL OF ALLERGY AND CLINICAL
IMMUNOLOGY, ELSEVIER, AMSTERDAM, NL,
vol. 129, no. 4, 15 February 2012 (2012-02-15),
pages 1020-1030, XP028478867, ISSN: 0091-6749,
DOI: 10.1016/J.JACI.2012.02.014 [retrieved on
2012-02-18]**
• **ANDREA OBERSTEINER ET AL:
"Pollen-Associated Microbiome Correlates with
Pollution Parameters and the Allergenicity of
Pollen", PLOS ONE, vol. 11, no. 2, 1 January 2016
(2016-01-01), pages e0149545-e0149545,
XP055302556, US ISSN: 1932-6203, DOI:
10.1371/journal.pone.0149545**

**(Cont. next page)**

EP 3 615 689 B1

- **KRISTINA LUNDBERG ET AL: "Human blood dendritic cell subsets exhibit discriminative pattern recognition receptor profiles", IMMUNOLOGY, vol. 142, no. 2, 1 June 2014 (2014-06-01), pages 279-288, XP055480486, GB ISSN: 0019-2805, DOI: 10.1111/imm.12252**
- **KRISTINA LUNDBERG ET AL: "Transcriptional Profiling of Human Dendritic Cell Populations and Models - Unique Profiles of In Vitro Dendritic Cells and Implications on Functionality and Applicability", PLOS ONE, vol. 8, no. 1, 14 January 2013 (2013-01-14), page e52875, XP055480490, DOI: 10.1371/journal.pone.0052875**

**Description**

**Field of the invention**

The present invention relates to a method for identifying proteins which are allergenic in a mammal, and arrays and analytical kits for use in such methods.

**Background**

Allergy is a chronic disease with increasing prevalence and it is of outmost importance for the industry and authorities to identify potential allergens as early as possible to limit the exposure of workers and the general populations. Several hundreds of chemicals are known to be able to cause allergic contact dermatitis [1, 2], a type IV delayed hypersensitivity reaction, whereas less chemicals are known to sensitize the respiratory tract and to induce type I allergic responses [3]. Most substances causing respiratory allergy are proteins of environmental origin e.g. allergens from house dust mite feces, pollen, or fungi, while others are present in an occupational setting such as enzymes used in flavor, fragrance, detergents and pharmaceutical production [4, 5]. The risk of developing adverse reactions following occupational exposure exists; thus, a strict focus on occupational safety is mandatory. Sensitization has been observed for workers exposed to certain industrial enzymes such as $\alpha$-amylase, proteases, pancreatinin, and trypsin [6, 7]. New enzymes are continuously developed for existing as well as for new applications, such as genetically modified enzymes used in food processing and flavor production and may also lead to occupational health risks [5, 7].

To date, no validated assay is available specifically for predicting the allergenicity of novel proteins, rendering a weight-of-evidence approach to be the most acceptable means of allergy safety assessment. There is, however, a growing consensus that the allergenic potential of compounds, including proteins, should be evaluated with regard to their biochemical characteristics and the protein's potential to induce a specific immune response (European COST Project impARAS [8]). A combination of physical traits of proteins, the molecular interaction between human cells and proteins, as well as their impact on cell-cell interactions play a role in understanding and eventually predicting protein allergenicity [9, 10].

The Genomic Allergen Rapid Detection (GARD) assay was initially developed to provide information about the capacity of chemicals to induce skin sensitization (accuracy: 89 % [13, 17]). This *in vitro* assay utilizes a myeloid cell line resembling dendritic cells (DCs) as a model system. DCs are antigen-presenting cells and central for the induction and regulation of adaptive immune responses [14]. This assay was recognized by both the European Reference Laboratory - European Center for Validation of Alternative Methods (EURL-ECVAM) and the OECD as a valuable method for addressing key event 3 (Dendritic cell activation and maturation) of the AOP for skin sensitization [15]. Forreryd *et al.* [16] successfully demonstrated that a modified protocol of the assay is able to predict respiratory chemical sensitizers with an accuracy of 84 % based on a biomarker signature consisting of 389 transcripts.

Hence, an *in vitro* assay specifically optimised for predicting the allergenicity of novel proteins remains desirable.

**Disclosure of the invention**

The inventors have now shown that a genomic biomarker profile can be developed using the GARD platform for the allergenic assessment specifically of proteins.

Accordingly, a first aspect of the invention provides provides a method for identifying proteins which are allergenic in a mammal comprising or consisting of the steps of:

(a) providing a population of dendritic cells or a population of dendritic-like cells;

(b) exposing the cells provided in step (a) to a test protein; and

(c) measuring in the cells of step (b) the expression of 23 or more biomarkers selected from the group defined in Table A;

wherein the expression of the 23 or more biomarkers measured in step (c) is indicative of the allergenicity of the test protein of step (b); and

wherein the 23 or more biomarkers selected from the group defined in Table A include the biomarkers defined by the following Probe Set ID numbers in Table A: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308.

In an additional or alternative embodiment three or more of the biomarkers measured in step (c) are selected from the group defined in Table A(i).

In an additional or alternative embodiment step (c) comprises measuring the expression of three or more biomarker listed in Table A(i), for example, 4, 5, 6, 7, 8, 9, 10, 11, 12, or 13 of the biomarkers listed in Table A(i). For example, step (c) may comprise measuring the expression of all of the biomarkers listed in Table A(i).

The method includes measuring the expression of TRIML2. The method includes measuring the expression of CYP1A2. The method may include or exclude measuring the expression of MAP9. The method may include or exclude measuring the expression of LOC100131971. The method may include or exclude measuring the expression of Transcript ID GENSCAN00000048751 /// ENST00000354794. The method may include or exclude measuring the expression of GRP. The method may include or exclude measuring the expression of Transcript ID GENSCAN00000015233 /// ENST00000358162. The method may include or exclude measuring the expression of MOBKL1B. The method may include or exclude measuring the expression of Transcript ID ENST00000411383 /// ENST00000386420. The method may include or exclude measuring the expression of BNC2. The method may include or exclude measuring the expression of SFTPA1 /// SFTPA1 B /// SFTPA2 /// SFTPA2B (Probe Set ID 7934708). The method may include or exclude measuring the expression of C21orf118. The method may include or exclude measuring the expression of Transcript ID ENST00000365169.

In an additional or alternative embodiment step (c) comprises measuring the expression of 22 or more biomarkers listed in Table A(ii), for example, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, or 378 of the biomarkers listed in Table A(ii). For example, step (c) may comprise measuring the expression of all of the biomarkers listed in Table A(ii).

The method may include or exclude measuring the expression of SFTPA1 /// SFTPA1B /// SFTPA2 /// SFTPA2B (Probe Set ID 7934698). The method may include or exclude measuring the expression of RPE65. The method may include or exclude measuring the expression of FAM167A. The method includes measuring the expression of Transcript ID ENST00000387309. The method may include or exclude measuring the expression of FLJ44313. The method may include or exclude measuring the expression of NTN4. The method may include or exclude measuring the expression of STAT4. The method may include or exclude measuring the expression of SLC45A2. The method may include or exclude measuring the expression of LOC100133036 /// FAM95B1 (Probe Set ID 8161381). The method may include or exclude measuring the expression of GLT6D1. The method may include or exclude measuring the expression of Transcript ID AF119888. The method may include or exclude measuring the expression of SYCP2L. The method may include or exclude measuring the expression of KLK3. The method may include or exclude measuring the expression of Transcript ID GENSCAN00000042517. The method may include or exclude measuring the expression of RP11-191L9.1. The method may include or exclude measuring the expression of SLC17A8. The method includes measuring the expression of ST8SIA2. The method may include or exclude measuring the expression of Transcript ID ENST00000319817. The method may include or exclude measuring the expression of Transcript ID ENST00000387801 /// ENST00000387652 /// ENST00000387676 /// ENST00000387734 /// ENST00000386042. The method may include or exclude measuring the expression of Transcript ID ENST00000385544. The method may include or exclude measuring the expression of IRX6. The method may include or exclude measuring the expression of LOC100133036 /// FAM95B1 (Probe Set ID 8155627). The method may include or exclude measuring the expression of Transcript ID GENSCAN00000024384 /// ENST00000364863. The method may include or exclude measuring the expression of Transcript ID ENST00000362375. The method may include or exclude measuring the expression of C12orf36. The method may include or exclude measuring the expression of RPRM. The method may include or exclude measuring the expression of OR10AD1. The method includes measuring the expression of Transcript ID ENST00000326734 /// BC118644. The method may include or exclude measuring the expression of Transcript ID ENST00000411186. The method may include or exclude measuring the expression of Transcript ID ENST00000387641. The method may include or exclude measuring the expression of LRRC55. The method may include or exclude measuring the expression of FERMT2. The method may include or exclude measuring the expression of LOC100130815.

The method may include or exclude measuring the expression of RTP2. The method may include or exclude measuring the expression of PLCZ1. The method may include or exclude measuring the expression of FLJ25328. The method may include or exclude measuring the expression of Transcript ID ENST00000411400 /// ENST00000385589. The method may include or exclude measuring the expression of Transcript ID GENSCAN00000027599 /// ENST00000286193. The method may include or exclude measuring the expression of Transcript ID GENSCAN00000026551 /// ENST00000329491. The method may include or exclude measuring the expression of CYP2A13 /// CYP2A7 /// CYP2A6. The method may include or exclude measuring the expression of PRY /// PRY2 (Probe Set ID 8176935). The method may include or exclude measuring the expression of GPR45. The method may include or exclude measuring the expression of Probe Set ID 8070930. The method may include or exclude measuring the expression of Transcript ID AK095738. The method may include or exclude measuring the expression of Transcript ID ENST00000377462. The method may include or exclude measuring the expression of CITED1. The method may include or exclude measuring the expression of Transcript ID ENST00000385536. The method may include or exclude measuring the expression of PXK. The method may include or exclude measuring the expression of Transcript ID ENST00000365399. The method may include or exclude measuring the expression of Transcript ID ENST00000387878. The method may include or exclude measuring the expression of MGAT5B. The method may include or exclude measuring the expression of RP11-35F15.2. The method may include or exclude measuring the expression of MAP1LC3B. The method may include or exclude measuring the expression of PRY /// PRY2 (Probe Set ID 8177395). The method includes measuring the expression of CYP2C19. The method may include or exclude measuring the expression of LOC646187. The method may include or exclude measuring the expression of LOC158381. The method may include or exclude measuring the expression of TERF1. The method may include or exclude measuring the expression of WBP5. The method may include or exclude measuring the expression of Transcript ID ENST00000340456 /// AK128036. The method may include or exclude measuring the expression of LOC153328. The method may include or exclude measuring the expression of RAB10. The method may include or exclude measuring the expression of DIO3OS. The method may include or exclude measuring the expression of PDE11A. The method may include or exclude measuring the expression of EGR1. The method may include or exclude measuring the expression of C9orf7. The method may include or exclude measuring the expression of Transcript ID ENST00000365097. The method may include or exclude measuring the expression of CHAC1. The method may include or exclude measuring the expression of Transcript ID ENST00000384640. The method may include or exclude measuring the expression of DAOA. The method may include or exclude measuring the expression of Transcript ID ENST00000356058 /// AK128129. The method may include or exclude measuring the expression of IFNA7 /// IFNA14. The method may include or exclude measuring the expression of POM121L1 /// DKFZp434K191 /// DKFZP434P211 (Probe Set ID 8071168). The method may include or exclude measuring the expression of Transcript ID AF304443. The method may include or exclude measuring the expression of Transcript ID ENST00000364415. The method may include or exclude measuring the expression of PSD. The method may include or exclude measuring the expression of IQCF2. The method includes measuring the expression of OR52A4. The method may include or exclude measuring the expression of FOS. The method includes measuring the expression of MSTP9 /// MST1. The method may include or exclude measuring the expression of MAF. The method may include or exclude measuring the expression of Transcript ID ENST00000388431 /// ENST00000388445 (Probe Set ID 7943954). The method may include or exclude measuring the expression of EMID2. The method may include or exclude measuring the expression of MDGA2. The method may include or exclude measuring the expression of Transcript ID hsa-mir-15a /// hsa-mir-15a. The method may include or exclude measuring the expression of LOC93432. The method may include or exclude measuring the expression of NPC1L1. The method may include or exclude measuring the expression of NR4A2. The method may include or exclude measuring the expression of Transcript ID BC008359. The method may include or exclude measuring the expression of OPN5. The method includes measuring the expression of Transcript ID ENST00000385543. The method may include or exclude measuring the expression of Transcript ID ENST00000385921 /// ENST00000410743. The method may include or exclude measuring the expression of AADACL2. The method may include or exclude measuring the expression of C12orf54. The method may include or exclude measuring the expression of Transcript ID ENST00000387268. The method may include or exclude measuring the expression of Transcript ID ENST00000364509. The method includes measuring the expression of PRY /// PRY2 (Probe Set ID 8176806). The method may include or exclude measuring the expression of WBP11P1. The method may include or exclude measuring the expression of SPRED3. The method may include or exclude measuring the expression of MAPT. The method may include or exclude measuring the expression of Probe Set ID 8058145. The method may include or exclude measuring the expression of Transcript ID ENST00000410136. The method may include or exclude measuring the expression of PGR. The method may include or exclude measuring the expression of SLC26A5. The method may include or exclude measuring the expression of LOC642538 /// LOC642521 (Probe Set ID 7934731). The method may include or exclude measuring the expression of CRP. The method may include or exclude measuring the expression of WDR38. The method includes measuring the expression of S100A5. The method may include or exclude measuring the expression of Transcript ID ENST00000411154 /// ENST00000387157. The method may include or exclude measuring the expression of CSN1S1. The method may include or exclude measuring the expression of Transcript ID ENST00000384294.

The method includes measuring the expression of AP3S1. The method may include or exclude measuring the expression of ENPP5. The method may include or exclude measuring the expression of FXYD7. The method may include or exclude measuring the expression of CADPS. The method may include or exclude measuring the expression of RNF38. The method may include or exclude measuring the expression of Transcript ID ENST00000404200 /// ENST00000401594 /// ENST00000366307. The method may include or exclude measuring the expression of ASB16. The method may include or exclude measuring the expression of AVPR1A. The method may include or exclude measuring the expression of Transcript ID ENST00000387477. The method may include or exclude measuring the expression of CBLL1. The method may include or exclude measuring the expression of C15orf51. The method may include or exclude measuring the expression of FOSB. The method may include or exclude measuring the expression of Transcript ID ENST00000384559. The method may include or exclude measuring the expression of LOC642538 /// LOC642521 (Probe Set ID 7934729). The method may include or exclude measuring the expression of C10orf90. The method includes measuring the expression of BCAN. The method may include or exclude measuring the expression of PPBPL2. The method may include or exclude measuring the expression of Transcript ID GENSCAN00000020848 /// ENST00000409669 /// ENST00000410082 /// ENST00000409686. The method may include or exclude measuring the expression of IL1F10. The method may include or exclude measuring the expression of C1D (Probe Set ID 7932964). The method may include or exclude measuring the expression of PRAMEF7 /// PRAMEF8 (Probe Set ID 7912606). The method may include or exclude measuring the expression of Transcript ID ENST00000387283. The method may include or exclude measuring the expression of FKBP9L. The method may include or exclude measuring the expression of LOC728264. The method may include or exclude measuring the expression of HAPLN2. The method may include or exclude measuring the expression of PRAMEF7 /// PRAMEF8 (Probe Set ID 7912591). The method may include or exclude measuring the expression of UNQ9370. The method may include or exclude measuring the expression of MAP1LC3C. The method includes measuring the expression of PRAMEF7 /// PRAMEF8 (Probe Set ID 7897991). The method may include or exclude measuring the expression of SLC15A1. The method may include or exclude measuring the expression of Transcript ID ENST00000388545. The method includes measuring the expression of C10orf110. The method may include or exclude measuring the expression of Transcript ID hsa-mir-375 /// hsa-mir-375. The method may include or exclude measuring the expression of GP1BB. The method may include or exclude measuring the expression of LOC100129581. The method may include or exclude measuring the expression of BRS3. The method may include or exclude measuring the expression of CCDC63. The method may include or exclude measuring the expression of ONECUT2. The method may include or exclude measuring the expression of Transcript ID ENST00000387825. The method may include or exclude measuring the expression of Transcript ID ENST00000364793. The method may include or exclude measuring the expression of TBX18. The method may include or exclude measuring the expression of DKFZP686I15217. The method may include or exclude measuring the expression of C9orf98. The method may include or exclude measuring the expression of MYH1. The method may include or exclude measuring the expression of CASQ1. The method may include or exclude measuring the expression of DUSP1. The method may include or exclude measuring the expression of Transcript ID AK125575. The method may include or exclude measuring the expression of ZNF781. The method may include or exclude measuring the expression of Transcript ID ENST00000354690. The method may include or exclude measuring the expression of Transcript ID ENST00000363355. The method may include or exclude measuring the expression of C1D (Probe Set ID 8052698). The method may include or exclude measuring the expression of Transcript ID ENST00000388656. The method may include or exclude measuring the expression of LOH3CR2A. The method may include or exclude measuring the expression of MTNR1A. The method may include or exclude measuring the expression of Transcript ID ENST00000388431 /// ENST00000388445 (Probe Set ID 7951701). The method may include or exclude measuring the expression of TMEM38B. The method may include or exclude measuring the expression of ENST00000387816. The method may include or exclude measuring the expression of UROC1. The method may include or exclude measuring the expression of Transcript ID ENST00000363309. The method may include or exclude measuring the expression of Transcript ID ENST00000316807. The method may include or exclude measuring the expression of C13orf1. The method may include or exclude measuring the expression of UGCG. The method may include or exclude measuring the expression of POM121L1 /// DKFZp434K191 /// DKFZP434P211 (Probe Set ID 8074714). The method may include or exclude measuring the expression of FLJ38773. The method may include or exclude measuring the expression of LRRFIP1. The method may include or exclude measuring the expression of FCRL6. The method may include or exclude measuring the expression of FLJ38723. The method may include or exclude measuring the expression of HSP90AA6P. The method may include or exclude measuring the expression of CALR3. The method may include or exclude measuring the expression of ST18. The method may include or exclude measuring the expression of Transcript ID GENSCAN00000025928 /// ENST00000312946 /// ENST00000402897. The method may include or exclude measuring the expression of PTGS2. The method may include or exclude measuring the expression of NICN1 /// AMT. The method may include or exclude measuring the expression of TTC28. The method may include or exclude measuring the expression of MCL1. The method may include or exclude measuring the expression of SULT1A1. The method may include or exclude measuring the expression of Transcript ID ENST00000386719 /// ENST00000410999. The method may include or exclude measuring the expression of IGLL3. The method may include or exclude measuring the expression of FAM98B. The method may include or exclude measuring the expression of SLC26A4. The method may include or exclude measuring the expression of Probe Set ID 8180281. The method may include or exclude measuring the expression of PDE12. The method may include or exclude measuring the expression of SLC1A1. The method may include or exclude measuring the expression of PLSCR4. The method may include or exclude measuring the expression of TPT1. The method may include or exclude measuring the expression of SNRPN /// SNORD116-25. The method includes measuring the expression of Transcript ID BC068044. The method may include or exclude measuring the expression of C1orf127. The method may include or exclude measuring the expression of FLJ11827. The method may include or exclude measuring the expression of CD2. The method may include or exclude measuring the expression of TMSB10. The method may include or exclude measuring the expression of PRY /// PRY2 (Probe Set ID 8177323). The method may include or exclude measuring the expression of DPM3. The method may include or exclude measuring the expression of LOC442132. The method may include or exclude measuring the expression of NAALAD2. The method includes measuring the expression of ANO5. The method may include or exclude measuring the expression of GPR160. The method may include or exclude measuring the expression of SCN2A. The method may include or exclude measuring the expression of Transcript ID ENST00000364421. The method may include or exclude measuring the expression of TSPYL1. The method may include or exclude measuring the expression of DR1. The method includes measuring the expression of Transcript ID ENST00000410179. The method may include or exclude measuring the expression of ELOVL5. The method may include or exclude measuring the expression of FAM127C. The method may include or exclude measuring the expression of TNFSF14. The method may include or exclude measuring the expression of FZD8. The method may include or exclude measuring the expression of ATPAF1. The method may include or exclude measuring the expression of Transcript ID ENST00000388700 /// ENST00000411294. The method may include or exclude measuring the expression of LOC100132357 (Probe Set ID 8161437). The method may include or exclude measuring the expression of C17orf82. The method may include or exclude measuring the expression of ACER2. The method may include or exclude measuring the expression of LOC100132357 (Probe Set ID 8161426). The method may include or exclude measuring the expression of C3orf58. The method may include or exclude measuring the expression of CBLN1. The method may include or exclude measuring the expression of PNPLA7. The method may include or exclude measuring the expression of ATP7B. The method may include or exclude measuring the expression of NCCRP1. The method may include or exclude measuring the expression of LOC100132357 (Probe Set ID 8155569). The method may include or exclude measuring the expression of ZNF835. The method may include or exclude measuring the expression of Transcript ID ENST00000322493. The method may include or exclude measuring the expression of Transcript ID ENST00000384168. The method may include or exclude measuring the expression of FOXJ3. The method may include or exclude measuring the expression of IKZF3. The method may include or exclude measuring the expression of Transcript ID ENST00000386866. The method may include or exclude measuring the expression of GABRR1. The method may include or exclude measuring the expression of MED31. The method may include or exclude measuring the expression of LRRC32. The method may include or exclude measuring the expression of MFSD6L. The method may include or exclude measuring the expression of CYP19A1. The method may include or exclude measuring the expression of ZNF565. The method may include or exclude measuring the expression of CSNK2A1P /// CSNK2A1. The method may include or exclude measuring the expression of DNAH11. The method may include or exclude measuring the expression of Transcript ID ENST00000365299. The method may include or exclude measuring the expression of Probe Set ID 8180232. The method may include or exclude measuring the expression of OSMR. The method may include or exclude measuring the expression of Transcript ID ENST00000391137. The method may include or exclude measuring the expression of SUMO4. The method may include or exclude measuring the expression of SCGB1D1. The method may include or exclude measuring the expression of RPL39L. The method may include or exclude measuring the expression of Transcript ID ENST00000363408. The method may include or exclude measuring the expression of Transcript ID ENST00000384108. The method may include or exclude measuring the expression of MESP2. The method may include or exclude measuring the expression of EHF. The method may include or exclude measuring the expression of ERO1L. The method may include or exclude measuring the expression of EEF1E1. The method may include or exclude measuring the expression of SLC7A3. The method may include or exclude measuring the expression of SPATA19. The method may include or exclude measuring the expression of NCR1. The method may include or exclude measuring the expression of KLK4. The method may include or exclude measuring the expression of KLHDC7B. The method may include or exclude measuring the expression of MBTPS2. The method may include or exclude measuring the expression of PAH. The method may include or exclude measuring the expression of C4orf27. The method may include or exclude measuring the expression of HUS1. The method may include or exclude measuring the expression of DNAH9. The method may include or exclude measuring the expression of FLJ27255. The method may include or exclude measuring the expression of TMEM33. The method may include or exclude measuring the expression of SGCZ. The method may include or exclude measuring the expression of HLA-DQB2. The method includes measuring the expression of KRT24. The method may include or exclude measuring the expression of GTSF1L. The method may include or exclude measuring the expression of NETO2. The method may include or exclude measuring the expression of TTTY9A /// TTTY9B (Probe Set ID 8177195). The method may include or exclude measuring the expression of MIR21.

The method may include or exclude measuring the expression of TTTY9A /// TTTY9B (Probe Set ID 8176692). The method may include or exclude measuring the expression of EVX2. The method may include or exclude measuring the expression of Transcript ID ENST00000363948. The method may include or exclude measuring the expression of TBXAS1. The method may include or exclude measuring the expression of ADAM12. The method may include or exclude measuring the expression of CD7. The method may include or exclude measuring the expression of ATXN10. The method may include or exclude measuring the expression of ZNF826. The method may include or exclude measuring the expression of SLC35F2. The method may include or exclude measuring the expression of FGF1. The method may include or exclude measuring the expression of IL8. The method may include or exclude measuring the expression of Transcript ID ENST00000364677. The method may include or exclude measuring the expression of CLSTN2. The method may include or exclude measuring the expression of FLJ00049. The method may include or exclude measuring the expression of RCOR1. The method may include or exclude measuring the expression of VPS37A. The method may include or exclude measuring the expression of Transcript ID hsa-mir-221 /// hsa-mir-221. The method may include or exclude measuring the expression of Probe Set ID 8091186. The method may include or exclude measuring the expression of Transcript ID ENST00000386767. The method may include or exclude measuring the expression of UBE2K. The method may include or exclude measuring the expression of MYH15. The method may include or exclude measuring the expression of DUSP22. The method may include or exclude measuring the expression of HIAT1. The method may include or exclude measuring the expression of KLHL33. The method may include or exclude measuring the expression of Transcript ID ENST00000411322. The method may include or exclude measuring the expression of C7orf29. The method may include or exclude measuring the expression of Transcript ID ENST00000387115 /// ENST00000408541. The method includes measuring the expression of Transcript ID ENST00000386002. The method may include or exclude measuring the expression of Transcript ID hsa-mir-33a /// hsa-mir-33a. The method may include or exclude measuring the expression of PTPLB. The method may include or exclude measuring the expression of TANC1. The method may include or exclude measuring the expression of DACT1. The method may include or exclude measuring the expression of PTPRD. The method may include or exclude measuring the expression of CDC26. The method may include or exclude measuring the expression of TSPYL3. The method may include or exclude measuring the expression of RXFP3. The method may include or exclude measuring the expression of Transcript ID ENST00000410526 /// ENST00000386460. The method may include or exclude measuring the expression of FLJ13224. The method may include or exclude measuring the expression of TAF1D /// SNORA40. The method may include or exclude measuring the expression of HPRT1. The method may include or exclude measuring the expression of C10orf10. The method includes measuring the expression of Transcript ID ENST00000364910. The method may include or exclude measuring the expression of MGC16121. The method may include or exclude measuring the expression of Probe Set ID 8180344. The method may include or exclude measuring the expression of Transcript ID ENST00000387217. The method may include or exclude measuring the expression of GDA. The method may include or exclude measuring the expression of SNORD63. The method may include or exclude measuring the expression of YWHAG. The method may include or exclude measuring the expression of SNRPN /// SNORD116-26. The method may include or exclude measuring the expression of ASNS. The method may include or exclude measuring the expression of Transcript ID ENST00000385636. The method may include or exclude measuring the expression of Transcript ID ENST00000389074. The method may include or exclude measuring the expression of Transcript ID ENST00000363891. The method may include or exclude measuring the expression of KLHL11. The method may include or exclude measuring the expression of KCNK6. The method may include or exclude measuring the expression of SVIP. The method may include or exclude measuring the expression of KLRA1. The method may include or exclude measuring the expression of CPSF6. The method may include or exclude measuring the expression of Transcript ID ENST00000384449. The method may include or exclude measuring the expression of Transcript ID ENST00000389758 /// ENST00000396517 /// ENST00000327506. The method may include or exclude measuring the expression of Transcript ID GENSCAN00000041083 /// ENST00000309074. The method may include or exclude measuring the expression of POGK. The method may include or exclude measuring the expression of TRPM6. The method may include or exclude measuring the expression of C9orf6. The method may include or exclude measuring the expression of Transcript ID ENST00000411285 /// ENST00000388598. The method may include or exclude measuring the expression of RAB2A. The method may include or exclude measuring the expression of NAV3. The method may include or exclude measuring the expression of Probe Set ID 8091118. The method may include or exclude measuring the expression of SLC22A4. The method may include or exclude measuring the expression of NAP1L5. The method may include or exclude measuring the expression of Transcript ID ENST00000363618. The method may include or exclude measuring the expression of TGFBRAP1. The method may include or exclude measuring the expression of RPL27A. The method may include or exclude measuring the expression of TP53TG3 /// LOC729355. The method may include or exclude measuring the expression of JSRP1. The method may include or exclude measuring the expression of Transcript ID ENST00000411174 /// ENST00000388411. The method may include or exclude measuring the expression of CCNA2. The method includes measuring the expression of AADACL3. The method may include or exclude measuring the expression of Transcript ID ENST00000363794. The method may include or exclude measuring the expression of TMEM184C. The method may include or exclude measuring the expression of POLR3G. The method may include or exclude measuring the expression of CLDN9 /// LOC100134406. The method may include or exclude measuring the expression of LRRC58. The method may include or exclude measuring the expression of Transcript ID GENSCAN00000001581. The method may include or exclude measuring the expression of Transcript ID hsa-mir-34b /// hsa-mir-34b. The method may include or exclude measuring the expression of OR10K2. The method may include or exclude measuring the expression of Transcript ID GENSCAN00000048378 /// ENST00000404638. The method may include or exclude measuring the expression of KRAS. The method may include or exclude measuring the expression of ORC5L. The method may include or exclude measuring the expression of MYO15B. The method may include or exclude measuring the expression of TNFRSF10D. The method may include or exclude measuring the expression of NIPA2. The method may include or exclude measuring the expression of Transcript ID ENST00000386231 /// ENST00000411190. The method may include or exclude measuring the expression of C17orf88. The method may include or exclude measuring the expression of PPP1 R15B. The method may include or exclude measuring the expression of CSNK2A2. The method may include or exclude measuring the expression of LOC100133036 /// FAM95B1 (Probe Set ID 8155418). The method may include or exclude measuring the expression of MPZL2. The method may include or exclude measuring the expression of LOC100134722 /// LOC100133005. The method may include or exclude measuring the expression of IER3IP1. The method may include or exclude measuring the expression of CCDC117. The method includes measuring the expression of DDIT4. The method may include or exclude measuring the expression of SEC24A.

In an additional or alternative embodiment step (c) comprises or consists of measuring the expression of 24 or more of the biomarkers listed in Table A, for example, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 of the biomarkers listed in Table A. For example, step (c) may comprise or consist of measuring the expression of all of the biomarkers listed in Table A.

By "expression" we mean the presence, level and/or amount of the biomarker.

By "biomarker" we include any biological molecule, or component or fragment thereof, the measurement of which can provide information useful in determining the allergenicity of a protein. Thus, in the context of Table A, the biomarker may be a nucleic acid molecule, such as a mRNA or cDNA. Alternatively, the biomarker may be a protein encoded by the nucleic acid molecule or carbohydrate moiety, or an antigenic component or fragment thereof.

In an additional or alternative embodiment the method comprises the further steps of:

d) exposing a separate population of the dendritic cells or dendritic-like cells to one or more negative control agent that is not allergenic in a mammal; and

e) measuring in the cells of step (d) the expression of the 23 or more biomarkers measured in step (c)

wherein the test protein is identified as allergenic in the event that the expression of the 23 or more biomarkers measured in step (e) differs from the expression of the 23 or more biomarkers measured in step (c).

A vehicle control may be used as the negative control agent. The vehicle control may comprise DMSO.

In an additional or alternative embodiment unstimulated cells may be used as the negative control. By "unstimulated cells" we include or mean cells which have not been exposed to a specific test protein. In other words, the separate population of cells in step (d) is not exposed to a test protein. However, the separate population of cells may be exposed to cell media containing serum (e.g. at about 20% by volume) which comprises negative control proteins.

In an additional or alternative embodiment the expression of the 23 or more biomarkers measured in step (c) is measured in the cells provided in step (a) prior to and following exposure to the test protein, and wherein the difference in expression between the 23 or more biomarkers prior to and following exposure to the test protein is indicative of the allergenicity of the test protein of step (b). Hence, the cells provided in step (a) may provide both the negative control and the test result.

By "differs from the expression of the 23 or more biomarkers measured in step (c)" and "difference in expression" we include that the presence and or amount in a first sample (e.g., a test protein sample) differs from that of a second sample (e.g., a control agent sample).

For example, the presence and/or amount in the test sample may differ from that of the one or more negative control sample in a statistically significant manner. Preferably the expression of the 23 or more biomarkers in the cell population exposed to the test protein is:

less than or equal to 80% of that of the cell population exposed to the negative control agent, for example, no more than 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% or 0% of that of the cell population exposed to the negative control or negative control agent; or

at least 120% of that of the cell population exposed to the negative control agent, for example, at least 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%, 151%, 152%, 153%, 154%, 155%, 156%, 157%, 158%, 159%, 160%, 161%, 162%, 163%, 164%, 165%, 166%, 167%, 168%, 169%, 170%, 171%, 172%, 173%, 174%, 175%, 176%, 177%, 178%, 179%, 180%, 181%, 182%, 183%, 184%, 185%, 186%, 187%, 188%, 189%, 190%, 191%, 192%, 193%, 194%, 195%, 196%, 197%, 198%, 199%, 200%, 225%, 250%, 275%, 300%, 325%, 350%, 375%, 400%, 425%, 450%, 475% or at least 500% of that of the cell population exposed to the negative control or negative control agent

By "differs from the expression of the 23 or more biomarkers measured in step (c)" we alternatively or additionally include that the test sample is classified as belonging to a different group as the one or more negative control sample. For example, where an SVM is used, the test sample is on the other side of the decision value threshold as the one or more negative control sample (e.g., if the test agent is classified as a protein allergen if one or more test (or replicate thereof) has an SVM decision value of ≤0, then the one or more positive control samples (or the majority thereof) should also have an SVM decision value of ≤0).

In an additional or alternative embodiment, the one or more negative control agent provided in step (d) is selected from the group consisting of: unstimulated cells; cell media; vehicle control; DMSO; LPS.

In an additional or alternative embodiment, the one or more negative control agent provided in step (d) is cell media. Preferably the media contains serum (preferably at about 20% by volume) which comprises negative control proteins.

In an additional or alternative embodiment, at least 2 negative controls and/or control non-allergenic agents are provided, for example, at least 3, 4, or at least 5 negative controls and/or control non-allergenic agents.

In an additional or alternative embodiment the method comprises the further steps of:

f) exposing a separate population of the dendritic cells or dendritic-like cells to one or more positive control agent that is allergenic in a mammal; and
g) measuring in the cells of step (f) the expression of the 23 or more biomarkers measured in step (c)

wherein the test protein is identified as allergenic in the event that the expression of the 23 or more biomarkers measured in step (f) corresponds to the expression of the 23 or more biomarkers measured in step (c).

By "corresponds to the expression of the 23 or more biomarkers measured in step (c)" we mean the expression of the 23 or more biomarkers in the cell population exposed to the test protein is identical to, or does not differ significantly from, that of the cell population exposed to the one more positive control agent. Preferably the expression of the 23 or more biomarkers in the cell population exposed to the test protein is between 81% and 119% of that of the cell population exposed to the one more positive control agent, for example, greater than or equal to 82%, 83%, 84%, 85%, 86%, 87%, 88%, 89%, 90%, 91%, 92%, 93%, 94%, 95%, 96%, 97%, 98% or 99% of that of the cell population exposed to the one more positive control agent, and less than or equal to 101%, 102%, 103%, 104%, 105%, 106%, 107%, 108%, 109%, 110%, 111%, 112%, 113%, 114%, 115%, 116%, 117%, 118% or 119% of that of the cell population exposed to the one more positive control agent.

By "corresponds to the expression of the 23 or more biomarkers measured in step (c)" we alternatively or additionally include that the test sample is classified as belonging to the same group as the one or more positive control sample. For example, where an SVM is used, the test sample is on the same side of the decision value threshold as the one or more positive control sample (e.g., if the test protein is classified as allergenic if one or more test (or replicate thereof) has an SVM decision value of >0, then the one or more positive control samples (or the majority thereof) should also have an SVM decision value of >0).

In an additional or alternative embodiment, the one or more positive control agent provided in step (f) comprises or consists of one or more agent selected from the group consisting of: Der p 1; and Der p 7.

In an additional or alternative embodiment, at least 2 control allergenic agents are provided.

In an additional or alternative embodiment, the control allergenic agents are allergenic proteins. In an additional or alternative embodiment, the control non-allergenic agents are non-allergenic proteins.

In an additional or alternative embodiment, the method is indicative of the allergenic potency of the protein to be tested. For example, the method may be used to predict the relative allergenic potency of a test protein compared to a positive control and/or compared to one or more additional test protein.

In an additional or alternative embodiment the method comprises the further step of:

(h) identifying the allergenicity of the test protein.

For example, step (h) may identify the test protein as being an allergen or a non-allergen. Alternatively or additionally, step (h) may identify the relative allergenicity or allergenic potency of the test protein compared to a positive control and/or one or more additional test proteins.

The identification may be performed using any suitable statistical method or machine learning algorithm known in the art, such as Random Forest (RF), Support Vector Machine (SVM), Principal Component Analysis (PCA), ordinary least squares (OLS), partial least squares regression (PLS), orthogonal partial least squares regression (O-PLS) and other multivariate statistical analyses (e.g., backward stepwise logistic regression model). For a review of multivariate statistical analysis see, for example, Schervish, Mark J. (November 1987). "A Review of Multivariate Analysis". Statistical Science 2 (4): 396-413. Preferably, Support Vector Machine (SVM) is used.

Typically, allergenic proteins are identified using a support vector machine (SVM), such as those available from http://cran.r-project.org/web/packages/e1071/index.html (e.g. e1071 1.5-24). However, any other suitable means may also be used. SVMs may also be used to determine the ROC AUCs of biomarker signatures comprising or consisting of one or more Table A biomarkers as defined herein.

Support vector machines (SVMs) are a set of related supervised learning methods used for classification and regression. Given a set of training examples, each marked as belonging to one of two categories, an SVM training algorithm builds a model that predicts whether a new example falls into one category or the other. Intuitively, an SVM model is a representation of the examples as points in space, mapped so that the examples of the separate categories are divided by a clear gap that is as wide as possible. New examples are then mapped into that same space and predicted to belong to a category based on which side of the gap they fall on.

More formally, a support vector machine constructs a hyperplane or set of hyperplanes in a high or infinite dimensional space, which can be used for classification, regression or other tasks. Intuitively, a good separation is achieved by the hyperplane that has the largest distance to the nearest training datapoints of any class (so-called functional margin), since in general the larger the margin the lower the generalization error of the classifier. For more information on SVMs, see for example, Burges, 1998, Data Mining and Knowledge Discovery, 2:121-167.

In one embodiment of the invention, the SVM is 'trained' prior to performing the methods of the invention using biomarker profiles of known agents (namely, known allergenic or non-allergenic agents). By running such training samples, the SVM is able to learn what biomarker profiles are associated with proteins capable of inducing allergy. Once the training process is complete, the SVM is then able to predict whether or not the biomarker sample tested is from an allergenic or non-allergenic protein.

Decision values for individual SVMs can be determined by the skilled person on a case-by-case basis. In one embodiment, the test protein is classified as allergenic if one or more test (or replicate thereof) have an SVM decision value of >0. In one embodiment, the test protein is classified as a non-allergenic protein if one or more test (or replicate thereof) have an SVM decision value of ≤0. This allows test proteins to be classified as allergenic or non-allergenic.

However, this training procedure can be by-passed by pre-programming the SVM with the necessary training parameters. For example, allergenic proteins can be identified according to the known SVM parameters using the SVM algorithm described in the Examples, based on the measurement of all the biomarkers listed in Table A.

It will be appreciated by skilled persons that suitable SVM parameters can be determined for any combination of the biomarkers listed Table A by training an SVM machine with the appropriate selection of data (i.e. biomarker measurements from cells exposed to known allergenic and/or non-allergenic agents). Alternatively, the Table A biomarkers may be used to identify allergenic proteins according to any other suitable statistical method known in the art.

Alternatively, the Table A data may be used to identify agents capable of inducing respiratory sensitization according to any other suitable statistical method known in the art (e.g., ANOVA, ANCOVA, MANOVA, MANCOVA, Multivariate regression analysis, Principal components analysis (PCA), Factor analysis, Canonical correlation analysis, Canonical correlation analysis, Redundancy analysis Correspondence analysis (CA; reciprocal averaging), Multidimensional scaling, Discriminant analysis, Linear discriminant analysis (LDA), Clustering systems, Recursive partitioning and Artificial neural networks).

Preferably, the methods of the invention have an accuracy of at least 60%, for example, 61%, 62%, 63%, 64%,

65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% accuracy. In a preferred embodiment, the methods of the invention have an accuracy of at least 93%

Preferably, the methods of the invention have a sensitivity of at least 60%, for example, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sensitivity. In a preferred embodiment, the methods of the invention have a sensitivity of at least 92%.

Preferably, the methods of the invention have a specificity of at least 60%, for example, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% specificity. In a preferred embodiment, the methods of the invention have a specificity of 100%.

By "accuracy" we mean the proportion of correct outcomes of a method, by "sensitivity" we mean the proportion of all positive proteins that are correctly classified as positives, and by "specificity" we mean the proportion of all negative proteins that are correctly classified as negatives.

In a preferred embodiment, step (c) comprises measuring the expression of a nucleic acid molecule of one or more of the biomarkers. The nucleic acid molecule may be a DNA molecule or a cDNA molecule or an mRNA molecule. Preferably, the nucleic acid molecule is an mRNA molecule. However, the nucleic acid molecule may be a cDNA molecule.

In one embodiment the measurement of the expression of one or more of the biomarkers in step (c) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation. Preferably, the expression of one or more biomarker(s) is measured using a DNA microarray.

In an additional or alternative embodiment one or more biomarkers measured in step (c) is measured using an array (e.g., a DNA array). In an additional or alternative embodiment one or more biomarkers measured in step (c) is measured using a whole genome array (e.g., the Affymetrix Human Gene 1.0 ST array or Affymetrix Human Gene 2.0 ST array). In an alternative or additional embodiment, the Nanostring nCounter system is used (e.g., custom Nanostring nCounter code sets based on selection from a whole genome array (e.g., Affymetrix Human Gene 1.0 ST array or Affymetrix Human Gene 2.0 ST array).

The method may comprise measuring the expression of one or more biomarkers in step (c) using one or more binding moieties, each capable of binding selectively to a nucleic acid molecule encoding one of the biomarkers identified in Table A. Preferably, the method comprises measuring the expression of two or more biomarkers in step (c) using two or more binding moieties, each capable of binding selectively to a nucleic acid molecule encoding one of the biomarkers identified in Table A. For example, the expression of any particular combination of biomarkers described above may be measured using an equivalent combination of binding moieties capable of binding selectively to each of those biomarkers.

In one embodiment the one or more binding moieties each comprise or consist of a nucleic acid molecule. In a further embodiment the one or more binding moieties each comprise or consist of DNA, RNA, PNA, LNA, GNA, TNA or PMO. Preferably, the one or more binding moieties each comprise or consist of DNA. In one embodiment, the one or more binding moieties are 5 to 100 nucleotides in length. However, in an alternative embodiment, they are 15 to 35 nucleotides in length.

The one or more binding moieties may comprise or consist of one or more probe from the Human Gene 1.0 ST Array (Affymetrix, Santa Clara, CA, USA). Probe identification numbers are provided in Table A herein.

Suitable binding agents (also referred to as binding molecules or binding moieties) may be selected or screened from a library based on their ability to bind a given nucleic acid, protein or amino acid motif, as discussed below.

In a preferred embodiment, the binding moiety comprises a detectable moiety.

By a "detectable moiety" we include a moiety which permits its presence and/or relative amount and/or location (for example, the location on an array) to be determined, either directly or indirectly.

Suitable detectable moieties are well known in the art.

For example, the detectable moiety may be a fluorescent and/or luminescent and/or chemiluminescent moiety which, when exposed to specific conditions, may be detected. Such a fluorescent moiety may need to be exposed to radiation (*i.e.* light) at a specific wavelength and intensity to cause excitation of the fluorescent moiety, thereby enabling it to emit detectable fluorescence at a specific wavelength that may be detected.

Alternatively, the detectable moiety may be an enzyme which is capable of converting a (preferably undetectable) substrate into a detectable product that can be visualised and/or detected. Examples of suitable enzymes are discussed in more detail below in relation to, for example, ELISA assays.

The detectable moiety may be a radioactive moiety and comprise or consists of a radioactive atom. The radioactive atom may be selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90.

Hence, the detectable moiety may be selected from the group consisting of: a fluorescent moiety; a luminescent moiety; a chemiluminescent moiety; a radioactive moiety (for example, a radioactive atom); or an enzymatic moiety.

Clearly, the agent to be detected (such as, for example, the one or more biomarkers in the test sample and/or control sample described herein and/or an antibody molecule for use in detecting a selected protein) must have sufficient of the appropriate atomic isotopes in order for the detectable moiety to be readily detectable.

In an alternative preferred embodiment, the detectable moiety of the binding moiety is a fluorescent moiety.

The radio- or other labels may be incorporated into the biomarkers present in the samples of the methods of the invention and/or the binding moieties of the invention in known ways. For example, if the binding agent is a polypeptide it may be biosynthesised or may be synthesised by chemical amino acid synthesis using suitable amino acid precursors involving, for example, fluorine-19 in place of hydrogen. Labels such as $^{99m}$Tc, $^{123}$I, $^{186}$Rh, $^{188}$Rh and $^{111}$In can, for example, be attached *via* cysteine residues in the binding moiety. Yttrium-90 can be attached via a lysine residue. The IODOGEN method (Fraker et al (1978) Biochem. Biophys. Res. Comm. 80, 49-57) can be used to incorporate $^{123}$I. Reference ("Monoclonal Antibodies in Immunoscintigraphy", J-F Chatal, CRC Press, 1989) describes other methods in detail. Methods for conjugating other detectable moieties (such as enzymatic, fluorescent, luminescent, chemiluminescent or radioactive moieties) to proteins are well known in the art.

It will be appreciated by persons skilled in the art that biomarkers in the sample(s) to be tested may be labelled with a moiety which indirectly assists with determining the presence, amount and/or location of said proteins. Thus, the moiety may constitute one component of a multicomponent detectable moiety. For example, the biomarkers in the sample(s) to be tested may be labelled with biotin, which allows their subsequent detection using streptavidin fused or otherwise joined to a detectable label.

The method provided in the first aspect of the present invention may comprise, in step (c), determining the expression of the protein of one or more biomarker defined in Table A. The method may comprise measuring the expression of one or more biomarkers in step (c) using one or more binding moieties each capable of binding selectively to one of the biomarkers identified in Table A. The one or more binding moieties may comprise or consist of an antibody or an antigen-binding fragment thereof such as a monoclonal antibody or fragment thereof.

The term "antibody" includes any synthetic antibodies, recombinant antibodies or antibody hybrids, such as but not limited to, a single-chain antibody molecule produced by phage-display of immunoglobulin light and/or heavy chain variable and/or constant regions, or other immunointeractive molecules capable of binding to an antigen in an immunoassay format that is known to those skilled in the art.

We also include the use of antibody-like binding agents, such as affibodies and aptamers.

A general review of the techniques involved in the synthesis of antibody fragments which retain their specific binding sites is to be found in Winter & Milstein (1991) Nature 349, 293-299.

Additionally, or alternatively, one or more of the first binding molecules may be an aptamer (see Collett et a/., 2005, Methods 37:4-15).

Molecular libraries such as antibody libraries (Clackson et al, 1991, Nature 352, 624-628; Marks et al, 1991, J Mol Biol 222(3): 581-97), peptide libraries (Smith, 1985, Science 228(4705): 1315-7), expressed cDNA libraries (Santi et al (2000) J Mol Biol 296(2): 497-508), libraries on other scaffolds than the antibody framework such as affibodies (Gunneriusson et al, 1999, Appl Environ Microbiol 65(9): 4134-40) or libraries based on aptamers (Kenan et al, 1999, Methods Mol Biol 118, 217-31) may be used as a source from which binding molecules that are specific for a given motif are selected for use in the methods of the invention.

The molecular libraries may be expressed *in vivo* in prokaryotic cells (Clackson *et al,* 1991, *op. cit.*; Marks *et al,* 1991, *op. cit.*) or eukaryotic cells (Kieke et al, 1999, Proc Natl Acad Sci USA, 96(10):5651-6) or may be expressed *in vitro* without involvement of cells (Hanes & Pluckthun, 1997, Proc Natl Acad Sci USA 94(10):4937-42; He & Taussig, 1997, Nucleic Acids Res 25(24):5132-4; Nemoto et al, 1997, FEBS Lett, 414(2):405-8).

In cases when protein based libraries are used, the genes encoding the libraries of potential binding molecules are often packaged in viruses and the potential binding molecule displayed at the surface of the virus (Clackson *et al,* 1991, *supra;* Marks *et al,* 1991, *supra;* Smith, 1985, *supra*).

Perhaps the most commonly used display system is filamentous bacteriophage displaying antibody fragments at their surfaces, the antibody fragments being expressed as a fusion to the minor coat protein of the bacteriophage (Clackson *et al,* 1991, *supra;* Marks *et al,* 1991, *supra*). However, other suitable systems for display include using other viruses (EP 39578), bacteria (Gunneriusson *et al,* 1999, *supra;* Daugherty et al, 1998, Protein Eng 11 (9):825-32; Daugherty et al, 1999, Protein Eng 12(7):613-21), and yeast (Shusta et al, 1999, J Mol Biol 292(5):949-56).

In addition, display systems have been developed utilising linkage of the polypeptide product to its encoding mRNA in so-called ribosome display systems (Hanes & Pluckthun, 1997, *supra;* He & Taussig, 1997, *supra;* Nemoto *et al,* 1997, *supra*), or alternatively linkage of the polypeptide product to the encoding DNA (see US Patent No. 5,856,090 and WO 98/37186).

The variable heavy ($V_H$) and variable light ($V_L$) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sci. USA 81, 6851-6855) .

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the $V_H$ and $V_L$ partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et a/ (1989) Nature 341, 544). A general review of the techniques involved in the synthesis of antibody fragments which retain their specific binding sites is to be found in Winter & Milstein (1991) Nature 349, 293-299.

The antibody or antigen-binding fragment may be selected from the group consisting of intact antibodies, Fv fragments (e.g. single chain Fv and disulphide-bonded Fv), Fab-like fragments (*e.g.* Fab fragments, Fab' fragments and F(ab)$_2$ fragments), single variable domains (*e.g.* $V_H$ and $V_L$ domains) and domain antibodies (dAbs, including single and dual formats [*i.e.* dAb-linker-dAb]). Preferably, the antibody or antigen-binding fragment is a single chain Fv (scFv).

The one or more binding moieties may alternatively comprise or consist of an antibody-like binding agent, for example an affibody or aptamer.

By "scFv molecules" we mean molecules wherein the $V_H$ and $V_L$ partner domains are linked via a flexible oligopeptide.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from *E. coli,* thus allowing the facile production of large amounts of the said fragments.

Whole antibodies, and F(ab')$_2$ fragments are "bivalent". By "bivalent" we mean that the said antibodies and F(ab')$_2$ fragments have two antigen combining sites. In contrast, Fab, Fv, ScFv and dAb fragments are monovalent, having only one antigen combining sites.

The antibodies may be monoclonal or polyclonal. Suitable monoclonal antibodies may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982).

When potential binding molecules are selected from libraries, one or more selector peptides having defined motifs are usually employed. Amino acid residues that provide structure, decreasing flexibility in the peptide or charged, polar or hydrophobic side chains allowing interaction with the binding molecule may be used in the design of motifs for selector peptides. For example:

(i) Proline may stabilise a peptide structure as its side chain is bound both to the alpha carbon as well as the nitrogen;
(ii) Phenylalanine, tyrosine and tryptophan have aromatic side chains and are highly hydrophobic, whereas leucine and isoleucine have aliphatic side chains and are also hydrophobic;
(iii) Lysine, arginine and histidine have basic side chains and will be positively charged at neutral pH, whereas aspartate and glutamate have acidic side chains and will be negatively charged at neutral pH;
(iv) Asparagine and glutamine are neutral at neutral pH but contain a amide group which may participate in hydrogen bonds;
(v) Serine, threonine and tyrosine side chains contain hydroxyl groups, which may participate in hydrogen bonds.

Typically, selection of binding molecules may involve the use of array technologies and systems to analyse binding to spots corresponding to types of binding molecules.

The one or more protein-binding moieties may comprise a detectable moiety. The detectable moiety may be selected from the group consisting of a fluorescent moiety, a luminescent moiety, a chemiluminescent moiety, a radioactive moiety and an enzymatic moiety.

In a further embodiment of the methods of the invention, step (c) may be performed using an assay comprising a second binding agent capable of binding to the one or more proteins, the second binding agent also comprising a detectable moiety. Suitable second binding agents are described in detail above in relation to the first binding agents.

Thus, the proteins of interest in the sample to be tested may first be isolated and/or immobilised using the first binding agent, after which the presence and/or relative amount of said biomarkers may be determined using a second binding agent.

In one embodiment, the second binding agent is an antibody or antigen-binding fragment thereof; typically a recombinant antibody or fragment thereof. Conveniently, the antibody or fragment thereof is selected from the group consisting of: scFv; Fab; a binding domain of an immunoglobulin molecule. Suitable antibodies and fragments, and methods for making the same, are described in detail above.

Alternatively, the second binding agent may be an antibody-like binding agent, such as an affibody or aptamer.

Alternatively, where the detectable moiety on the protein in the sample to be tested comprises or consists of a member of a specific binding pair (*e.g.* biotin), the second binding agent may comprise or consist of the complimentary member of the specific binding pair (*e.g.* streptavidin).

Where a detection assay is used, it is preferred that the detectable moiety is selected from the group consisting of: a fluorescent moiety; a luminescent moiety; a chemiluminescent moiety; a radioactive moiety; an enzymatic moiety. Examples of suitable detectable moieties for use in the methods of the invention are described above.

Preferred assays for detecting serum or plasma proteins include enzyme linked immunosorbent assays (ELISA), radioimmunoassay (RIA), immunoradiometric assays (IRMA) and immunoenzymatic assays (IEMA), including sandwich assays using monoclonal and/or polyclonal antibodies. Exemplary sandwich assays are described by David et al in US Patent Nos. 4,376,110 and 4,486,530. Antibody staining of cells on slides may be used in methods well known in cytology laboratory diagnostic tests, as well known to those skilled in the art.

Thus, in one embodiment the assay is an ELISA (Enzyme Linked Immunosorbent Assay) which typically involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemiluminescent systems based on enzymes such as luciferase can also be used.

Conjugation with the vitamin biotin is frequently used since this can readily be detected by its reaction with enzyme-linked avidin or streptavidin to which it binds with great specificity and affinity.

In an alternative embodiment, the assay used for protein detection is conveniently a fluorometric assay. Thus, the detectable moiety of the second binding agent may be a fluorescent moiety, such as an *Alexa* fluorophore (for example *Alexa*-647).

Preferably, steps (c), (e), and/or (g) of the methods described in the first aspect are performed using an array. The array may be a bead-based array or a surface-based array. The array may be selected from the group consisting of: macroarray; microarray; nanoarray.

Arrays *per se* are well known in the art. Typically they are formed of a linear or two-dimensional structure having spaced apart (*i.e.* discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution. The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, polynucleotides and other suitable molecules and/or conducting an immunoassay. The binding processes are well known in the art and generally consist of cross-linking covalently binding or physically adsorbing a protein molecule, polynucleotide or the like to the solid support. Alternatively, affinity coupling of the probes via affinity-tags or similar constructs may be employed. By using well-known techniques, such as contact or non-contact printing, masking or photolithography, the location of each spot can be defined. For reviews see Jenkins, R.E., Pennington, S.R. (2001, Proteomics, 2,13-29) and Lal et al (2002, Drug Discov Today 15;7(18 Suppl):S143-9).

Typically the array is a microarray. By "microarray" we include the meaning of an array of regions having a density of discrete regions of at least about 100/cm$^2$, and preferably at least about 1000/cm$^2$. The regions in a microarray have typical dimensions, *e.g.* diameter, in the range of between about 10-250 $\mu$m, and are separated from other regions in the array by about the same distance. The array may alternatively be a macroarray or a nanoarray.

Once suitable binding molecules (discussed above) have been identified and isolated, the skilled person can manufacture an array using methods well known in the art of molecular biology.

In an additional or alternative embodiment one or more biomarkers measured in step (c) comprise or consist of one or more homologous gene product expressed by human cells. In an additional or alternative embodiment one or more biomarkers measured in step (c) is a protein or polypeptide. In an additional or alternative embodiment one or more biomarker measured in step (c) is a nucleic acid (e.g., DNA, mRNA or cDNA etc).

In an additional or alternative embodiment method is performed *in vitro, in vivo, ex vivo* or *in silico.* For example, the method may in particular be performed *in vitro.*

By "test protein" we include any protein or proteinaceous entity (or mixture of proteins or proteinaceous entities) for which allergenic or sensitization status is to be determined.

By "allergenic" we include or mean a protein (or mixture of proteins) which is an allergen, and/or which is capable of inducing an allergic response, in a mammal.

In an additional or alternative embodiment the allergenicity comprises a hypersensitivity response (e.g., a cell-mediated hypersensitivity response). In an additional or alternative embodiment the hypersensitivity response is a type I hypersensitivity response. In an additional or alternative embodiment the hypersensitivity response is respiratory allergy.

In an additional or alternative embodiment, the method is for identifying the sensitization status of a protein in a mammal. For example, the expression of the 23 or more biomarkers measured in step (c) may be indicative of the sensitization status of the test protein.

By "sensitization status" we include or mean whether or not a test protein (or mixture of test proteins) is a sensitizer or not (e.g., a skin sensitizer and/or a respiratory sensitizer).

In an additional or alternative embodiment, the method is for identifying proteins which are capable of inducing respiratory sensitization in a mammal. For example, the expression of the 23 or more biomarkers measured in step (c) may be indicative of the respiratory sensitizing effect of the test protein.

In one embodiment, the method is for identifying proteins capable of inducing a respiratory hypersensitivity response. Preferably, the hypersensitivity response is a humoral hypersensitivity response, for example, a type I hypersensitivity response. In one embodiment, the method is for identifying agents capable of inducing respiratory allergy.

By "indicative of the respiratory sensitizing effect of the test protein" we include determining whether or not the test protein is a respiratory sensitizer and/or determining the potency of the test protein as a respiratory sensitizer.

By proteins "capable of inducing respiratory sensitization" we mean any protein capable of inducing and triggering a Type I immediate hypersensitivity reaction in the respiratory tract of a mammal. Preferably the mammal is a human. Preferably, the Type I immediate hypersensitivity reaction is DC-mediated and/or involves the differentiation of T cells into Th2 cells. Preferably the Type I immediate hypersensitivity reaction results in humoral immunity and/or respiratory allergy.

The conducting zone of the mammalian lung contains the trachea, the bronchi, the bronchioles, and the terminal bronchioles. The respiratory zone contains the respiratory bronchioles, the alveolar ducts, and the alveoli. The conducting zone is made up of airways, has no gas exchange with the blood, and is reinforced with cartilage in order to hold open the airways. The conducting zone humidifies inhaled air and warms it to 37°C (99°F). It also cleanses the air by removing particles via cilia located on the walls of all the passageways. The respiratory zone is the site of gas exchange with blood.

In one embodiment, the protein "capable of inducing respiratory sensitization" is a protein capable of inducing and triggering a Type I immediate hypersensitivity reaction at a site of lung epithelium in a mammal. Preferably, the site of lung epithelium is in the respiratory zone of the lung, but may alternatively or additionally be in the conductive zone of the lung.

In an additional or alternative embodiment, the method is for identifying food proteins which are allergenic in a mammal. For example, the expression of the 23 or more biomarkers measured in step (c) may be indicative of the allergenicity of the food protein. Preferably, the allergenicity of the food protein is due to a Type 1 hypersensitivity response.

The mammal may be any domestic or farm animal. Preferably, the mammal is a rat, mouse, guinea pig, cat, dog, horse or a primate. Most preferably, the mammal is human.

In an additional or alternative embodiment the population of dendritic cells or population of dendritic-like cells comprises or consists of immortal cells. By "immortal" we mean cells that are not limited by a point at which they can no longer continue to divide, which might otherwise be due to DNA damage or shortened telomeres.

In an additional or alternative embodiment the population of dendritic cells or population of dendritic-like cells comprises or consists of non-naturally occurring cells. By "non-naturally occurring" cells, we mean that the cells are different to, modified from, or variants of, those which would be found in nature; in other words, they are not cells which would normally occur in nature.

In an additional or alternative embodiment the population of dendritic cells or population of dendritic-like cells is a population of dendritic-like cells. In an additional or alternative embodiment the dendritic-like cells are myeloid dendritic-like cells. In an additional or alternative embodiment the myeloid dendritic-like cells are derived from myeloid dendritic cells. In an additional or alternative embodiment the cells derived from myeloid dendritic cells are myeloid leukaemia-derived cells. In an additional or alternative embodiment the myeloid leukaemia-derived cells are selected from the group consisting of KG-1, THP-1, U-937, HL-60, Monomac-6, AML-193 and MUTZ-3. In an additional or alternative embodiment the dendritic-like cells are MUTZ-3 cells. MUTZ-3 cells are human acute myelomonocytic leukemia cells that are available from Deutsche Sammlung für Mikroorganismen und Zellkulturen GmbH (DSMZ), Braunschweig, Germany (www.dsmz.de; DMSZ No. ACC 295).

By "dendritic-like cells" we mean non-dendritic cells that exhibit functional and phenotypic characteristics specific to dendritic cells such as morphological characteristics, expression of costimulatory molecules and MHC class II molecules, and the ability to pinocytose macromolecules and to activate resting T cells.

In one embodiment, the dendritic-like cells, after stimulation with cytokine, present antigens through CD1d, MHC class I and II and/or induce specific T-cell proliferation.

In one embodiment, the dendritic-like cells are CD34$^+$ dendritic cell progenitors. Optionally, the CD34$^+$ dendritic cell progenitors can acquire, upon cytokine stimulation, the phenotypes of presenting antigens through CD1d, MHC class I and II, induce specific T-cell proliferation, and/or displaying a mature transcriptional and phenotypic profile upon stimulation with inflammatory mediators (i.e. similar phenotypes to immature dendritic cells or Langerhans-like dendritic cells).

In one embodiment, the population of dendritic cells or population of dendritic-like cells is a population of dendritic cells. Preferably, the dendritic cells are primary dendritic cells. Preferably, the dendritic cells are myeloid dendritic cells.

Dendritic cells may be recognized by function, by phenotype and/or by gene expression pattern, particularly by cell surface phenotype. These cells are characterized by their distinctive morphology, high levels of surface MHC-class II expression and ability to present antigen to CD4+ and/or CD8+ T cells, particularly to naïve T cells (Steinman et al. (1991) Ann. Rev. Immunol. 9: 271).

The cell surface of dendritic cells is unusual, with characteristic veil-like projections, and is characterized by expression of the cell surface markers CD11c and MHC class II. Most DCs are negative for markers of other leukocyte lineages, including T cells, B cells, monocytes/macrophages, and granulocytes. Subpopulations of dendritic cells may also express additional markers including 33D1, CCR1, CCR2, CCR4, CCR5, CCR6, CCR7, CD1a-d, CD4, CD5, CD8alpha, CD9, CD11b, CD24, CD40, CD48, CD54, CD58, CD80, CD83, CD86, CD91, CD117, CD123 (IL3Ra), CD134, CD137, CD150, CD153, CD162, CXCR1, CXCR2, CXCR4, DCIR, DC-LAMP, DC-SIGN, DEC205, E-cadherin, Langerin, Mannose receptor, MARCO, TLR2, TLR3 TLR4, TLR5, TLR6, TLR9, and several lectins.

The patterns of expression of these cell surface markers may vary along with the maturity of the dendritic cells, their tissue of origin, and/or their species of origin. Immature dendritic cells express low levels of MHC class II, but are capable of endocytosing antigenic proteins and processing them for presentation in a complex with MHC class II molecules. Activated dendritic cells express high levels of MHC class 11, ICAM-1 and CD86, and are capable of stimulating the proliferation of naive allogeneic T cells, e. g. in a mixed leukocyte reaction (MLR).

Functionally, dendritic cells or dendritic-like cells may be identified by any convenient assay for determination of antigen presentation. Such assays may include testing the ability to stimulate antigen- primed and/or naive T cells by presentation of a test antigen, followed by determination of T cell proliferation, release of IL-2, and the like.

Methods of detecting and/or measuring the concentration of protein and/or nucleic acid are well known to those skilled in the art, see for example Sambrook and Russell, 2001, Cold Spring Harbor Laboratory Press.

Preferred methods for detection and/or measurement of protein include Western blot, North-Western blot, immunosorbent assays (ELISA), antibody microarray, tissue microarray (TMA), immunoprecipitation, *in situ* hybridisation and other immunohistochemistry techniques, radioimmunoassay (RIA), immunoradiometric assays (IRMA) and immunoenzymatic assays (IEMA), including sandwich assays using monoclonal and/or polyclonal antibodies. Exemplary sandwich assays are described by David et al., in US Patent Nos. 4,376,110 and 4,486,530. Antibody staining of cells on slides may be used in methods well known in cytology laboratory diagnostic tests, as well known to those skilled in the art.

Typically, ELISA involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemi-luminescent systems based on enzymes such as luciferase can also be used.

Conjugation with the vitamin biotin is frequently used since this can readily be detected by its reaction with enzyme-linked avidin or streptavidin to which it binds with great specificity and affinity.

In an additional or alternative embodiment, the method comprises one or more of the following steps:

(i) cultivating dendritic or dendritic-like cells;
(ii) seeding cells of (i) in one or more wells, preferably at steady state growth phase, e.g. wells of one or more multi-well assay plate;
(iii) adding to one or more well(s) of (ii) the protein(s) to be tested;
(iv) adding to one or more separate well(s) of (ii) positive control(s), e.g. Der p 1 and/or Der p 7;
(v) adding to one or more separate well(s) of (ii) negative control(s), e.g. DMSO; and/or leaving one or more separate well(s) of (ii) unstimulated to obtain a medium control;
(vi) incubating cells in wells of (iii)-(v), preferably for about 24 hours; and, optionally, harvesting cells from wells of (iii)-(v); and, further optionally, removing supernatant and storing in TRIzol reagent;
(vii) isolating purified total RNA from the cells of (vi) and, optionally, converting mRNA into cDNA;
(viii) quantifying expression levels of individual mRNA transcripts from (vii), e.g. using an array, such as an Affymetrix Human Gene 1.0 ST array;
(ix) exporting and normalizing data from (viii), e.g. using appropriate algorithms;
(x) isolating data from (ix) originating from biomarkers of the GARD Protein Allergen Prediction Signature (i.e. the biomarkers of Table A);

(xi) applying a prediction model to the data of (x), e.g. a frozen SVM model previously established and trained on historical data, e.g. data obtained in Example 1, to predict the allergenicity (e.g. classify as allergen/non-allergen), of tested protein(s) and negative/positive control(s).

A second aspect of the invention provides an array for use in the method according to the first aspect of the invention, the array comprising 23 or more binding moieties as defined in the first aspect of the invention, wherein the 23 or more binding moieties include binding moieties with specificity for each of the biomarkers defined by the following Probe Set ID numbers in Table A: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308.

In an additional or alternative embodiment the array consists of each of the biomarkers as defined in the first aspect of the invention.

In an additional or alternative embodiment the one or more binding moiety is immobilised.

In an additional or alternative embodiment the array is a bead-based array. In an additional or alternative embodiment the array is a surface-based array.

In an additional or alternative embodiment the array is selected from the group consisting of: macroarray; microarray; nanoarray.

The array may comprise or consist of a particular selection of biomarker-specific binding moieties which correlates to any particular selection of biomarkers as defined in the first aspect.

A third aspect of the invention provides the use of 23 or more biomarkers as defined in the first aspect of the invention for determining the allergenicity of a protein, wherein the 23 or more biomarkers selected from the group defined in Table A include the biomarkers defined by the following Probe Set ID numbers in Table A: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308.

In an additional or alternative embodiment there is provided the use of 23 or more binding moieties each with specificity for a biomarker selected from the group defined in Table A for determining the allergenicity of a protein, preferably wherein three or more of the binding moieties have specificity for a biomarker selected from the group defined in Table A(i).

A fourth aspect of the invention provides an analytical kit for use in a method according the first aspect of the invention comprising:

(a) an array according to the second aspect of the invention; and
(b) instructions for performing the method as defined in the first aspect of the invention (optional).

In an additional or alternative embodiment the analytical kit further comprising one or more control agents as defined in the first aspect of the invention.

Also disclosed herein, and not forming part of the claimed invention, is a method of treating or preventing an allergic reaction (for example a type I hypersensitivity reaction, such as respiratory asthma) in a patient comprising the steps of:

(a) providing one or more test protein that the patient is or has been exposed to;
(b) determining whether the one or more test protein provided in step (a) is allergenic using a method of the present invention; and
(c) where one or more test protein is identified as allergenic, reducing or preventing exposure of the patient to the one or more test proteins and/or providing appropriate treatment for the symptoms of allergenicity.

Preferably, the one or more test protein that the patient is or has been exposed to is a protein that the patient is presently exposed to at least once a month, for example, at least once every two weeks, at least once every week, or at least once every day.

Treatments of the symptoms of allergy may include, for example: short-acting beta2-adrenoceptor agonists (SABA), such as salbutamol; anticholinergic medications, such as ipratropium bromide; other adrenergic agonists, such as inhaled epinephrine; corticosteroids such as beclomethasone; long-acting beta-adrenoceptor agonists (LABA) such as salmeterol and formoterol; leukotriene antagonists such as montelukast and zafirlukast; mast cell stabilizers (such as cromolyn sodium) are another non-preferred alternative to corticosteroids; antihistamines; desensitization therapies via prolonged low concentration exposure; epinephrine shots (for acute anaphylactic shock symptoms); and/or avoidance of the sensitizing agent.

Preferably, the method of treatment is consistent with the method described in the first aspect of the invention, and one or more of the embodiments described therein.

Also disclosed herein, and not forming part of the claimed invention, is a computer program for operating the methods the invention, for example, for interpreting the expression data of step (c) (and subsequent expression measurement steps) and thereby determining whether one or more test protein is allergenic. The computer program may be a programmed SVM. The computer program may be recorded on a suitable computer-readable carrier known to persons skilled in the art. Suitable computer-readable-carriers may include compact discs (including CD-ROMs, DVDs, Blue Rays and the like), floppy discs, flash memory drives, ROM or hard disc drives. The computer program may be installed on a computer suitable for executing the computer program.

The skilled person will appreciate that all non-conflicting embodiments may be used in combination. Hence, embodiments from one aspect of the invention may equally be applied to a second aspect of the invention.

Preferred, non-limiting examples which embody certain aspects of the invention will now be described, with reference to the following figures:

**Figure 1**. Principal component analysis plot visualizing differences in transcript expression levels based on at least biological triplicate samples treated with the indicated substances (multigroup ANOVA comparing treatments; FDR=0.01, 1037 genes. Unstim=control cells "treated" only with (vehicle) cell culture medium).

**Figure 2.** Principal component analysis plot visualizing differences in transcript expression levels of all 33 samples (at least biological triplicates treated with the indicated substances) based on an input of the identified 391 potential biomarkers. Unstim=control cells "treated" only with (vehicle) cell culture medium.

**Figure 3.** A series of leave-one-out cross-validations was performed. For each cross-validation exercise, all replicates of the indicated treatment were removed and the backward elimination procedure was performed as described in Materials and Methods. Resulting predictions for the respective treatment are presented as boxplots depicting Support vector machine (SVM) decision values for each replicate. Negative DVs correspond to non-allergens, positive DVs to allergens.

**Figure 4.** Key Pathway Advisor analysis results in 27 significantly regulated pathways using the 391 transcripts as input, which were identified by a two group comparison between six protein allergens and controls followed by the application of a backward elimination algorithm.

**Figure 5**. Key Pathway Advisor analysis based on an input of the most significant 272 transcripts resulting from a two-group comparison of unstimulated control samples and protease-treated samples. The most significant pathways are presented (Union p-values$\leq$0.001, corresponding to a -log p-value of 3).

**Figure 6.** Changes in cell surface expression levels of CD86, CD14, CD1a, and HLA-DR in MUTZ-3 cells stimulated with the indicated substances for 24 hours as determined by flow cytometry (n=3, error bars show standard deviations). Data are normalized to respective control (unstimulated) sample (control=100%). MFI=mean fluorescence intensity.

**Figure 7.** Changes in Rantes and MIP-1$\alpha$ protein levels in MUTZ-3 supernatants after stimulation with the indicated substances for 24 hours, as determined by Bio-Plex® technology. (n=3, error bars show standard deviations).

**Figure 8:** Changes in protein levels of the indicated cytokines in MUTZ-3 supernatants after stimulation with the indicated substances for 24 hours, as determined by Bio-Plex®. (n=3, error bars show standard deviations).

**Figure 9:** Viability assessed with propidium iodide staining and flow cytometry; cells were treated with the indicated substances for 24 hours (n=3, error bars show standard deviations).

**Figure 10:** Changes in GM-CSF protein levels in MUTZ-3 supernatants after stimulation with the indicated substances for 24 hours, as determined by Bio-Plex® technology (n=3, error bars show standard deviations). GM-CSF was part of the complete cell culture medium (added to 40 ng/ml) in all samples, which is reflected by values out of range (oor) of the upper part of the standard curve (left bar), proteases seem to degrade GM-CSF (middle and right bar).

**Figure 11:** RNA microarray expression data of the indicated cytokines. (n=3, error bars show standard deviations. Microarray data is normalized using the scan algorithm [48, 49], and should thus not be interpreted in a linear fashion).

**Figure 12:** GARD Protein Allergen Prediction Signature (PAPS) Decision Values for biological triplicate samples of test substances.

## EXAMPLE 1

*Introduction*

Allergy is a chronic disease with increasing prevalence and it is of outmost importance for the industry and authorities to identify potential allergens as early as possible to limit the exposure of workers and the general populations. Several hundreds of chemicals are known to be able to cause allergic contact dermatitis [1, 2], a type IV delayed hypersensitivity reaction, whereas less chemicals are known to sensitize the respiratory tract and to induce type I allergic responses [3]. Most substances causing respiratory allergy are proteins of environmental origin e.g. allergens from house dust mite feces, pollen, or fungi, while others are present in an occupational setting such as enzymes used in flavor, fragrance, detergents and pharmaceutical production [4, 5]. The risk of developing adverse reactions following occupational exposure exists; thus, a strict focus on occupational safety is mandatory. Sensitization has been observed for workers exposed to certain industrial enzymes such as $\alpha$-amylase, proteases, pancreatinin, and trypsin [6, 7]. New enzymes are continuously developed for existing as well as for new applications, such as genetically modified enzymes used in food processing and flavor production and may also lead to occupational health risks [5, 7].

To date, no validated assay is available specifically for predicting the sensitizing potential of novel proteins, rendering a weight-of-evidence approach to be the most acceptable means of allergy safety assessment. There is, however, a growing consensus that the allergenic potential of compounds, including proteins, should be evaluated with regard to their biochemical characteristics and the protein's potential to induce a specific immune response (European COST Project impARAS [8]). A combination of physical traits of proteins, the molecular interaction between human cells and proteins, as well as their impact on cell-cell interactions play a role in understanding and eventually predicting protein allergenicity [9, 10].

The Adverse Outcome Pathway (AOP) concept is a framework for collecting and organizing information relevant to an adverse outcome at different levels of biological organization [11]. These AOPs are based on available information on substance-response and response-response relationships and allow the development of relevant predictive animal-free test methods and approaches, as well as the contextualization of the results across a diverse range of biological mechanisms and toxicity endpoints. The mechanisms driving respiratory sensitization are not fully disclosed yet, but emerging data suggest that events driving respiratory and skin sensitization can be structured in the same adverse outcome pathway (AOP). In contrast to skin sensitization, properly evaluated test methods addressing the key events of respiratory sensitization induction are not yet available [12].

The Genomic Allergen Rapid Detection (GARD) assay was initially developed to provide information about the capacity of chemicals to induce skin sensitization (accuracy: 89 % [13, 17]). This *in vitro* assay utilizes a myeloid cell line resembling dendritic cells (DCs) as a model system. DCs are antigen-presenting cells and central for the induction and regulation of adaptive immune responses [14]. This assay was recognized by both the European Reference Laboratory - European Center for Validation of Alternative Methods (EURL-ECVAM) and the OECD as a valuable method for addressing key event 3 (Dendritic cell activation and maturation) of the AOP for skin sensitization [15]. Forreryd *et al.* [16] successfully demonstrated that a modified protocol of the assay is able to predict respiratory chemical sensitizers with an accuracy of 84 % based on a biomarker signature consisting of 389 transcripts.

In this example, this protocol is assessed for its capacity to provide a mechanistic understanding of the protein sensitization at DC level. A potential gene profile for the classifications of proteinaceous allergens was identified. For this purpose, the effect of eight respiratory protein allergens on the gene expression in the myeloid cell line was investigated using Affymetrix RNA expression array technology. A panel of potential biomarkers distinguishing the allergens from control samples was identified using data-driven approaches including machine learning, and cross-validation exercises indicated that the identified transcripts are indeed useful for classification. These biomarkers were further investigated with regard to associated biological pathways.

*Materials and Methods*

*Respiratory allergens*

All allergens contain low levels of endotoxin as summarized in Table 1. LoToxTM recombinant Der p 7 (#LTR-DP7-1) and LoToxTM natural Der p 1 (#LTN-DP1-1) were obtained from Indoor biotechnologies, Charlottesville, USA. The other allergens were provided by Novozymes A/S, Bagsvaerd, Denmark, and tested for endotoxin content by Sahlgrenska Universitetssjukhuset, Bakteriologiska laboratoriet, Göteborg.

**Table 1** Identity and concentrations of allergens and controls.

| Substance | Test compound concentration | Endotoxin in well conc. [EU/mL] |
|---|---|---|
| amylase 1 | 25 µg/mL | 0.04 |
| amylase 2 | 25 µg/mL | 0.059 |
| protease 1 | 25 µg/mL | <0.0012 |
| protease 2 | 25 µg/mL | <0.0006 |
| glycohydrolase | 25 µg/mL | 0.033 |
| lipase | 25 µg/mL | 0.022 |
| Der p 1 | 25 µg/mL | <0.75 |
| Der p 7 | 25 µg/mL | <0.25 |
| DMSO | 0.1% | NA |
| unstimulated | | NA |

*Cell culture and stimulations*

The maintenance of the cell line was performed as described in [17]. In short, the MUTZ-3 derivative cells were cultured in MEM alpha modification (Nordic Biolabs/GE Healthcare BioSciences, Täby, Sweden) supplemented with 20 % fetal bovine serum (Thermo Fisher Scientific/Life Technologies, Stockholm, Sweden) and 40 ng/mL recombinant human GM-CSF (Miltenyi Biotec Norden AB, Lund, Sweden). A dose finding experiment was performed based on earlier protocols optimized for this cell line to identify the highest enzyme concentration resulting in a relative cell viability of ≥ 90 % after 24 hours of incubation. Longer exposures (48 hours) to certain enzymes resulted in substantially decreased cell viability (data not shown). A phenotypic control analysis of cells prior to each experiment was carried out by flow cytometry (see below) in order to assure an immature state. In short, three batches of cells were exposed to allergens and control substances dissolved in complete cell culture medium for 24 hours in at least three independent experiments (Table 1). Proteins present in serum served as non-allergen protein controls. Phenotypic controls and viability of the cells were assessed after the stimulation period by analyzing cell surface expression using flow cytometry as described below. Cells aimed for RNA extraction were lysed in TRIzol® (Life Technologies/Thermo Fisher Scientific, Waltham, USA) and stored until further use in minus 20°C.

*Flow cytometry*

As a quality control both before and during the experiments, the following monoclonal antibodies were used during phenotypic analysis as described previously [18]: CD1a (DakoCytomation, Glostrup, Denmark), CD34, CD86, HLA-DR (BD Biosciences, Franklin Lakes, NJ), all FITC-conjugated; CD14 (DakoCytomation), CD54, CD80 (BD Biosciences), all PE-conjugated. FITC- and PE-conjugated mouse IgG1 (BD Biosciences) were used as isotype controls and Propidium iodide was included as a marker for non-viable cells (BD Biosciences). After 24 hours incubation with the allergens and control samples, viability, CD14, CD1a, HLA-DR and CD86 expression were assessed. FACS samples were analyzed on a FACSCanto II instrument with FACS Diva software for data acquisition. 10 000 events were acquired and further analysis was performed in FCS Express V4 (De Novo Software, Los Angeles, CA). An endotoxin stimulation was included as internal control for cell quality.

*RNA extraction, cDNA and array hybridization*

RNA isolation from cells lysed in TRIzol® was performed according to manufacturer's instructions. Labeled sense DNA is synthesized according to Affymetrix' protocols using the recommended kits and controls. The cDNA was hybridized to Human Gene 1.0 ST arrays and further processed and scanned as recommended by the supplier (Affymetrix, Cleveland, USA).

*Data acquisition and analysis*

The dataset, comprising 33 samples in total, was quality-controlled and then normalized using Single Channel Array Normalization [19, 20]. Statistical analysis was performed primarily by Analysis of Variation (ANOVA). ANOVAs and PCA visualization of results were performed with Qlucore 3.2 (Qlucore AB, Lund, Sweden). Significance was evaluated with the multiple-hypothesis corrected p-value (q-value, in this article referred to as false discovery rate (FDR) [21]). An FDR ≤0.05 was considered as statistically significant. Decision values were calculated in a support vector machine (SVM) model [22], constructed in R (R Development Core Team, 2008) and using the package e1071 (R package e1071). The same R package was used to program the backward elimination algorithm [23]. The backward elimination algorithm was applied to a dataset consisting of nine non-allergen and 24 allergen samples and the 1052 most significant genes based on a two group comparison between allergens and controls (p=0.007, FDR=0.19212). Cross-validations were performed in a similar manner based on an input where one treatment at a time had been removed. All additional statistical computing and handling of data was performed in Excel (Microsoft Office 2013) and R (www.R-project.org).

*Bio-Plex 200 cytokine analysis*

Cell supernatants were analyzed using a BioPlex 200 system (Bio-Rad, Hercules, USA) with the Novex® Human Cytokine Magnetic 30-Plex Panel kit (# LHC6003M, Invitrogen/Thermo Fisher Scientific, Waltham, USA) according to the manufacturer's recommendations. Biological triplicates, based on three batches of cells, were analysed in technical duplicates (maximum CV accepted was 20 %) and concentrations in range were accepted according to the Bio-Plex 200 Manager 6.1 software (Bio-Rad, Hercules, USA) unless stated otherwise. Data are presented as mean values; error bars represent standard deviations. Due to a limited number of samples (biological triplicates, technical duplicates) and non-normally distributed data, p-values as a measure of statistical significance are not appropriate to provide. An endotoxin stimulation was included as internal assay control.

*Key pathway analysis*

The Key Pathway Advisor (KPA) tool [24] versions 16.4 and 16.6, were used to investigate the identified 391 genes (shown in Table A) comprising the protein prediction signature in a biological context. KPA associates e.g. differentially expressed genes derived from RNA microarray data with both upstream and downstream processes in order to allow biological interpretation. The investigated data set consisted of 33 samples, comprising allergen and control samples in at least three replicates. Both p values and fold changes were used as input based on a two group comparison between allergens and non-allergens (Key Hubs Calculation Algorithm: causal reasoning analysis; Key Hubs p-value threshold=0.01; Key Processes p-value threshold=0.05; unrecognized IDs: 105). An analysis using the same parameters was performed with 272 genes as input, which resulted as the most significant genes from a p-value filtering of proteases versus unstimulated samples (p=0.001335, FDR=0.05) based on a variance-prefiltered data set containing 10054 variables. 254 key hubs were predicted and nine IDs were not recognized.

**Scripts**

Listed below are details of the script, written in R code, used to perform the method:

```
# Required files:
# - GARD_PAPS.R
# - raw affymetrix files of test samples in subdir: raw_affy/
# - Annotation of the new data describing the unstimulated samples
raw_affy/annotation.rds
# - Historical data stored in trainingset.rds
# Load required dependencies
source('~/GARD_PAPS.R')
# Read Training Data
train = readRDS('~/trainingset.rds')
# Read new data and annotations
new_data = read_raw_affy('~/raw_affy/*.CEL')
new_data_ref = readRDS('~/raw_affy/annotation.rds')
# Normalize the new data
normalized_data = normalize_train_test(train = train, test = new_data,
test_reference = new_data_ref)
# Train model on historical data
model = train_svm(normalized_data$train)
# Predict New Samples
```

```
predictions = predict_test_samples(model = model,
data=normalized_data$test)
```

## *Results*

### *Statistical analysis of expression array data*

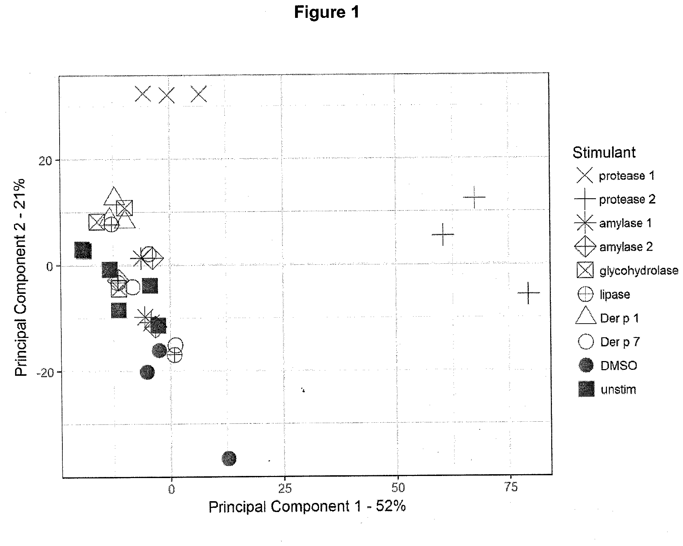

The data set consisted of 33 samples in total, nine non-allergen controls and 24 allergen samples (Table 1). When comparing these two groups (i.e. controls, "unstimulated" and "DMSO", versus allergens), no significantly regulated genes could be detected based on a false discovery rate (FDR) of 0.05. With a chosen cut-off of p=0.0001 (FDR=0.0847), 31 variables were left. This indicates that only smaller differences exist between the group of protein allergens and non-allergen controls. When comparing all treatments to each other (multigroup ANOVA, FDR=0.01), 1037 significantly regulated genes were identified. All samples cluster rather tightly except the proteases as presented in a Principal Component Analysis (PCA) plot in Figure 1, indicating that the RNA expression patterns induced by each protease differ somewhat from all the other samples.

### *Identification of a protein allergen prediction signature*

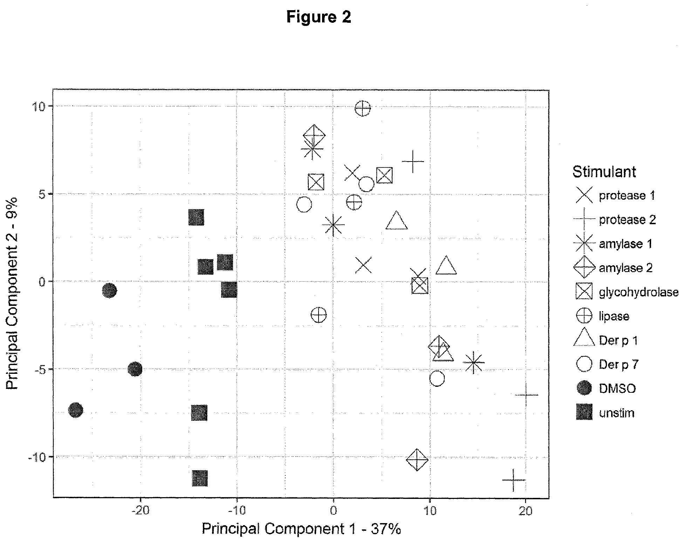

In order to develop a classification approach, a so-called wrapper method was used to identify the most relevant genes for distinguishing allergens from non-allergens after initial p-value filtering. The used backward elimination algorithm [23] based on support vector machine (SVM) predictions [25] results in an optimized list of genes. Each gene or feature in this list has been evaluated not only for its importance for the classification itself, but also how it performs together with the other features. Using an input of the most significant 1052 genes, based on a two-group comparison (allergen versus non-allergen, p=0.007, FDR=0.19), the algorithms identified a signature consisting of 391 genes (see Table A). When the dataset is visualized by PCA using these potential biomarkers as an input, a clear separation between allergens and non-allergens can be seen (Fig. 2).

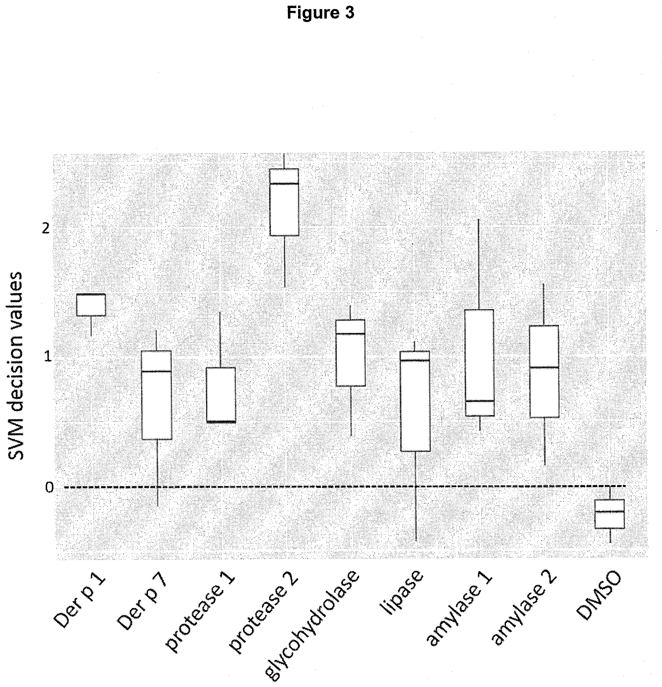

In order to estimate the accuracy of the model and to ensure that the presented results were not achieved by chance, a series of leave-one-out cross-validations was performed. For each cross-validation exercise, all replicates of one stimulation were removed and the backward elimination procedure was repeated as described above. The obtained gene signature was then used to predict the left out samples using an SVM model based on a training set consisting of the remaining samples in the dataset. All stimulations were removed once except "unstimulated"; without the "unstimulated" samples, the dataset would be too unbalanced to be used for training of a model. The resulting SVM predictions are visualized as boxplots in Fig. 3. All allergens were correctly predicted as such by majority voting, even though one replicate each for lipase and Der p 7 was predicted as non-allergen (negative decision value). SVM predictions were also performed after a similar exercise based on a dataset where DMSO and Der p 1 samples were left out and subsequently used as a test set. Also here, DMSO was correctly classified as non-allergen and Der p 1 as an allergen (data not shown).

### *Biological pathway analysis reveals association of gene lists with immunological pathway regulation*

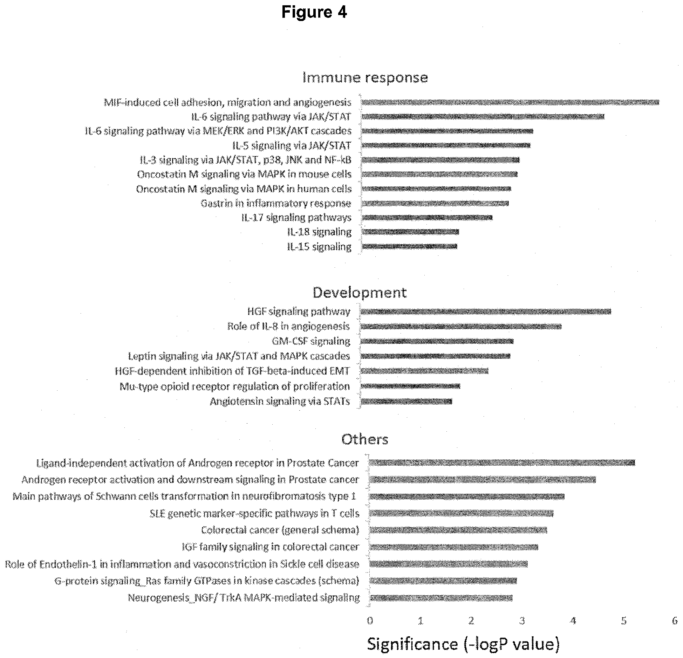

The association of the 391 identified biomarkers with biological pathways was investigated using the Key Pathway Advisor tool, and the gene list together with associated p-values and fold changes based on a two-group comparison (allergen yes/no) were used as input. Of in total 27 significant pathways identified (Fig. 4), roughly one third was allocated to immune system-related pathways, e.g. IL-3, IL-5, IL-6, IL-15, IL-17 and IL-18 signalling pathways. Examples of suggested key hubs, all with increased activity, were MHC class I, NFκβ variants p50 and p50/p65, IL-10, IL-6R, IL-10R, FcγRIIα, glutaredoxin, and integrins α6β4 and α2B.

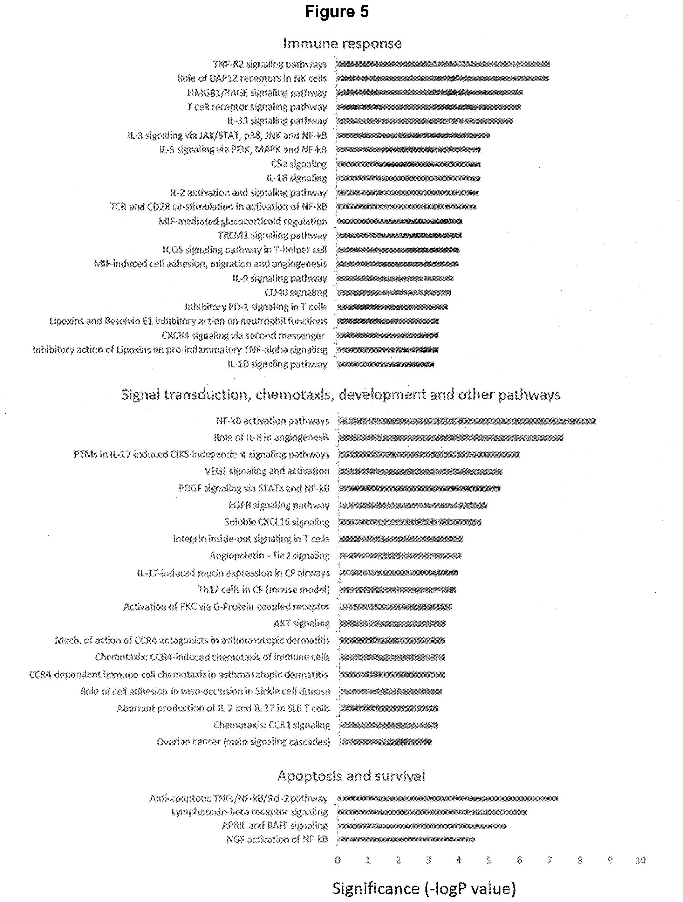

When investigating biological pathways based on a comparison between proteases and unstimulated samples and the resulting 272 most significant genes, 59 significant pathways were identified and over 40 % are related to immune responses as judged by their name (Fig. 5), e.g. IL-2, IL-10, IL-33 and CD40 signalling. Further, "CCR4-induced chemotaxis of immune cells", "CCR4-dependent immune cell chemotaxis in asthma and atopic dermatitis" and NFκB-related pathways are associated to inflammatory responses. NPκB also appeared as a predicted key hub, along with other molecules such as MyD88, an adaptor protein used by almost all TLRs to activate NFκB. Toll-like receptor 4 and high-mobility group box-1 (HMGB1), a transcriptional regulator involved in inflammation and cell differentiation, were two more examples of predicted key hubs

### *Protein allergens affect both surface marker expression and secreted cytokines*

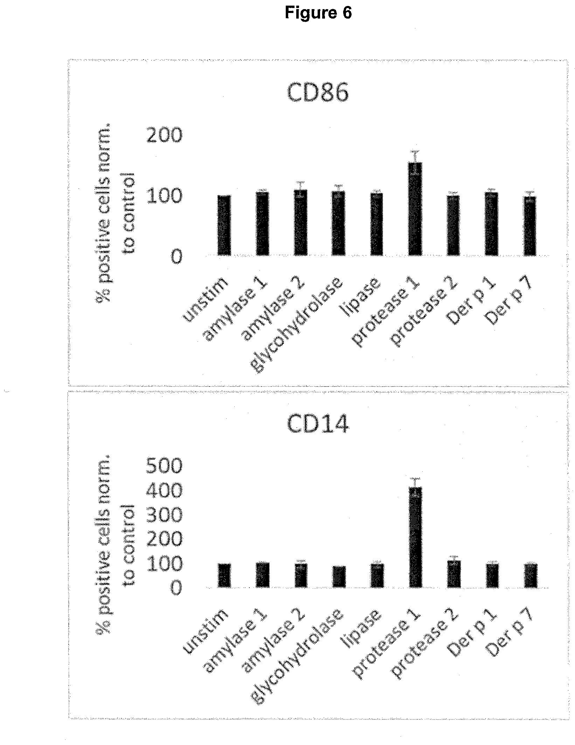

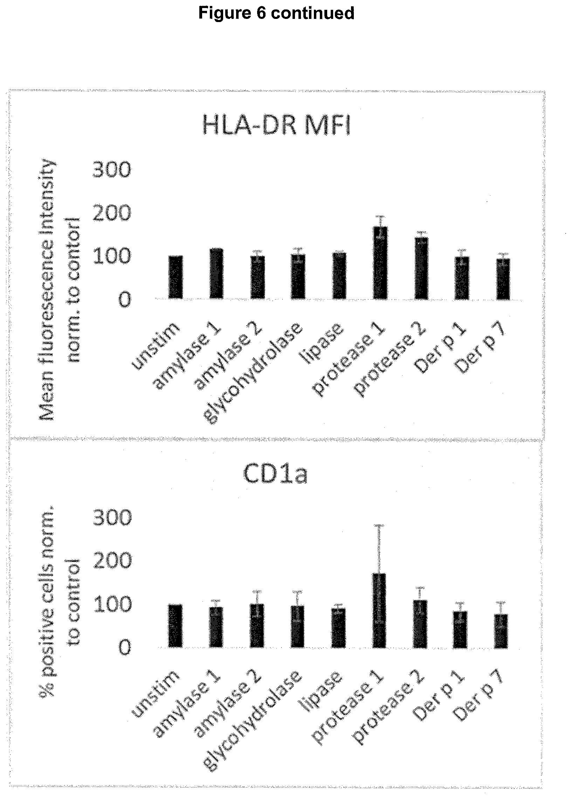

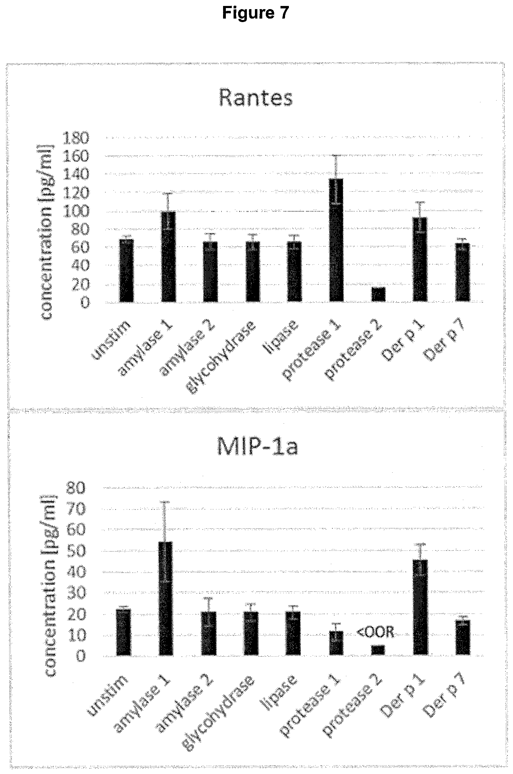

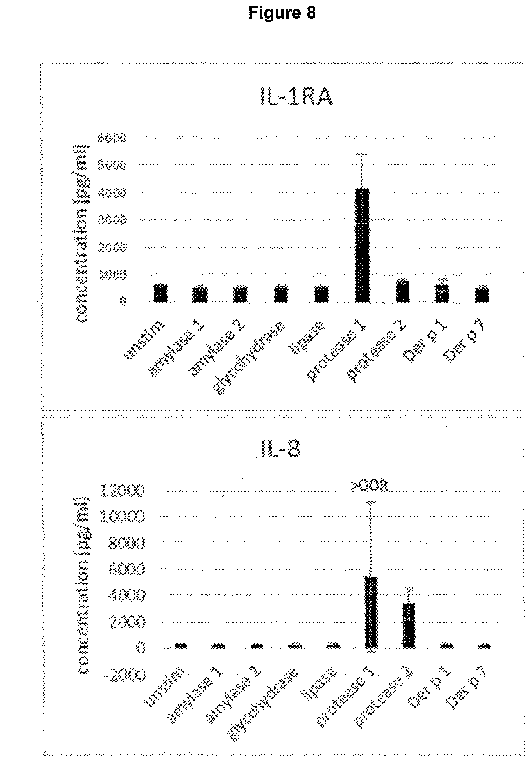

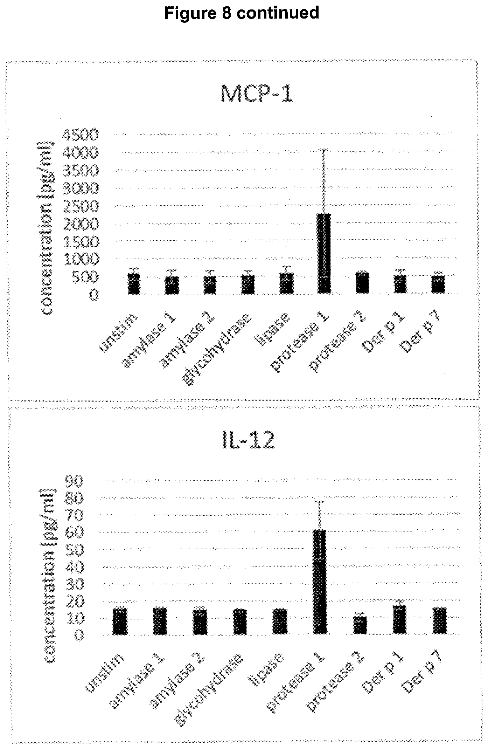

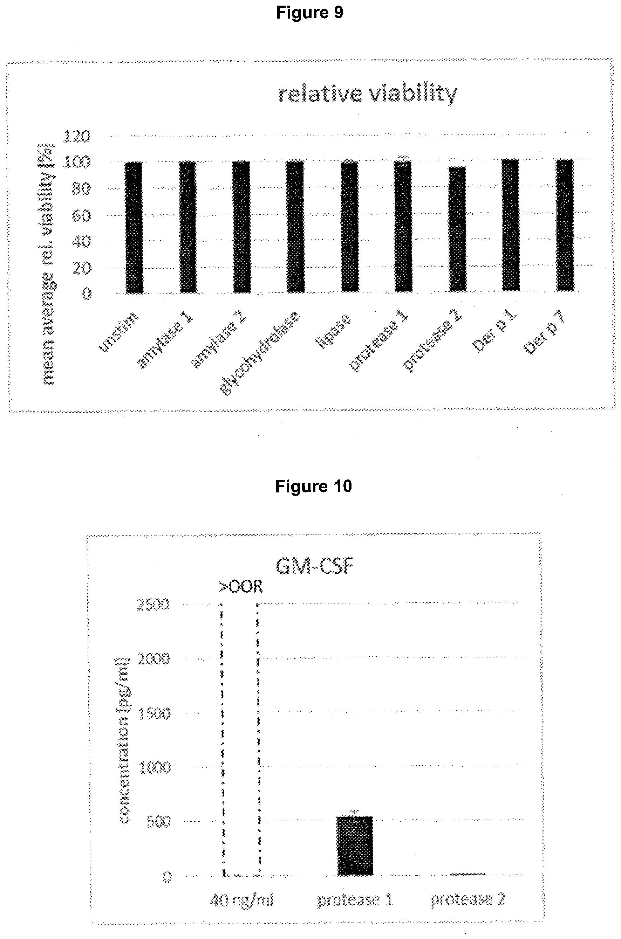

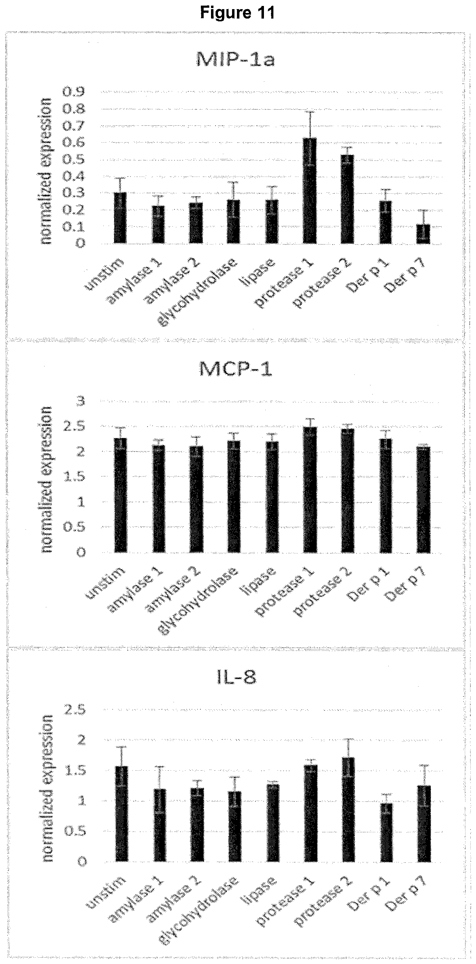

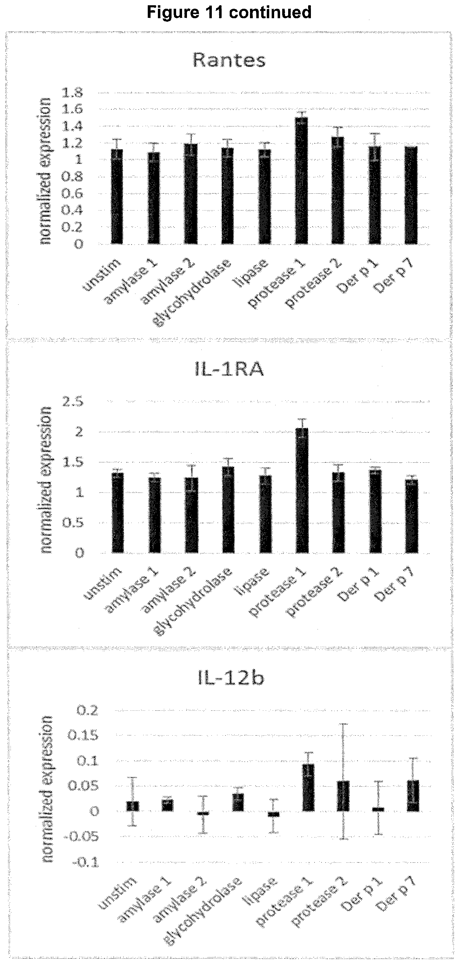

As a complement to the transcriptional analysis, effects on the protein level were investigated by FACS, focusing on a set of differentiation and activation markers as described in Fig. 6. All cells were positive for HLA-DR in the unstimulated control as well as after exposure to all substances (data not shown). There was no protein allergen that reduced the presence of any investigated marker; however, protease 1 seemed to increase expression of e.g. CD86, a known activation marker. Notably, the relative viability after all treatments was $\geq$90 % (Fig. 9). When screening 30 soluble cytokines using Bio-Plex® technology, Der p 1 and amylase 1 treatment increased the levels of MIP-1$\alpha$ and Rantes in supernatants (Fig. 7). The proteases reduced the levels of several cytokines; likely at least for some by degradation as supported by the results for GM-CSF. GM-CSF is added to the cell medium at 40 ng/mL, but after incubation with the proteases, the values were lowered to a few pg/mL (protease 2) and to approximately 500 pg/mL (protease 1), respectively (Fig. 10), whereas the values of all the other allergens and controls were as expected out of range at the upper limit. However, the proteases increased the levels of e.g. IL-8 (Fig. 8), and protease 1 treatment induced higher levels of Rantes (Fig. 7A), IL-12, MCP-1 and IL-1RA levels (Fig. 8). The data on cytokine protein expression were in part correlated with the changes on RNA level (Fig. 11).

### *Discussion*

In this example, the development of an *in vitro* method for the prediction of protein allergens using eight known respiratory allergens as model substances is described. Using data-driven approaches based on machine learning, a list of biomarkers was identified (Table A), which was further investigated with regard to associated biological pathways.

Comparing the RNA expression pattern of cells exposed to controls and those exposed to the respiratory protein allergens, no significantly regulated transcripts based on an FDR of 0.05 could be observed. This could be due to several factors, e.g. relatively small differences between the groups, but also high variation in the transcriptional changes induced by the structurally and functionally different enzymes. Unexpectedly, even when comparing the intended positive controls, Der p 1 and Der p 7, separately to the unstimulated control samples, no significantly regulated genes could be identified. On the protein level, however, Der p 1 seemed to increase Rantes and MIP-1$\alpha$ cytokine levels (Fig. 7), which would be consistent with a pro-inflammatory response induced by Der p 1 [26, 27]. As endotoxins even at low concentrations may activate cells it is important to consider their effects as they may obliterate allergen signals. Der p 1 and Der p 7 have therefore been obtained as "low endotoxin" preparations; nevertheless they contain the highest amounts of endotoxin of all samples considering final concentrations (Table 1, 0.75 EU/mL and 0.25 EU/mL, respectively). As comparison, standard endotoxin levels in fetal bovine serum usually account to $\leq$1ng/mL or $\leq$10 EU/mL, respectively, according to the industrial standard [28]. However, endotoxins are likely also an intrinsic part of allergic reactions in "real life", as endotoxins are more or less ubiquitously present. Furthermore, Der p 1 preparations, including the product used here, commonly do not exhibit cysteine protease activity due to the production process. For Der p 1, cysteine protease activity has been described to enhance synthesis of Der p 1-specific IgE and allergenicity in a mouse inhalation model [29, 30]. On the other hand, data presented by Porter *et al.* [31] indicate that Der p 1 in house dust samples does not have measurable protease activity. In the here presented study, Der p 1's cysteine protease activity was not restored by reducing treatment, as we could not detect any difference in activation of monocyte-derived dendritic cells when comparing cells stimulated with Der p 1 treated with DTT in order to activate its cysteine activity with non-treated Der p 1 (data not shown). Nevertheless, the identified biomarker signature consisting of 391 transcripts was able to separate the dataset into two clearly distinct groups, i.e. controls and allergens (Fig. 2). Though separate statistical analyses could not identify significantly regulated transcripts between these two groups, this together with the cross-validation results (Fig. 3) indicates that several transcripts together in a signature may still be useful for the prediction of the protein allergens, for example those capable of sensitizing the respiratory tract.

Biological pathway analysis using the KPA tool and the potential biomarker signature as input, revealed an overrepresentation of numerous pathways linked to immune responses. Further evidence supporting the relevance of the identified pathways for respiratory sensitization is provided by the current understanding of the NF-$\kappa$B signaling pathway, which was a suggested upstream regulator for the identified transcriptional changes. NF-$\kappa$B activation is causally related to increased release of various signal molecules such as IL-33, IL-25 [32, 33], endogenous danger factors (e.g. HMGB-1, uric acid and ATP by epithelial cells and DCs); further to DC activation and migration [34], and the induction of ovalbumin [35] and Der p 2 sensitization [36]. The release of ATP and uric acid drives the activation of the NLRP3 inflammasome complex resulting in cleavage of pro-IL-1$\beta$ to mature IL-1$\beta$ through caspase 1 [37]. Uric acid may play an important role in Th2 skewing [38].

Although human and animal data indicate that the here investigated protein allergens do not differ significantly in terms of allergenicity [39], in our *in vitro* system, protease 1 and 2 induced a particular RNA expression pattern and also influenced the protein levels of certain cell surface markers and cytokines. This may not necessarily be associated with their allergenicity, as it could also be related to cytotoxic and adjuvant properties as described for several cysteine and serine proteases and certain proteases used in detergents [40-43]. When the cells were exposed to the proteases for 48 hours, relative cell viability decreased (data not shown), however, the relative viability never dropped below 90 % after 24 hours (Fig. 10). Although it is expected that proteases are able to degrade certain proteins such as cytokines as shown for GM-CSF (Fig. 11), based on the here presented investigations, it is not possible to make conclusions about which mechanisms are involved. It seems likely that both cytokines and cell surface proteins can be cleaved and thus direct and indirect effects on e.g. cell differentiation and activation status can be exerted. Importantly, in order to be able to test these and similar substances in vitro, it is important to carefully choose concentrations not exceeding a certain cytotoxicity. The responses may otherwise be dominated by events reflecting cytotoxicity; comparable to how the irritant properties of certain contact allergens can mask the effects linked to sensitization [44]. On the other hand, irritant or cytotoxic effects may also allow or facilitate sensitization by creating a pro-inflammatory environment as shown for several skin sensitizing chemicals [45-47]. For proteases, impairment of barrier functions and thus higher permeability may play a role as well [41, 42].

Taken together, our results indicate that the identified 391 biomarkers can be useful in order to predict the allergenicity of proteins including proteases, and particularly the sensitizing ability of respiratory protein allergens, including proteases. The results of a series of cross-validations support a valid model (Fig. 3). The biomarker signature also seems to reflect relevant biological pathways.

## EXAMPLE 2

This example describes allergenic predictions of two samples of Tropomyosin with different species origin, along with appropriate controls. It provides a proof of concept of the applicability of the GARD Protein Allergen Prediction Signature of Table A (herein referred to as "GARD PAPS") to perform allergenic predictions on protein samples.

The readout of GARD is a set of genomic predictors, referred to as the GARD Prediction Signature (GPS).

In this example, the functionality of GARD PAPS was further demonstrated by assessing the allergenicity two Tropomyosin samples, with different species origin.

The genetic material of the cells are isolated from cell samples stimulated with the test substances. The transcriptional levels of the GARD PAPS are quantified and compared to a reference data set by the use of multivariate statistical prediction models. Each sample is assigned a decision value based on its transcriptional levels of the GARD PAPS, as measured by Affymetrix microarray technology. Final predictions are based on the mean value from biological triplicate samples.

In this example, results from 2 proteins, an LPS control and unstimulated cells are presented. For a results summary, see Table 2.

**Table 2.** Summary Results.

| Test substance ID | GARD PAPS Prediction |
|---|---|
| Purified natural shrimp tropomyosin | Sensitizer |
| Tropomyosin from pork muscle | Non-sensitizer |
| LPS | Non-sensitizer |
| Unstimulated cells | Non-sensitizer |

### Results

*Test substance handling and GARD input concentrations*

All proteins and test substances were stored and prepared according to instructions provided by the supplier. The proteins were screened for cytotoxic effects and the GARD input concentration was established for each protein. Results from this screening and resulting GARD PAPS input concentrations are presented in Table 3.

**Table 3.** Test substance(s) details.

| Test substance ID | Vehicle | Max. screen[I] | Rv90[II] | GARD input concentration[III] |
|---|---|---|---|---|
| Tropomyosin shrimp | PBS | 25 | - | 25 |
| Tropomyosin pork | PBS | 25 | - | 25 |
| LPS | PBS | - | - | $10^{-4}$ |

(continued)

| Test substance ID | Vehicle | Max. screen[I] | Rv90[II] | GARD input concentration[III] |
|---|---|---|---|---|
| Unstimulated cells | - | - | - | - |

I) The highest concentration used in screening titration range. Concentration is given in $\mu$g/ml.
II) Concentration yielding 90% relative viability. Concentration is given in $\mu$g/ml.
III) Based on Max. screen and Rv90. Concentration is given in $\mu$g/ml.

LPS is here used as a negative control, ensuring that observed signals generated by either of the two Tropomyosin samples are not due to endotoxin contaminants. Endotoxin contents of the Tropomyosin samples were quantified using a LAL test. The LPS concentration used as a negative controls was set to correspond to the highest endotoxin concentration present in either Tropomyosin sample.

All test proteins and substances were assayed in biological triplicates.

### GARD PAPS classifications

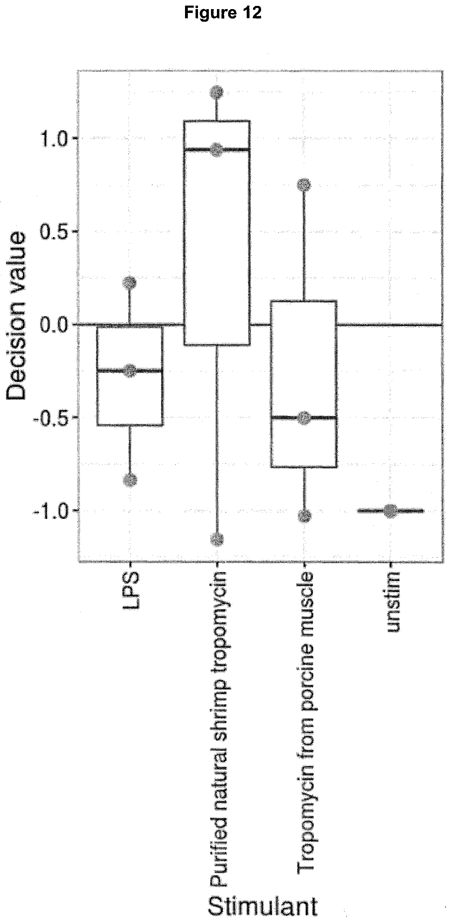

All replicates of test substances were assigned decision values using the GARD PAPS prediction model, as described (see materials and methods below). Decision values from test substances are presented in Figure 12.

### Materials & Methods

The comprehensive materials and methods for the GARD testing strategy, used to generate data described in this example, is included below.

### Deviations from standard protocols

The cytotoxic effects of the test proteins were monitored in the concentration range 1-25 $\mu$g/ml. No cytotoxicity could be detected and 25 $\mu$g/ml was used as the GARD input concentration, based on findings in scientific literature on in vitro methods for protein allergen predictions.

When stimulating the human myeloid cell line with the test substances, the proteins were first dissolved in PBS to a concentration of 1000 $\mu$g/ml. 50 $\mu$l of the dissolved proteins were added to 1.95 ml of seeded cells. LPS was diluted in PBS to a final concentration of 0.1 $\mu$g/ml and 2 $\mu$l were added to 1.998 ml of cell suspension.

The cells were stimulated for 24 h after which they were lysed in TRIzol reagent. RNA was purified, labeled and hybridized to arrays by the SCIBLU core facility for Affymetrix technology, Lund, Sweden.

The quantified transcription levels were single chain array normalized (SCAN) and the GARD PAPS was extracted from the data set. Unstimulated samples, from the test samples and the reference samples used to build the prediction model, were used to remove batch effects between the two data sets.

Final classifications were made using a support vector machine (SVM) which had been trained on the reference samples used to establish the GARD PAPS.

### Cell line maintenance and seeding of cells for stimulation

The human myeloid leukemia-derived cell line is maintained in $\alpha$-MEM (Thermo Scientific Hyclone, Logan, UT) supplemented with 20% (volume/volume) fetal calf serum (Life Technologies, Carlsbad, CA) and 40 ng/ml rhGM-CSF (Bayer HealthCare Pharmaceuticals, Seattle, WA), as described (Johansson et al., 2011). A media change during expansion is performed every 3-4 days, or when cell-density exceeds 5-600.000 cells/ml. Proliferating progenitor cells are used for the assay, with no further differentiation steps applied. During media exchange, cells are counted and suspended to 200.000 cells/ml. Working stocks of cultures are grown for a maximum of 20 passages or two months after thawing. For chemical stimulation of cells, 1.8 ml is seeded in 24-well plates at a concentration of 222.000 cells/ml. The compound to be used for stimulation is added in a volume of 200 $\mu$l, diluting the cell density to 200.000 cells/ml during incubation.

### Phenotypic analysis

Prior to any chemical stimulation, a qualitative phenotypic analysis is performed to ensure that proliferating cells are in an immature stage. All cell surface staining and washing steps are performed in PBS containing 1% BSA (w/v). Cells are incubated with specific mouse monoclonal antibodies (mAbs) for 15 min at 4°C. The following mAbs are used for flow cytometry: FITC-conjugated CD1a (DakoCytomation, Glostrup, Denmark), CD34, CD86, and HLA-DR (BD Biosciences, San Diego, CA), PE-conjugated CD14 (DakoCytomation), CD54 and CD80 (BD Biosciences). Mouse IgG1, conjugated to FITC or PE are used as isotype controls (BD Biosciences) and propidium iodide (PI) (BD Biosciences) is used to assess cell viability. FACSDiva software is used for data acquisition with FACSCanto II instrument (BD Bioscience). 10,000 events are acquired, gates are set based on light scatter properties to exclude debris and non-viable cells, and quadrants are set according to the signals from isotype controls. Further data analysis is performed, using FCS Express V3 (De Novo Software, Los Angeles, CA). For a reference phenotype of unstimulated cells, see Johansson et al., 2011. Accepted ranges of listed phenotypic markers are listed in Table 4.

**Table 4.** Accepted ranges of proportion of positive cells for acceptance criteria I: Phenotypic analysis.

| Phenotypic biomarker | Accepted range of positive cells (%) |
|---|---|
| CD86 | 10-40 |
| CD54 | >95 |
| HLA-DR | >60 |
| CD80 | <10 |
| CD34 | 35-70 |
| CD14 | 5-50 |
| CD1a | 10-60 |
| Propidium Iodide (Cell viability) | <15 |

*Chemical handling and assessment of cytotoxicity*

All chemicals are stored according to instructions from the supplier, in order to ensure stability of compounds. Chemicals are dissolved in water when possible or DMSO for hydrophobic compounds. As many chemicals will have a toxic effect on the cells, cytotoxic effects of test substances are monitored. Some chemicals are poorly dissolved in cell media; therefore the maximum soluble concentration is assessed as well. The chemical that is to be tested is titrated to concentrations ranging from 1 $\mu$M to the maximum soluble concentration in cell media. For freely soluble compounds, 500 $\mu$M is set as the upper end of the titration range. For cell stimulations, chemicals are dissolved in its appropriate solvent as 1000x stocks of target in-well concentration, called stock A. A 10x stock, called stock B, is prepared by taking 10 $\mu$l of stock A to 990 $\mu$l of cell media. 200 $\mu$l of stock B is then added to the wells containing 1.8 ml seeded cells. For the samples dissolved in DMSO, the in-well concentration of DMSO will thus be 0.1%. Following incubation for 24h at 37°C and 5% $CO_2$, harvested cells are stained with PI and analyzed with a flow cytometer. PI-negative cells are defined as viable, and the relative viability of cells stimulated with each concentration in the titration range is calculated as

$$Relative\ viability = \frac{fraction\ of\ viable\ stimulated\ cells}{fraction\ of\ viable\ unstimulated\ cells} \cdot 100$$

For toxic compounds, the concentration yielding 90% relative viability (Rv90) is used for the GARD assay, the reason being that this concentration demonstrates bioavailability of the compound used for stimulation, while not impairing immunological responses. For non-toxic compounds, a concentration of 500 $\mu$M is used if possible. For non-toxic compounds that are insoluble at 500 $\mu$M in cell media, the highest soluble concentration is used. Whichever of these three criteria is met, only one concentration will be used for the genomic assay. The concentration to be used for any given chemical is termed the 'GARD input concentration'.

*Chemical exposure of cells for GARD*

Once the GARD input concentration for chemicals to be assayed is established, the cells are stimulated again as described above, this time only using the GARD input concentration. All assessments of test substances are assayed in biological triplicates, performed at different time-points and using different cell cultures. Following incubation for 24h at 37°C and 5% $CO_2$, cells from one well are lysed in 0.5ml TRIzol reagent (Life Technologies) and stored at -20°C until RNA is extracted. In parallel, a small sample of stimulated cells is taken for PI staining and analysis with flow cytometry, to ensure the expected relative viability of stimulated cells is reached.

*Preparation of benchmark controls*

In addition to any test substance(s) to be assayed within a campaign, a set of benchmark controls are performed, for the purpose of prediction model calibration and estimation of prediction performance. For details regarding benchmark controls used in each specific campaign, see the main document to which this appendix is attached.

*Isolation of RNA and GPS quantification using Nanostring nCounter system*

RNA isolation from lysed cells is performed using commercially available kits (Direct-Zol RNA MiniPrep, Zymo Research, Irvine, CA). Total RNA is quantified and quality controlled using BioAnalyzer equipment (Agilent, Santa Clara, CA). A total of 100ng of RNA is used as sample input in a hybridization assay with GPS specific reporter probe CodeSet (Nanostring, Seattle, WA). The hybridized RNA-CodeSet sample is prepared on chip using nCounter Prepstation and individual transcripts of the GPS is quantified using Nanostring Digital Analyzer (Nanostring).

*Data acquisition and normalization*

Raw data is exported from the Digital Analyzer and counts of individual transcripts of the GPS are single-chip normalized with a count per total counts algorithm. Normalized data consists of a S by V matrix, where S denotes the number of samples in the GARD campaign, and V denotes the number of quantified transcripts of the GPS.

*Data analysis - Generation of calibrated support vector machine decision values*

All further downstream analysis is performed using application-based software, developed in the open source statistical environment R. A support vector machine (SVM) is trained using historical data used for GPS establishment (Johansson et al., 2011). All samples from test substances and benchmark controls from the specific GARD campaign are predicted using the trained SVM, assigning each sample with a SVM decision value. The predictor performance is estimated by identification of the area under the receiver operating characteristic (ROC AUC) of the predicted class of benchmark controls. The full details of this methodology are the same as for Example 1.

*GARD classifications of test substance(s)*

The GARD prediction model is defined as follows:
If the mean decision value of all available biological replicates of a test substance is greater than zero, the test substance is classified as a sensitizer.

**Table A**

| Probe Set ID | Transcript ID | Transcript Description | Gene Title | Gene Symbol | |
|---|---|---|---|---|---|
| **Table A(i)** | | | | | |
| 8104 107 | ENST0000032 6754 /// BC111959 /// NM_173553 | Tripartite motif family-like protein 2 gene:ENSG00000179046 /// Homo sapiens tripartite motif family-like 2, mRNA (cDNA clone MGC:138164 IMAGE:8327427), complete cds. /// Homo sapiens tripartite motif family-like 2 (TRIML2), mRNA. | tripartite motif family-like 2 | TRIML2 | 1 |
| 7984 862 | ENST0000034 3932 /// M55053 /// NM_000761 | Isoform 2 of Cytochrome P450 1A2 gene: ENSG00000140505 /// Human cytochrome P-3-450 mRNA, complete cds. /// Homo sapiens cytochrome P450, family 1, subfamily A, polypeptide 2 (CYP1A2), mRNA. | cytochrome P450, family 1, subfamily A, polypeptide 2 | CYP1A2 | 2 |

(continued)

| Probe Set ID | Transcript ID | Transcript Description | Gene Title | Gene Symbol | |
|---|---|---|---|---|---|
| 8103 341 | ENST0000031 1277 /// ENST0000039 3836 /// ENST0000037 9248 /// ENST0000039 3834 /// AY690636 /// NM_00103958 0 | Isoform 1 of Microtubule-associated protein 9 gene: ENSG00000164114 /// Isoform 2 of Microtubule-associated protein 9 gene:ENSG00000164114 /// Putative uncharacterized protein MAP9 gene:ENSG00000164114 /// Putative uncharacterized protein MAP9 gene: ENSG00000164114 /// Homo sapiens ASAP mRNA, complete cds. /// Homo sapiens microtubule-associated protein 9 (MAP9), mRNA. | microtubule-associated protein 9 | MAP9 | 3 |
| 8021 468 | GENSCAN000 00031245 /// XM_00172247 2 | cdna:Genscan chromosome: NCBI36:18:55579 770: 55587249:1 /// PREDICTED: Homo sapiens similar to 40S ribosomal protein S26 (LOC100131971), mRNA. | similar to 40S ribosomal protein S26 | LOC1001 31971 | 4 |
| 8166 945 | GENSCAN000 00048751 /// ENST0000035 4794 | cdna:Genscan chromosome: NCBI36:X:440284 78:44030436: 1 /// cdna:pseudogene chromosome: NCBI36:X:440282 57:44030436:1 qene: ENSG00000 198414 | - | - | 5 |
| 8021 461 | ENST0000025 6857 /// BC004488 /// NM_00101251 2 /// NM_ 00101251 3 /// NM_002091 | gastrin-releasing peptide isoform 1 preproprotein qene: ENSG00000134443 /// Homo sapiens gastrin-releasing peptide, mRNA (cDNA clone MGC:10712 IMAGE:3936083), complete cds. /// Homo sapiens gastrin-releasing peptide (GRP), transcript variant 2, mRNA. /// Homo sapiens gastrin-releasing peptide (GRP), transcript variant 3, mRNA. /// Homo sapiens gastrin-releasing peptide (GRP), transcript variant 1, mRNA. | gastrin-releasing peptide | GRP | 6 |
| 8088 903 | GENSCAN000 00015233 /// ENST0000035 8162 | cdna:Genscan chromosome: NCBI36:3:755574 84:75756994:-1 /// cdna:pseudogene chromosome:NCBI36:3:757307 26:75731229:-1 qene: ENSG00000196454 | - | - | 7 |

(continued)

| Probe Set ID | Transcript ID | Transcript Description | Gene Title | Gene Symbol | |
|---|---|---|---|---|---|
| 8053 064 | ENST0000040 9969 /// ENST0000026 4089 /// ENST0000037 7668 /// AK001650 /// NM_018221 | cdna:known chromosome: NCBI36:2:742356 73:74259503:- 1 gene:ENSG00000114978 /// Isoform 1 of Mps one binder kinase activator-like 1B gene: ENSG00000114978 /// Putative uncharacterized protein MOBKL1B (Fragment) gene: ENSG00000114978 /// Homo sapiens cDNA FLJ10788 fis, clone NT2RP4000498, moderately similar to MOB1 PROTEIN. /// Homo sapiens MOB1, Mps One Binder kinase activator-like 1B (yeast) (MOBKL1B), mRNA. | MOB1, Mps One Binder kinase activator-like 1B (yeast) | MOBKL1B | 8 |
| 7945 130 | ENST0000041 1383 /// ENST0000038 6420 | ncrna:misc_RNA chromosome: NCBI36:11 :12678 2956: 126783275:1 gene: ENSG00000223315 /// ncrna: snRNA_pseudogene chromosome:NCBI36:11 :12678 2956:126783196:1 pene: ENSG00000209155 | - | - | 9 |
| 8160 260 | ENST0000038 0672 /// ENST0000038 0667 /// ENST0000038 0666 /// AY438376 /// NM_017637 | Isoform 1 of Zinc finger protein basonuclin-2 gene: ENSG00000173068 /// Basonuclin 2 gene: ENSG00000173068 /// Isoform 2 of Zinc finger protein basonuclin- 2 gene:ENSG00000173068 /// Homo sapiens basonuclin2 mRNA, complete cds. /// Homo sapiens basonuclin 2 (BNC2), mRNA. | basonuclin 2 | BNC2 | 10 |

(continued)

| Probe Set ID | Transcript ID | Transcript Description | Gene Title | Gene Symbol | |
|---|---|---|---|---|---|
| 7934 708 | ENST0000037 2329 /// ENST0000037 2327 /// ENST0000037 2325 /// ENST0000039 8636 /// ENST0000037 2316 /// ENST0000037 2313 /// ENST0000037 2308 /// ENST0000039 4569 /// AK298029 /// AK298034 /// BC157866 /// BC157890 /// NM 006926 /// NM_ 00109866 8 /// NM_00109377 0 /// NM_005411 | Pulmonary surfactant-associated protein A2 qene: ENSG00000182314 /// cDNA FLJ54288, moderately similar to Pulmonary surfactant-associated protein A1 gene: ENSG00000185303 /// Pulmonary surfactant-associated protein A1 gene: ENSG00000185303/// Pulmonary surfactant-associated protein A1 gene: ENSG00000185303 /// Pulmonary surfactant-associated protein A2 gene: ENSG00000122854 /// cDNA FLJ54288, moderately similar to Pulmonary surfactant-associated protein A1 gene: ENSG00000122852 /// Pulmonary surfactant-associated protein A1 gene: ENSG00000122852 /// Pulmonary surfactant-associated protein A1 gene: ENSG00000122852 /// sapiens cDNA FLJ51913 complete cds, highly similar to Pulmonary surfactant-associated protein A1 precursor. /// Homo sapiens cDNA FLJ50593 complete cds, moderately similar to Pulmonary surfactant-associated protein A1 precursor. /// Homo sapiens surfactant protein A2B, mRNA (cDNA clone MGC:189761 IMAGE:9057085), complete cds. /// Homo sapiens surfactant protein A2B, mRNA (cDNA clone MGC:189714 IMAGE:8862711), complete cds. /// Homo sapiens surfactant protein A2B (SFTPA2B), mRNA. /// Homo sapiens surfactant protein A2 (SFTPA2), mRNA. /// Homo sapiens surfactant protein A1 (SFTPA1), mRNA. /// Homo sapiens surfactant protein A1 B (SFTPA1B), mRNA. | surfactant protein A1 /// surfactant protein A1B /// surfactant protein A2 /// surfactant protein A2B | SFTPA1 /// SFTPA1 B /// SFTPA2 /// SFTPA2B | 11 |

(continued)

| Probe Set ID | Transcript ID | Transcript Description | Gene Title | Gene Symbol | |
|---|---|---|---|---|---|
| 8068 046 | ENST0000040 7713 /// AF304442 | B lymphocyte activation-related protein BC-1514 gene: ENSG00000219130 /// Homo sapiens B lymphocyte activation-related protein BC-1514 mRNA, complete cds. | chromosome 21 open reading frame 118 | C21orf118 | 12 |
| 8131 301 | ENST0000036 5169 | ncrna:snRNA chromosome: NCBI36:7:518621 8:5186319:1 gene:ENSG00000202039 | - | - | 13 |
| Table A(ii) | | | | | |

(continued)

| Probe Set ID | Transcript ID | Transcript Description | Gene Title | Gene Symbol | |
|---|---|---|---|---|---|
| 7934 698 | ENST0000037 2329 /// ENST0000037 2327 /// ENST0000037 2325 /// ENST0000039 8636 /// ENST0000037 2316/// ENST0000037 2313 /// ENST0000037 2308 /// ENST0000039 4569 /// AK298029 /// AK298034 /// BC157866 /// BC157890 /// NM_006926 /// NM_00109866 8 /// NM_ 00109377 0 /// NM_005411 | Pulmonary surfactant-associated protein A2 gene: ENSG00000182314 /// cDNA FLJ54288, moderately similar to Pulmonary surfactant-associated protein A1 gene: ENSG00000185303 /// Pulmonary surfactant-associated protein A1 gene: ENSG00000185303 /// Pulmonary surfactant-associated protein A1 gene: ENSG00000185303 /// Pulmonary surfactant-associated protein A2 gene: ENSG00000122854 /// cDNA FLJ54288, moderately similar to Pulmonary surfactant-associated protein A1 gene: ENSG00000122852 /// Pulmonary surfactant-associated protein A1 gene: ENSG00000122852 /// Pulmonary surfactant-associated protein A1 gene: ENSG00000122852 /// sapiens cDNA FLJ51913 complete cds, highly similar to Pulmonary surfactant-associated protein A1 precursor. /// Homo sapiens cDNA FLJ50593 complete cds, moderately similar to Pulmonary surfactant-associated protein A1 precursor. /// Homo sapiens surfactant protein A2B, mRNA (cDNA clone MGC:189761 IMAGE:9057085), complete cds. /// Homo sapiens surfactant protein A2B, mRNA (cDNA clone MGC:189714 IMAGE:8862711), complete cds. /// Homo sapiens surfactant protein A2B (SFTPA2B), mRNA. /// Homo sapiens surfactant protein A2 (SFTPA2), mRNA. /// Homo sapiens surfactant protein A1 (SFTPA1), mRNA. /// Homo sapiens surfactant protein A1 B (SFTPA1B), mRNA. | surfactant protein A1 /// surfactant protein A1B /// surfactant protein A2 /// surfactant protein A2B | SFTPA1 /// SFTPA1 B /// SFTPA2 /// SFTPA2B | 14 |

(continued)

| Probe Set ID | Transcript ID | Transcript Description | Gene Title | Gene Symbol | |
|---|---|---|---|---|---|
| 7916 882 | ENST0000026 2340 /// U18991 /// NM_000329 | Retinal pigment epithelium-specific 65 kDa protein gene: ENSG00000116745 /// Human retinal pigment epithelium-specific 61 kDa protein (RPE65) mRNA, complete cds. /// Homo sapiens retinal pigment epithelium-specific protein 65kDa (RPE65), mRNA. | retinal pigment epithelium-specific protein 65kDa | RPE65 | 15 |
| 8149 324 | ENST0000028 4486 /// ENST0000039 8342 /// AL834122 /// NM_053279 | UPF0484 protein FAM167A gene:ENSG00000154319 /// FAM167A protein gene: ENSG00000154319 /// Homo sapiens mRNA; cDNA DKFZp761 F1821 (from clone DKFZp761 F1821). /// Homo sapiens family with sequence similarity 167, member A (FAM167A), mRNA. | family with sequence similarity 167, member A | FAM167A | 1 6 |
| 7914 992 | ENST0000038 7309 | ncrna:rRNA_pseudogene chromosome:NCBI36:1 :375028 86:37502974:-1 gene: ENSG00000210044 | - | - | 1 7 |
| 8021 774 | ENST0000040 5150 /// AK126293 | FLJ44313 protein gene: ENSG00000220032 /// Homo sapiens cDNA FLJ44313 fis, clone TRACH2025911. | FLJ44313 protein | FLJ44313 | 1 8 |
| 7965 573 | ENST0000034 4911 /// ENST0000034 3702 /// AF278532 /// NM_021229 | Isoform 2 of Netrin-4 gene: ENSG00000074527 /// Isoform 1 of Netrin-4 gene: ENSG00000074527 /// Homo sapiens beta-netrin mRNA, complete cds. /// Homo sapiens netrin 4 (NTN4), mRNA. | netrin 4 | NTN4 | 1 9 |
| 8057 771 | ENST0000035 8470 /// ENST0000039 2320 /// BC031212 /// NM_003151 | Signal transducer and activator of transcription 4 gene: ENSG00000138378 /// Signal transducer and activator of transcription 4 gene: ENSG00000138378 /// Homo sapiens signal transducer and activator of transcription 4, mRNA (cDNA clone MGC:39492 IMAGE:4830583), complete cds. /// Homo sapiens signal transducer and activator of transcription 4 (STAT4), mRNA. | signal transducer and activator of transcription 4 | STAT4 | 2 0 |

(continued)

| Probe Set ID | Transcript ID | Transcript Description | Gene Title | Gene Symbol | |
|---|---|---|---|---|---|
| 8111 417 | ENST0000034 2059 /// ENST0000029 6589 /// ENST0000038 2102 /// ENST0000034 5083 /// AF172849 /// NM_016180 /// NM_00101250 9 | Isoform AIM-1c of Membrane-associated transporter protein gene:ENSG00000164175 /// Isoform AIM-1a of Membrane-associated transporter protein gene:ENSG00000164175 /// membrane-associated transporter protein isoform b gene:ENSG00000164175 /// Isoform AIM-1b of Membrane-associated transporter protein gene:ENSG00000164175 /// Homo sapiens AIM-1 protein mRNA, complete cds. /// Homo sapiens solute carrier family 45, member 2 (SLC45A2), transcript variant 1, mRNA. /// Homo sapiens solute carrier family 45, member 2 (SLC45A2), transcript variant 2, mRNA. | solute carrier family 45, member 2 | SLC45A2 | 2 1 |
| 8161 381 | ENST0000031 6269 /// AK125850 /// AL833349 | hypothetical protein gene: ENSG00000204831 /// Homo sapiens cDNA FLJ43862 fis, clone TESTI4007775. /// Homo sapiens mRNA; cDNA DKFZp686P0734 (from clone DKFZp686P0734 ). | hypothetical protein LOC100133036 /// family with sequence similarity 95, member B1 | LOC1001 *33036* /// FAM95B1 | 22 |
| 8165 032 | ENST0000037 1763 /// AY336054 /// NM_182974 | Glycosyltransferase 6 domain-containing protein 1 gene: ENSG00000204007 /// Homo sapiens gycosyltransferase family 6 like-protein mRNA, complete cds. /// Homo sapiens glycosyltransferase 6 domain containing 1 (GLT6D1), mRNA. | glycosyltransferase 6 domain containing 1 | GLT6D1 | 23 |
| 8113 276 | AF119888 | Homo sapiens PRO2613 mRNA, complete cds. | - | --- | 24 |
| 8116 874 | ENST0000028 3141 /// ENST0000034 1041 /// AK128130 /// NM_00104027 4 | Isoform 1 of Synaptonemal complex protein 2-like gene: ENSG00000153157 /// Isoform 1 of Synaptonemal complex protein 2-like gene:ENSG00000153157 /// Homo sapiens cDNA FLJ46251 fis, clone TESTI4021713, weakly similar to Homo sapiens synaptonemal complex protein 2 (SYCP2). /// Homo sapiens synaptonemal complex protein 2-like (SYCP2L), mRNA. | synaptonemal complex protein 2-like | SYCP2L | 25 |

(continued)

| Probe Set ID | Transcript ID | Transcript Description | Gene Title | Gene Symbol | |
|---|---|---|---|---|---|
| 8030 753 | ENST0000032 6003 /// ENST0000032 6052 /// ENST0000036 0617 /// BC005307 /// NM_001648 /// NM_00103004 7 /// NM_ 00103004 8 /// NM_00103004 9 /// NM_ 00103005 0 | Prostate-specific antigen gene: ENSG00000142515 /// prostate specific antigen isoform 5 preproprotein gene: ENSG00000142515 /// prostate specific antigen isoform 3 preproprotein gene: ENSG00000142515 /// Homo sapiens kallikrein-related peptidase 3, mRNA (cDNA clone MGC:12378 IMAGE:3950475), complete cds. /// Homo sapiens kallikrein-related peptidase 3 (KLK3), transcript variant 1, mRNA. /// Homo sapiens kallikrein-related peptidase 3 (KLK3), transcript variant 3, mRNA. /// Homo sapiens kallikrein-related peptidase 3 (KLK3), transcript variant 4, mRNA. /// Homo sapiens kallikrein-related peptidase 3 (KLK3), transcript variant 5, mRNA. /// Homo sapiens kallikrein-related peptidase 3 (KLK3), transcript variant 6, mRNA. | kallikrein-related peptidase 3 | KLK3 | 26 |
| 8098 439 | GENSCANOOO 00042517 | cdna:Genscan chromosome: NCBI36:4:182680 810: 182745578:1 | --- | --- | 27 |
| 8076 819 | ENST0000038 0990 /// AK128136 | Conserved hypothetical protein gene:ENSG00000205634 /// Homo sapiens cDNA FLJ46257 fis, clone TESTI4024240. | FLJ46257 protein | RP11-191L9.1 | 28 |
| 7957 819 | ENST0000039 2989 /// ENST0000032 3346 /// AK128319 /// NM_139319 | Isoform 2 of Vesicular glutamate transporter 3 gene: ENSG00000179520 /// Isoform 1 of Vesicular glutamate transporter 3 gene:ENSG00000179520 /// Homo sapiens cDNA FLJ46460 fis, clone THYMU3021404, highly similar to Homo sapiens solute carrier family 17 (sodium-dependent inorganic phosphate cotransporter), member 8 (SLC17A8), mRNA. /// Homo sapiens solute carrier family 17 (sodium-dependent inorganic phosphate cotransporter), member 8 (SLC17A8), mRNA. | solute carrier family 17 (sodium-dependent inorganic phosphate cotransporter), member 8 | SLC17A8 | 29 |

(continued)

| Probe Set ID | Transcript ID | Transcript Description | Gene Title | Gene Symbol | |
|---|---|---|---|---|---|
| 7986 229 | ENST0000026 8164 /// ENST0000037 8973 /// BC096202 /// NM_006011 | Alpha-2,8-sialyltransferase 8B gene:ENSG00000140557 /// ST8SIA2 protein gene: ENSG00000140557 /// Homo sapiens ST8 alpha-N-acetyl-neuraminide alpha-2,8-sialyltransferase 2, mRNA (cDNA clone MGC:116854 IMAGE: 40004644), complete cds. /// Homo sapiens ST8 alpha-N-acetyl-neuram inide alpha-2,8-sialyltransferase 2 (ST8SIA2), mRNA. | ST8 alpha-N-acetyl-neuraminide alpha-2,8-sialyltransferase 2 | ST8SIA2 | 30 |
| 7995 310 | ENST0000031 9817 | Putative uncharacterized protein MGC3480 ciene: ENSG00000179755 | --- | --- | 31 |
| 8138 920 | ENST0000038 7801 /// ENST0000038 7652 /// ENST0000038 7676 /// ENST0000038 7734 /// ENST0000038 6042 | ncrna:snRNA_pseudogene chromosome:NCBI36:12:62305 744:62305830:-1 gene: ENSG00000210536 /// ncrna: snRNA_pseudogene chromosome:NCBI36:7:297014 50:29701536:1 gene: ENSG00000210387 /// ncrna: snRNA_pseudogene chromosome:NCBI36:7:327244 96:32724582:-1 gene: ENSG00000210411 /// ncrna: snRNA_pseudogene chromosome:NCBI36:7: 35155590:35155669:-1 gene: ENSG00000210469 /// ncrna: snRNA_pseudogene chromosome:NCBI36:7:102669 899:102669985:-1 gene: ENSG00000208777 | --- | --- | 32 |
| 8055 492 | ENST0000038 5544 | ncrna:Mt_tRNA_pseudogene chromosome:NCBI36:2: 140697 147:140697215:-1 gene: ENSG00000208279 | --- | --- | 33 |
| 7995 674 | ENST0000029 0552 /// AK125053 /// NM_024335 | Iroquois-class homeodomain protein IRX-6 gene: ENSG00000159387 /// Homo sapiens cDNA FLJ43063 fis, clone BRTHA3008310, moderately similar to Mus musculus mRNA for iroquois homeobox protein 6. /// Homo sapiens iroquois homeobox 6 (IRX6), mRNA. | iroquois homeobox 6 | IRX6 | 34 |

(continued)

| Probe Set ID | Transcript ID | Transcript Description | Gene Title | Gene Symbol | |
|---|---|---|---|---|---|
| 8155 627 | ENST0000031 6269 /// AK125850 /// AL833349 | hypothetical protein gene: ENSG00000204831 /// Homo sapiens cDNA FLJ43862 fis, clone TESTI4007775. /// Homo sapiens mRNA; cDNA DKFZp686P0734 (from clone DKFZp686P0734 ). | hypothetical protein LOC100133036 /// family with sequence similarity 95, member B1 | LOC1001 33036 /// FAM95B1 | 35 |
| 7941 608 | GENSCAN000 00024384 /// ENST0000036 4863 | cdna:Genscan chromosome: NCBI36:11 :65945 227: 65956926:1 /// ncrna:snoRNA chromosome:NCBI36:11 :65956 813:65956949: 1 gene: ENSG00000201733 | --- | --- | 36 |
| 7957 495 | ENST0000036 2375 | ncrna:misc_RNA chromosome: NCBI36:12:87348 350: 87348458: 1 gene: ENSG00000199245 | --- | --- | 37 |
| 7961 413 | ENST0000031 8426 /// BC101220 /// NM_182558 | Putative uncharacterized protein C12orf36 gene: ENSG00000180861 /// Homo sapiens chromosome 12 open reading frame 36, mRNA (cDNA clone MGC:120138 IMAGE: 40022214), complete cds. /// Homo sapiens chromosome 12 open reading frame 36 (C12orf36), mRNA. | chromosome 12 open reading frame 36 | C12orf36 | 38 |
| 8055 941 | ENST0000032 5926 /// BC002908 /// NM_019845 | Protein reprimo gene: ENSG00000177519 /// Homo sapiens reprimo, TP53 dependent G2 arrest mediator candidate, mRNA (cDNA clone MGC:11260 IMAGE:3942270), complete cds. /// Homo sapiens reprimo, TP53 dependent G2 arrest mediator candidate (RPRM), mRNA. | reprimo, TP53 dependent G2 arrest mediator candidate | RPRM | 39 |
| 7962 792 | ENST0000031 0248 /// NM_ 00100413 4 | Olfactory receptor 10AD1 gene: ENSG00000172640 /// Homo sapiens olfactory receptor, family 10, subfamily AD, member 1 (OR10AD1), mRNA. | olfactory receptor, family 10, subfamily AD, member 1 | OR10AD1 | 40 |
| 7896 756 | ENST0000032 6734 /// BC118644 | similar to hCG1735895 gene: ENSG00000177757 /// Homo sapiens cDNA clone IMAGE: 40030978. | --- | --- | 41 |
| 8054 939 | ENST0000041 1186 | ncrna:misc_RNA chromosome: NCBI36:2:124343 303: 124343617:-1 gene: ENSG00000223118 | --- | --- | 42 |

(continued)

| Probe Set ID | Transcript ID | Transcript Description | Gene Title | Gene Symbol | |
|---|---|---|---|---|---|
| 7976 057 | ENST0000038 7641 | ncrna:snoRNA_pseudogene chromosome:NCBI36:14:81998 185:81998284:1 gene: ENSG0000021 0376 | --- | --- | 43 |
| 7940 002 | ENST0000033 2362 /// BC136737 /// NM_00100521 0 | Leucine rich repeat containing 55 gene:ENSG00000183908 /// Homo sapiens leucine rich repeat containing 55, mRNA (cDNA clone MGC:168350 IMAGE: 9020727), complete cds. /// Homo sapiens leucine rich repeat containing 55 (LRRC55), mRNA. | leucine rich repeat containing 55 | LRRC55 | 44 |
| 7979 204 | ENST0000039 5631 /// ENST0000034 3279 /// ENST0000034 1590 /// Z24725 /// NM_006832 /// NM_00113499 9 /// NM_ 00113500 0 | Isoform 1 of Fermitin family homolog 2 gene: ENSG00000073712 /// fermitin family homolog 2 isoform 2 gene: ENSG00000073712 /// Isoform 1 of Fermitin family homolog 2 gene:ENSG00000073712 /// H.sapiens mitogen inducible gene mig-2, complete CDS. /// Homo sapiens fermitin family homolog 2 (Drosophila) (FERMT2), transcript variant 1, mRNA. /// Homo sapiens fermitin family homolog 2 (Drosophila) (FERMT2), transcript variant 2, mRNA. /// Homo sapiens fermitin family homolog 2 (Drosophila) (FERMT2), transcript variant 3, mRNA. | fermitin family homolog 2 (Drosophila) | FERMT2 | 45 |
| 7981 190 | AL834311 | Homo sapiens mRNA; cDNA DKFZp434O1614 (from clone DKFZp434O1614). | hypothetical LOC100130815 | LOC1001 30815 | 46 |
| 8092 686 | ENST0000035 8241 /// BC068081 /// NM_001004312 | Receptor-transporting protein 2 gene:ENSG00000198471 /// Homo sapiens receptor (chemosensory) transporter protein 2, mRNA (cDNA clone MGC:78665 IMAGE:6212901), complete cds. /// Homo sapiens receptor (chemosensory) transporter protein 2 (RTP2), mRNA. | receptor (chemosensory) transporter protein 2 | RTP2 | 47 |

(continued)

| Probe Set ID | Transcript ID | Transcript Description | Gene Title | Gene Symbol | |
|---|---|---|---|---|---|
| 7961 604 | ENST0000026 6505 /// ENST0000031 8197 /// AY035866 /// NM_033123 | Isoform 1 of 1-phosphatidylinositol-4,5-bisphosphate phosphodiesterase zeta-1 gene:ENSG00000139151 /// cDNA FLJ40236 fis, clone TESTI2023214, weakly similar to 1-PHOSPHATIDYLINOSITOL-4,5-BISPHOSPHATE PHOSPHODIESTERASE DELTA 1 gene: ENSG00000139151 /// Homo sapiens testis-development related NYD-SP27 mRNA, complete cds. /// Homo sapiens phospholipase C, zeta 1 (PLCZ1), mRNA. | phospholipase C, zeta 1 | PLCZ1 | 48 |
| 8026 503 | ENST0000039 7365 /// ENST0000034 3017 /// NR_ 024335 /// NR_ 024336 | Putative uncharacterized protein FLJ25328 gene: ENSG00000167459 /// FLJ25328 protein (Fragment) gene: ENSG00000167459 /// Homo sapiens hypothetical LOC148231 (FLJ25328), transcript variant 1, non-coding RNA. /// Homo sapiens hypothetical LOC148231 (FLJ25328), transcript variant 2, non-coding RNA. | hypothetical LOC148231 | FLJ25328 | 49 |
| 8084 887 | ENST0000041 1400 /// ENST0000038 5589 | ncrna:misc_RNA chromosome: NCBI36:3:195346 160: 195346450:1 gene: ENSG00000223332 /// ncrna: scRNA_pseudogene chromosome:NCBI36:3: 195346 161:195346454:1 gene: ENSG00000208324 | --- | --- | 50 |
| 7904 417 | GENSCANOOO 00027599/// ENST0000028 6193 | cdna:Genscan chromosome: NCBI36:1 :119806 478: 119858791:1 /// cdna: pseudogene chromosome: NCBI36:1 :119811 367: 119817360:1 qene: ENSG00000 187481 | --- | --- | 51 |
| 8112 666 | GENSCANOOO 00026551 /// ENST0000032 9491 | cdna:Genscan chromosome: NCBI36:5:743191 49:74349847:-1 /// cdna:pseudogene chromosome:NCBI36:5:743217 74:74322218:-1 qene: ENSG00000182383 | --- | --- | 52 |

(continued)

| Probe Set ID | Transcript ID | Transcript Description | Gene Title | Gene Symbol | |
|---|---|---|---|---|---|
| 8036 969 | ENST0000030 1141 /// ENST0000030 1146 /// ENST0000029 1764 /// ENST0000035 9667 /// ENST0000033 0436 /// AF209774 /// M33317 /// M33318 /// NM_ 000764 /// NM_ 000762 /// NM_ 000766 /// NM_ 030589 | Cytochrome P450 2A6 gene: ENSG00000213052 /// Cytochrome P450 2A7 gene: ENSG00000198077 /// cytochrome P450, family 2, subfamily A, polypeptide 7 isoform 2 gene: ENSG00000198077 /// cdna: known chromosome:NCBI36: 19: 46107 659:46108594:-1 gene: ENSG00000198251 /// Cytochrome P450 2A13 gene: ENSG00000197838 /// Homo sapiens cytochrome P450 2A13 (CYP2A13)mRNA, complete cds. /// Human cytochrome P450IIA4 (CYP2A4) mRNA, complete cds. /// Human cytochrome P450IIA3 (CYP2A3) mRNA, complete cds. /// Homo sapiens cytochrome P450, family 2, subfamily A, polypeptide 7 (CYP2A7), transcript variant 1, mRNA. /// Homo sapiens cytochrome P450, family 2, subfamily A, polypeptide 6 (CYP2A6), mRNA. /// Homo sapiens cytochrome P450, family 2, subfamily A, polypeptide 13 (CYP2A13), mRNA. /// Homo sapiens cytochrome P450, family 2, subfamily A, polypeptide 7 (CYP2A7), transcript variant 2, mRNA. | cytochrome P450, family 2, subfamily A, polypeptide 13 /// cytochrome P450, family 2, subfamily A, polypeptide 7 /// cytochrome P450, family 2, subfamily A, polypeptide 6 | CYP2A13 /// CYP2A7 /// CYP2A6 | 53 |

(continued)

| Probe Set ID | Transcript ID | Transcript Description | Gene Title | Gene Symbol | |
|---|---|---|---|---|---|
| 8176 935 | ENST0000030 3804 /// ENST0000030 3728 /// ENST0000034 3584 /// ENST0000030 3593 /// ENST0000030 6589 /// ENST0000033 8673 /// AF000988 /// NM_00100275 8 /// NM_004676 | Isoform 1 of PTPN13-like protein, Y-linked gene: ENSG00000169807 /// Isoform 1 of PTPN13-like protein, Y-linked gene:ENSG00000169789 /// Isoform 1 of PTPN13-like protein, Y-linked gene: ENSG00000169763 /// Isoform 2 of PTPN13-like protein, Y-linked gene:ENSG00000169763 /// Isoform 1 of PTPN13-like protein, Y-linked gene: ENSG00000172283 /// Isoform 2 of PTPN13-like protein, Y-linked gene:ENSG00000172283 /// Homo sapiens testis-specific PTP-BL Related Y protein (PRY) mRNA, complete cds. /// Homo sapiens PTPN13-like, Y-linked 2 (PRY2), mRNA. /// Homo sapiens PTPN13-like, Y-linked (PRY), mRNA. | PTPN13-like, Y-linked /// PTPN13-like, Y-linked 2 | PRY /// PRY2 | 54 |
| 8044 124 | ENST0000025 8456 /// U92642 /// NM 007227 | High-affinity lysophosphatidic acid receptor homolog gene: ENSG00000135973 /// Human hiph-affinity lysophosphatidic acid receptor homolog mRNA, complete cds. /// Homo sapiens G protein-coupled receptor 45 (GPR45), mRNA. | G protein-coupled receptor 45 | GPR45 | 55 |
| 8070 930 | --- | --- | --- | --- | 56 |
| 8015 037 | AK095738 | Homo sapiens cDNA FLJ38419 fis, clone FEBRA2009846. | --- | --- | 57 |
| 7969 482 | ENST0000037 7462 | similar to hCG30005 gene: ENSG00000102794 | --- | --- | 58 |
| 8173 524 | ENST0000037 3619 /// ENST0000024 6139 /// U65092 /// NM_004143 | Cbp/p300-interacting transactivator 1 gene: ENSG00000125931 /// Cbp/p300-interacting transactivator 1 gene: ENSG00000125931 /// Human melanocyte-specific gene 1 (msg1) mRNA, complete cds. /// Homo sapiens Cbp/p300-interacting transactivator, with Glu/Asp-rich carboxy-terminal domain, 1 (CITED1), mRNA. | Cbp/p300-interacting transactivator, with Glu/Asp-rich carboxy-terminal domain, 1 | CITED1 | 59 |

(continued)

| Probe Set ID | Transcript ID | Transcript Description | Gene Title | Gene Symbol | |
|---|---|---|---|---|---|
| 8143 747 | ENST0000038 5536 | ncrna:tRNA_pseudogene chromosome:NCBI36:7:148966 447:148966513:-1 gene: ENSG00000208271 | - | - | 60 |
| 8080 781 | ENST0000035 6151 /// ENST0000030 2779 /// ENST0000038 3715 /// ENST0000038 3716 /// AY274811 /// NM_017771 | Isoform 1 of PX domain-containing protein kinase-like protein gene: ENSG00000168297 /// Isoform 4 of PX domain-containing protein kinase-like protein gene: ENSG00000168297 /// Isoform 2 of PX domain-containing protein kinase-like protein gene: ENSG00000168297 /// Isoform 6 of PX domain-containing protein kinase-like protein gene: ENSG00000168297 /// Homo sapiens PX serine/threonine kinase mRNA, complete cds. /// Homo sapiens PX domain containing serine/threonine kinase (PXK), mRNA. | PX domain containing serine/threonine kinase | PXK | 61 |
| 8111 118 | ENST0000036 5399 | ncrna:snoRNA chromosome: NCBI36:5: 151638 95: 15164029:-1 qene: ENSG00000202269 | --- | --- | 62 |
| 7934 883 | ENST0000038 7878 | ncrna:scRNA_pseudogene chromosome:NCBI36:10:89582 555:89582827:-1 gene: ENSG00000210613 | --- | --- | 63 |

(continued)

| Probe Set ID | Transcript ID | Transcript Description | Gene Title | Gene Symbol | |
|---|---|---|---|---|---|
| 8010 113 | ENST0000037 4998 /// ENST0000037 4999 /// ENST0000030 1618 /// AB 114297 /// NM_ 144677 /// NM_ 198955 | Isoform 3 of Alpha-1,6-mannosylglycoprotein 6-beta-N-acetylglucosaminyltransferase B gene:ENSG00000167889 /// Isoform 1 of Alpha-1,6-mannosylglycoprotein 6-beta-N-acetylglucosaminyltransferase B gene:ENSG00000167889 /// beta (1,6)-N-acetylglucosaminyltransferase V isoform 1 gene: ENSG00000167889 /// Homo sapiens hGnTVb mRNA for UDP-N-acetylglucosamine: alpha1,6-D-mannoside beta1,6-N-acetylglucosaminyltransferase b, complete cds. /// Homo sapiens mannosyl (alpha-1,6-)-glycoprotein beta-1,6-N-acetyl-glucosaminyltransferase, isozyme B (MGAT5B), transcript variant 1, mRNA. /// Homo sapiens mannosyl (alpha-1,6-)-glycoprotein beta-1,6-N-acetyl-glucosaminyltransferase, isozyme B (MGAT5B), transcript variant 2, mRNA. | mannosyl (alpha-1,6-)-glycoprotein beta-1,6-N-acetyl-glucosaminyltransferase, isozyme B | MGAT5B | 64 |
| 8175 524 | ENST0000037 0540 /// NM_ 00101340 3 | Uncharacterized protein LOC347487 gene: ENSG00000203933 /// Homo sapiens hypothetical LOC347487 (LOC347487), mRNA. | hypothetical LOC347487 | RP11-35F15.2 | 65 |
| 7997 740 | ENST0000026 8607 /// BC045759 /// NM_022818 | Microtubule-associated proteins 1A/1B light chain 3B gene: ENSG00000140941 /// Homo sapiens microtubule-associated protein 1 light chain 3 beta, mRNA (cDNA clone MGC:48651 IMAGE:4828857), complete cds. /// Homo sapiens microtubule-associated protein 1 light chain 3 beta (MAP1LC3B), mRNA. | microtubule-associated protein 1 light chain 3 beta | MAP1LC3 B | 66 |

(continued)

| Probe Set ID | Transcript ID | Transcript Description | Gene Title | Gene Symbol | |
|---|---|---|---|---|---|
| 8177 395 | ENST0000030 3804 /// ENST0000030 3728 /// ENST0000034 3584 /// ENST0000030 3593 /// ENST0000030 6589 /// ENST0000033 8673 /// AF000988 /// NM_00100275 8 /// NM 004676 | Isoform 1 of PTPN13-like protein, Y-linked gene: ENSG00000169807 /// Isoform 1 of PTPN13-like protein, Y-linked gene:ENSG00000169789 /// Isoform 1 of PTPN13-like protein, Y-linked gene: ENSG00000169763 /// Isoform 2 of PTPN13-like protein, Y-linked gene:ENSG00000169763 /// Isoform 1 of PTPN13-like protein, Y-linked gene: ENSG00000172283 /// Isoform 2 of PTPN13-like protein, Y-linked gene:ENSG00000172283 /// Homo sapiens testis-specific PTP-BL Related Y protein (PRY) mRNA, complete cds. /// Homo sapiens PTPN13-like, Y-linked 2 (PRY2), mRNA. /// Homo sapiens PTPN13-like, Y-linked (PRY), mRNA. | PTPN13-like, Y-linked /// PTPN13-like, Y-linked 2 | PRY /// PRY2 | 67 |
| 7929 478 | ENST0000037 1321 /// M61854 /// NM_000769 | Cytochrome P450 2C19 gene: ENSG00000165841 /// Human cytochrome P4502C19 (CYP2C19) mRNA, clone 11a. /// Homo sapiens cytochrome P450, family 2, subfamily C, polypeptide 19 (CYP2C19), mRNA. | cytochrome P450, family 2, subfamily C, polypeptide 19 | CYP2C19 | 68 |
| 8102 781 | GENSCAN000 00015129 /// XR_ 016991 | cdna:Genscan chromosome: NCBI36:4:132863 736: 133029672:-1 /// PREDICTED: Homo sapiens similar to hCG2026352 (LOC646187), mRNA. | similar to hCG2026352 | LOC6461 87 | 69 |
| 8155 026 | ENST0000032 9395 /// BC031276 /// NR_003581 /// NR_003582 | Putative FetA-like protein gene: ENSG00000179766 /// Homo sapiens ATPase, Class I, type 8B family pseudogene, mRNA (cDNA clone MGC:39768 IMAGE:5295199), complete cds. /// Homo sapiens ATPase, Class I, type 8B family pseudogene (LOC158381), transcript variant 1, non-coding RNA. /// Homo sapiens ATPase, Class I, type 8B family pseudogene (LOC158381), transcript variant 2, non-coding RNA. | ATPase, Class I, type 8B family pseudogene | LOC1583 81 | 7 0 |

(continued)

| Probe Set ID | Transcript ID | Transcript Description | Gene Title | Gene Symbol | |
|---|---|---|---|---|---|
| 8146 914 | ENST0000027 6603 /// ENST0000027 6602 /// U74382 /// NM_003218 /// NM_017489 | Isoform 1 of Telomeric repeat-binding factor 1 gene: ENSG00000147601 /// Isoform 2 of Telomeric repeat-binding factor 1 gene:ENSG00000147601 /// Human telomeric repeat DNA-binding protein (PIN2) mRNA, complete cds. /// Homo sapiens telomeric repeat binding factor (NIMA-interacting) 1 (TERF1), transcript variant 2, mRNA. /// Homo sapiens telomeric repeat binding factor (NIMA-interacting) 1 (TERF1), transcript variant 1, mRNA. | telomeric repeat binding factor (NIMA-interacting) 1 | TERF1 | 71 |
| 8169 022 | ENST0000037 2661 /// ENST0000037 2656 /// BC023544 /// NM 001006612 /// NM_016303/// NM_00100661 4 /// NM_ 00100661 3 | WW domain-binding protein 5 gene:ENSG00000185222 /// WW domain-binding protein 5 gene: ENSG00000185222 /// Homo sapiens WW domain binding protein 5, mRNA (cDNA clone MGC:15211 IMAGE:4122244), complete cds. /// Homo sapiens WW domain binding protein 5 (WBP5), transcript variant 2, mRNA. /// Homo sapiens WW domain binding protein 5 (WBP5), transcript variant 1, mRNA. /// Homo sapiens WW domain binding protein 5 (WBP5), transcript variant 4, mRNA. /// Homo sapiens WW domain binding protein 5 (WBP5), transcript variant 3, mRNA. | WW domain binding protein 5 | WBP5 | 72 |
| 7944 952 | ENST0000034 0456 /// AK128036 | cdna:known chromosome: NCBI36:11 :12448 8267: 124501887:1 gene: ENSG00000187686 /// Homo sapiens cDNA FLJ46155 fis, clone TESTI4001517. | --- | --- | 73 |
| 8108 199 | BC025747 | Homo sapiens similar to CG4995 gene product, mRNA (cDNA clone MGC:35539 IMAGE: 5200129), complete cds. | similar to CG4995 gene product | LOC1533 28 | 74 |

(continued)

| Probe Set ID | Transcript ID | Transcript Description | Gene Title | Gene Symbol | |
|---|---|---|---|---|---|
| 8040 618 | ENST0000026 4710 /// AK023223 /// NM_016131 | Ras-related protein Rab-10 gene: ENSG00000084733 /// Homo sapiens cDNA FLJ13161 fis, clone NT2RP3003589, similar to Homo sapiens ras-related GTP-binding protein mRNA. /// Homo sapiens RAB10, member RAS oncogene family (RAB10), mRNA. | RAB10, member RAS oncogene family | RAB10 | 75 |
| 7981 326 | hsa-mir-1247 /// hsa-mir-1247 /// AF305836 /// AF469206 /// BC065701 | MI0006382 Homo sapiens miR-1247 stem-loop /// MI0006382 Homo sapiens miR-1247 stem-loop /// Homo sapiens uterine-derived 14 kDa protein mRNA, complete cds. /// Homo sapiens clone 8 DIO3AS mRNA, partial sequence; alternatively spliced. /// Homo sapiens deiodinase, iodothyronine, type III opposite strand, mRNA (cDNA clone IMAGE:6205020). | DIO3 opposite strand (non-protein coding) | DIO3OS | 76 |
| 8057 004 | ENST0000035 8450 /// ENST0000028 6063 /// ENST0000038 9683 /// ENST0000040 9504 /// AB036704 /// NM_00107735 8 /// NM. 016953 /// NM_00107719 6 /// NM_ 00107719 7 | Isoform 2 of Dual 3',5'-cyclic-AMP and -GMP phosphodiesterase 11A gene:ENSG00000128655 /// Isoform 1 of Dual 3',5'-cyclic-AMP and -GMP phosphodiesterase 11A gene:ENSG00000128655 /// Isoform 4 of Dual 3',5'-cyclic-AMP and -GMP phosphodiesterase 11A gene:ENSG00000128655 /// cdna:known chromosome: NCBI36:2:178202 021: 178495768:-1 gene: ENSG00000128655 /// Homo sapiens HSPDE11A mRNA for phosphodiesterase 11A, complete cds. /// Homo sapiens phosphodiesterase 11A (PDE11A), transcript variant 2, mRNA. /// Homo sapiens phosphodiesterase 11A (PDE11A), transcript variant 4, mRNA. /// Homo sapiens phosphodiesterase 11A (PDE11A), transcript variant 1, mRNA. /// Homo sapiens phosphodiesterase 11A (PDE11A), transcript variant 3, mRNA. | phosphodiesterase 11A | PDE11A | 77 |

(continued)

| Probe Set ID | Transcript ID | Transcript Description | Gene Title | Gene Symbol | |
|---|---|---|---|---|---|
| 8108 370 | ENST0000023 9938 /// M62829 /// NM_001964 | Early growth response protein 1 gene:ENSG00000120738 /// Human transcription factor ETR103 mRNA, complete cds. /// Homo sapiens early growth response 1 (EGR1), mRNA. | early growth response 1 | EGR1 | 78 |
| 8159 078 | ENST0000031 6948 /// ENST0000029 1722 /// AK074852 /// NM_017586 /// NM_00113577 5 | Isoform 1 of Transmembrane protein C9orf7 gene: ENSG00000160325 /// Isoform 2 of Transmembrane protein C9orf7 gene:ENSG00000160325 /// Homo sapiens cDNA FLJ90371 fis, clone NT2RP2004524. /// Homo sapiens chromosome 9 open reading frame 7 (C9orf7), transcript variant 1, mRNA. /// Homo sapiens chromosome 9 open reading frame 7 (C9orf7), transcript variant 2, mRNA. | chromosome 9 open reading frame 7 | C9orf7 | 79 |
| 8035 956 | ENST0000036 5097 | ncrna:misc_RNA chromosome: NCBI36:19:38045 533: 38045838:-1 gene: ENSG00000201967 | --- | --- | 80 |
| 7982 868 | ENST0000024 9798 /// ENST0000039 7434 /// BC019625 /// NM_024111 | Cation transport regulator-like protein 1 gene: ENSG00000128965 /// Cation transport protein-like protein gene:ENSG00000128965 /// Homo sapiens ChaC, cation transport regulator homolog 1 (E. coli), mRNA (cDNA clone MGC: 24988 IMAGE:4473135), complete cds. /// Homo sapiens ChaC, cation transport regulator homolog 1 (E. coli) (CHAC1), mRNA. | ChaC, cation transport regulator homolog 1 (E. coli) | CHAC1 | 81 |
| 8084 878 | ENST0000038 4640 | ncrna:misc_RNA chromosome: NCBI36:3:194824672: 194824784:1 qene: ENSG00000207370 | --- | --- | 82 |

(continued)

| Probe Set ID | Transcript ID | Transcript Description | Gene Title | Gene Symbol | |
|---|---|---|---|---|---|
| 7969 959 | ENST0000037 5936 /// ENST0000032 9625 /// DQ343761 /// NM_172370 | Isoform 1 of D-amino acid oxidase activator gene: ENSG00000182346 /// Putative uncharacterized protein DAOA gene:ENSG00000182346 /// Homo sapiens schizophrenia and bipolar disorder associated protein G72 form A mRNA, complete cds, alternatively spliced. /// Homo sapiens D-amino acid oxidase activator (DAOA), mRNA. | D-amino acid oxidase activator | DAOA | 83 |
| 8137 330 | ENST0000035 6058 /// AK128129 | cDNA FLJ46250 fis, clone TESTI4021569, moderately similar to ATP-binding cassette, sub-family B, member 8, mitochondrial gene: ENSG00000197150 /// Homo sapiens cDNA FLJ46250 fis, clone TESTI4021569, moderately similar to ATP-binding cassette, sub-family B, member 8, mitochondrial precursor. | --- | --- | 84 |
| 8160 383 | ENST0000025 9555 /// ENST0000038 0220 /// ENST0000023 9347 /// M34913 /// V00542 /// NM_021057 /// NM_002172 | Interferon alpha-14 gene: ENSG00000186809 /// Interferon alpha-14 gene: ENSG00000186809 /// Interferon alpha-7 gene: ENSG00000214042 /// Human interferon-alpha-J1 (IFN-alpha-J1) mRNA, complete cds. /// Messenger RNA for human leukocyte (alpha) interferon. /// Homo sapiens interferon, alpha 7 (IFNA7), mRNA. /// Homo sapiens interferon, alpha 14 (IFNA14), mRNA. | interferon, alpha 7 /// interferon, alpha 14 | IFNA7 /// IFNA14 | 85 |

(continued)

| Probe Set ID | Transcript ID | Transcript Description | Gene Title | Gene Symbol | |
|---|---|---|---|---|---|
| 8071 168 | ENST0000034 2005 /// ENST0000032 9949 /// ENST0000040 2027 /// ENST0000024 8992 /// AK292412 /// AK302597 /// AY358961 /// NR_003714 | cDNA FLJ60978, weakly similar to Nuclear envelope pore membrane protein POM 121 gene:ENSG00000182356 /// Putative uncharacterized protein ENSP00000383394 gene: ENSG00000217261 /// POM121-like 1 protein gene: ENSG00000183169 /// POM121-like gene:ENSG00000128262 /// Homo sapiens cDNA FLJ76724 complete cds. /// Homo sapiens cDNA FLJ60978 complete cds, weakly similar to Nuclear envelope pore membrane protein POM 121. /// Homo sapiens clone DNA107786 POM121-like (UNQ2565) mRNA, complete cds. /// Homo sapiens POM121-like protein (DKFZP434P211), non-coding RNA. | POM121 membrane glycoprotein-like 1 (rat) /// POM121 membrane glycoprotein-like 1 pseudogene /// POM121 membrane glycoprotein-like 1 pseudogene | POM121L 1 /// DKFZp43 4K191 /// DKFZP43 4P211 | 86 |
| 8067 965 | AF304443 | Homo sapiens B lymphocyte activation-related protein BC-2048 mRNA, complete cds. | --- | --- | 87 |
| 8175 589 | ENST0000036 4415 | ncrna:rRNA chromosome: NCBI36:X:146897 312: 146897427:-1 gene: ENSG0000020 1285 | --- | --- | 88 |
| 7935 990 | ENST0000040 6432 /// ENST0000002 0673 /// BC142643 /// NM_002779 | Isoform 1 of PH and SEC7 domain-containing protein 1 gene:ENSG00000059915 /// Isoform 1 of PH and SEC7 domain-containing protein 1 gene:ENSG00000059915 /// Homo sapiens pleckstrin and Sec7 domain containing, mRNA (cDNA clone MGC: 164849 IMAGE:40147927), complete cds. /// Homo sapiens pleckstrin and Sec7 domain containing (PSD), mRNA. | pleckstrin and Sec7 domain containing | PSD | 89 |
| 8080 138 | ENST0000033 3127 /// AK128883 /// NM_203424 | IQ domain-containing protein F2 gene: ENSG00000184345 /// Homo sapiens cDNA FLJ46915 fis, clone TESTI2014474. /// Homo sapiens IQ motif containing F2 (IQCF2), mRNA. | IQ motif containing F2 | IQCF2 | 90 |

(continued)

| Probe Set ID | Transcript ID | Transcript Description | Gene Title | Gene Symbol | |
|---|---|---|---|---|---|
| 7946 021 | ENST0000038 0369 /// NM_ 00100522 2 | Putative olfactory receptor 52A4 gene:ENSG00000205494 /// Homo sapiens olfactory receptor, family 52, subfamily A, member 4 (OR52A4), mRNA. | olfactory receptor, family 52, subfamily A, member 4 | OR52A4 | 91 |
| 7975 779 | ENST0000030 3562 /// BX647104 /// NM_005252 | Proto-oncogene protein c-fos gene:ENSG00000170345 /// Homo sapiens mRNA; cDNA DKFZp686J04124 (from clone DKFZp686J04124). /// Homo sapiens v-fos FBJ murine osteosarcoma viral oncogene homolog (FOS), mRNA. | v-fos FBJ murine osteosarcoma viral oncogene homolog | FOS | 9 2 |
| \7912 863 | ENST0000038 3728 /// ENST0000030 8124 /// ENST0000038 9184 /// ENST0000033 4998 /// AY192149 /// L11924 /// NR_ 002729 /// NM_ 020998 | Hepatocyte growth factor-like protein homolog gene: ENSG00000173531 /// macrophage stimulating 1 gene: ENSG00000173531 /// 64 kDa protein gene: ENSG00000186715 /// Isoform 2 of Putative macrophage-stimulating protein MSTP9 gene: ENSG00000186715 /// Homo sapiens brain-rescue-factor-1 mRNA, complete cds. /// Homo sapiens macrophage-stimulating protein (MST1 mRNA, complete cds. /// Homo sapiens macrophage stimulating, pseudogene 9 (MSTP9), non-coding RNA. /// Homo sapiens macrophage stimulating 1 (hepatocyte growth factor-like) (MST1), mRNA. | macrophage stimulating, pseudogene 9 /// macrophage stimulating 1 (hepatocyte growth factor-like) | MSTP9 /// MST1 | 93 |

(continued)

| Probe Set ID | Transcript ID | Transcript Description | Gene Title | Gene Symbol | |
|---|---|---|---|---|---|
| 8002 969 | ENST0000039 3350 /// ENST0000032 6043 /// BC081542 /// NM_005360 /// NM_00103180 4 | Isoform Short of Transcription factor Maf gene: ENSG00000178573 /// Isoform Long of Transcription factor Maf gene:ENSG00000178573 /// Homo sapiens v-maf musculoaponeurotic fibrosarcoma oncogene homolog (avian), mRNA (cDNA clone MGC:71685 IMAGE:30347784), complete cds. /// Homo sapiens v-maf musculoaponeurotic fibrosarcoma oncogene homolog (avian) (MAF), transcript variant 1, mRNA. /// Homo sapiens v-maf musculoaponeurotic fibrosarcoma oncogene homolog (avian) (MAF), transcript variant 2, mRNA. | v-maf musculoaponeurotic fibrosarcoma oncogene homolog (avian) | MAF | 94 |
| 7943 954 | ENST0000038 8431 /// ENST0000038 8445 | ncrna:scRNA_pseudogene chromosome:NCBI36:11 :11205 3855:112053924:-1 gene: ENSG00000211166 /// ncrna: scRNA_pseudogene chromosome:NCBI36:11 :11298 6610:112986679:1 gene: ENSG00000211180 | - | - | 95 |
| 8135 099 | ENST0000039 7927 /// ENST0000031 3669 /// BC110393 /// NM_133457 | Isoform 1 of Collagen alpha-1 (XXVI) chain gene: ENSG00000160963 /// Isoform 2 of Collagen alpha-1 (XXVI) chain gene:ENSG00000160963 /// Homo sapiens EMI domain containing 2, mRNA (cDNA clone MGC:117329 IMAGE:5195867), complete cds. /// Homo sapiens EMI domain containing 2 (EMID2), mRNA. | EMI domain containing 2 | EMID2 | 96 |

(continued)

| Probe Set ID | Transcript ID | Transcript Description | Gene Title | Gene Symbol | |
|---|---|---|---|---|---|
| 7978 801 | ENST0000039 9232 /// ENST0000039 9222 /// ENST0000035 7362 /// AY369208 /// NM_182830 /// NM_00111349 8 | MAM domain-containing glycosylphosphatidylinositol anchor protein 2 gene: ENSG00000139915 /// MAM domain containing 1 isoform 2 gene:ENSG00000139915 /// MAM domain containing 1 isoform 2 gene:ENSG00000139915 /// Homo sapiens MAM domain-containing glycosyl phosphatidyl inositol anchor 2 (MDGA2) mRNA, complete cds. /// Homo sapiens MAM domain containing glycosylphosphatidylinositol anchor 2 (MDGA2), transcript variant 2, mRNA. /// Homo sapiens MAM domain containing glycosyl phosphatidyl inositol anchor 2 (MDGA2), transcript variant 1, mRNA. | MAM domain containing glycosyl phosphatidyl i nositol anchor 2 | MDGA2 | 97 |
| 7971 661 | hsa-mir-15a /// hsa-mir-15a | MI0000069 Homo sapiens miR-15a stem-loop /// MI0000069 Homo sapiens miR-15a stem-loop | --- | --- | 98 |
| 8136 709 | ENST0000039 7504 /// BC111973 /// NR_003715 | Putative maltase-glucoamylase-like protein LOC93432 gene: ENSG00000214088 /// Homo sapiens maltase-glucoamylase-like pseudogene, mRNA (cDNA clone IMAGE:8327441), complete cds. /// Homo sapiens maltase-glucoamylase-like pseudogene (LOC93432), non-coding RNA. | maltase-glucoamylase-like pseudo gene | LOC9343 2 | 99 |
| 8139 367 | ENST0000028 9547 /// ENST0000038 1160 /// ENST0000038 1159 /// AF192522 /// NM_013389 /// NM_00110164 8 | Niemann-Pick C1-like protein 1 isoform 1 gene: ENSG00000015520 /// Isoform 2 of Niemann-Pick C1-like protein 1 gene:ENSG00000015520 /// Isoform 3 of Niemann-Pick C1-like protein 1 gene: ENSG00000015520 /// Homo sapiens Niemann-Pick C1-like protein 1 (NPC1L1) mRNA, complete cds. /// Homo sapiens NPC1 (Niemann-Pick disease, type C1, gene)-like 1 (NPC1L1), transcript variant 1, mRNA. /// Homo sapiens NPC1 (Niemann-Pick disease, type C1, gene)-like 1 (NPC1L1), transcript variant 2, mRNA. | NPC1 (Niemann-Pick disease, type C1, gene)-like 1 | NPC1L1 | 100 |

(continued)

| Probe Set ID | Transcript ID | Transcript Description | Gene Title | Gene Symbol | |
|---|---|---|---|---|---|
| 8055 952 | ENST0000033 9562 /// ENST0000040 9572 /// ENST0000040 9108 /// BC009288 /// NM_006186 | Nuclear receptor subfamily 4 group A member 2 gene: ENSG00000153234 /// cdna: known chromosome:NCBI36:2: 156889 856:156907106:-1 gene: ENSG00000153234 /// cdna: known chromosome:NCBI36:2: 156890 502:156895465:-1 gene: ENSG00000153234 /// Homo sapiens nuclear receptor subfamily 4, group A, member 2, mRNA (cDNA clone MGC:14354 IMAGE:4298967), complete cds. /// Homo sapiens nuclear receptor subfamily 4, group A, member 2 (NR4A2), mRNA. | nuclear receptor subfamily 4, group A, member 2 | NR4A2 | 101 |
| 7942 809 | BC008359 | Homo sapiens cDNA clone IMAGE:3606756. | --- | --- | 102 |
| 8120 151 | ENST0000024 4799 /// ENST0000039 3699 /// ENST0000037 1211 /// ENST0000039 3696 /// BX647224 /// NM_181744 /// NM_00103005 1 | Opsin-5 gene: ENSG00000124818 /// Opsin-5 gene:ENSG00000124818 /// opsin 5 isoform 2 gene: ENSG00000124818 /// opsin 5 isoform 2 gene: ENSG00000124818 /// Homo sapiens mRNA; cDNA DKFZp686D0636 (from clone DKFZp686D0636). /// Homo sapiens opsin 5 (OPN5), transcript variant 1, mRNA. /// Homo sapiens opsin 5 (OPN5), transcript variant 2, mRNA. | opsin 5 | OPN5 | 103 |
| 8143 710 | ENST0000038 5543 | ncrna:tRNA_pseudogene chromosome:NCBI36:7:148684 678:148684753:-1 gene: ENSG00000208278 | --- | --- | 104 |
| 8091 676 | ENST0000038 5921 /// ENST0000041 0743 | ncrna:rRNA_pseudogene chromosome:NCBI36:3:158374 399:158374523:-1 gene: ENSG00000208656 /// ncrna: rRNA chromosome:NCBI36:3: 158374 401:158374523:-1 gene: ENSG00000222675 | --- | --- | 105 |

(continued)

| Probe Set ID | Transcript ID | Transcript Description | Gene Title | Gene Symbol | |
|---|---|---|---|---|---|
| 8083 409 | ENST0000035 6517 /// BC065724 /// NM_207365 | Isoform 1 of Arylacetamide deacetylase-like 2 gene: ENSG00000197953 /// Homo sapiens arylacetamide deacetylase-like 2, mRNA (cDNA clone MGC:72001 IMAGE: 6663150), complete cds. /// Homo sapiens arylacetamide deacetylase-like 2 (AADACL2), mRNA. | arylacetamide deacetylase-like 2 | AADACL2 | 106 |
| 7955 119 | ENST0000038 0491 /// ENST0000031 4014 /// BC031670 | Isoform 2 of Uncharacterized protein C12orf54 gene: ENSG00000177627 /// Isoform 1 of Uncharacterized protein C12orf54 gene: ENSG00000177627 /// Homo sapiens chromosome 12 open reading frame 54, mRNA (cDNA clone MGC:35033 IMAGE: 5165130), complete cds. | chromosome 12 open reading frame 54 | C12orf54 | 107 |
| 8005 757 | ENST0000038 7268 | ncrna:Mt_tRNA_pseudogene chromosome:NCBI36:17:21952 378:21952445:1 qene: ENSG00000210003 | --- | --- | 108 |
| 8129 313 | ENST0000036 4509 | ncrna:snRNA chromosome: NCBI36:6:121905 331: 121905471:-1 qene: ENSG00000201379 | --- | --- | 109 |

(continued)

| Probe Set ID | Transcript ID | Transcript Description | Gene Title | Gene Symbol | |
|---|---|---|---|---|---|
| 8176 806 | ENST0000030 3804 /// ENST0000034 1740 /// ENST0000033 8793 /// ENST0000030 3728 /// ENST0000034 3584 /// ENST0000030 3593 /// ENST0000030 6589 /// ENST0000033 8673 /// AF517635 /// NM_00100275 8 /// NM_004676 | Isoform 1 of PTPN13-like protein, Y-linked gene: ENSG00000169807 /// Isoform 2 of PTPN13-like protein, Y-linked gene:ENSG00000169807 /// Isoform 2 of PTPN13-like protein, Y-linked gene: ENSG00000169789 /// Isoform 1 of PTPN13-like protein, Y-linked gene:ENSG00000169789 /// Isoform 1 of PTPN13-like protein, Y-linked gene: ENSG00000169763 /// Isoform 2 of PTPN13-like protein, Y-linked gene:ENSG00000169763 /// Isoform 1 of PTPN13-like protein, Y-linked gene: ENSG00000172283 /// Isoform 2 of PTPN13-like protein, Y-linked gene:ENSG00000172283 /// Homo sapiens testis-specific PTP-BL related Y protein mRNA, complete cds, alternatively spliced. /// Homo sapiens PTPN13-like, Y-linked 2 (PRY2), mRNA. /// Homo sapiens PTPN13-like, Y-linked (PRY), mRNA. | PTPN13-like, Y-linked /// PTPN13-like, Y-linked 2 | PRY /// PRY2 | 110 |
| 8020 842 | NR_003558 | Homo sapiens WW domain binding protein 11 pseudogene 1 (WBP11P1), non-coding RNA. | WW domain binding protein 11 pseudogene 1 | WBP11P1 | 111 |
| 8028 389 | ENST0000039 6877 /// ENST0000033 8502 /// DQ323928 /// NM_00103961 6 /// NM_ 00104252 2 | sprouty-related, EVH1 domain containing 3 isoform b gene: ENSG00000188766 /// Sprouty-related, EVH1 domain-containing protein 3 gene: ENSG00000188766 /// Homo sapiens Spred3 mRNA, complete cds. /// Homo sapiens sprouty-related, EVH1 domain containing 3 (SPRED3), transcript variant 2, mRNA. /// Homo sapiens sprouty-related, EVH1 domain containing 3 (SPRED3), transcript variant 1, mRNA. | sprouty-related, EVH1 domain containing 3 | SPRED3 | 112 |

(continued)

| Probe Set ID | Transcript ID | Transcript Description | Gene Title | Gene Symbol | |
|---|---|---|---|---|---|
| 8007 828 | ENST0000034 4290 /// ENST0000026 2410 /// ENST0000035 1559 /// ENST0000034 0799 /// ENST0000035 4326 /// ENST0000034 7967 /// ENST0000033 4239 /// BC114948 /// NM_00112306 7 /// NM_016835 /// NM_016834 /// NM_016841 /// NM_005910 /// NM_00112306 6 | Isoform Tau-G of Microtubule-associated protein tau gene: ENSG00000186868 /// Isoform PNS-tau of Microtubule-associated protein tau gene: ENSG00000186868 /// Isoform Tau-F of Microtubule-associated protein tau gene: ENSG00000186868 /// Isoform Tau-E of Microtubule-associated protein tau gene: ENSG00000186868 /// Isoform Tau-C of Microtubule-associated protein tau gene: ENSG00000186868 /// Isoform Tau-D of Microtubule-associated protein tau gene: ENSG00000186868 /// Isoform Tau-A of Microtubule-associated protein tau gene: ENSG00000186868 /// Homo sapiens microtubule-associated protein tau, mRNA (cDNA clone IMAGE:40007445), complete cds. /// Homo sapiens microtubule-associated protein tau (MAPT), transcript variant 5, mRNA. /// Homo sapiens microtubule-associated protein tau (MAPT), transcript variant 1, mRNA. /// Homo sapiens microtubule-associated protein tau (MAPT), transcript variant 3, mRNA. /// Homo sapiens microtubule-associated protein tau (MAPT), transcript variant 4, mRNA. /// Homo sapiens microtubule-associated protein tau (MAPT), transcript variant 2, mRNA. /// Homo sapiens microtubule-associated protein tau (MAPT), transcript variant 6, mRNA. | microtubule-associated protein tau | MAPT | 113 |
| 8058 145 | --- | --- | --- | --- | 114 |
| 8055 348 | ENST0000041 0136 | ncrna:misc_RNA chromosome: NCBI36:2:133865 128: 133865440:-1 gene: ENSG00000222068 | --- | --- | 115 |

(continued)

| Probe Set ID | Transcript ID | Transcript Description | Gene Title | Gene Symbol | |
|---|---|---|---|---|---|
| 7951 165 | ENST0000026 3463 /// ENST0000032 5455 /// X51730 /// NM_000926 | Progesterone receptor, isoform CRA_c gene: ENSG00000082175 /// Isoform B of Progesterone receptor gene: ENSG00000082175 /// Human mRNA and promoter DNA for progesterone receptor. /// Homo sapiens progesterone receptor (PGR), mRNA. | progesterone receptor | PGR | 116 |
| 8141 922 | ENST0000033 9444 /// ENST0000039 3735 /// ENST0000035 6767 /// ENST0000039 3730 /// ENST0000035 4356 /// ENST0000030 6312 /// ENST0000039 3732 /// ENST0000039 3729 /// ENST0000039 3723 /// ENST0000039 3727 /// ENST0000039 3722 /// AF523354 /// NM_206884 /// NM_198999 /// NM_206883 /// NM_206885 | Isoform 2 of Prestin gene: ENSG00000170615 /// Isoform 3 of Prestin gene: ENSG00000170615 /// Isoform 4 of Prestin gene: ENSG00000170615 /// SLC26A5 protein gene: ENSG00000170615 /// Prestin gene:ENSG00000170615 /// Isoform 1 of Prestin gene: ENSG00000170615 /// SLC26A5 protein gene: ENSG00000170615 /// SLC26A5 protein gene: ENSG00000170615 /// Prestin isoform SLC26A5e gene: ENSG00000170615 /// Prestin gene:ENSG00000170615 /// Putative uncharacterized protein SLC26A5 gene: ENSG00000170615 /// Homo sapiens prestin (PRES) mRNA, complete cds. /// Homo sapiens solute carrier family 26, member 5 (prestin) (SLC26A5), transcript variant c, mRNA. /// Homo sapiens solute carrier family 26, member 5 (prestin) (SLC26A5), transcript variant a, mRNA. /// Homo sapiens solute carrier family 26, member 5 (prestin) (SLC26A5), transcript variant b, mRNA. /// Homo sapiens solute carrier family 26, member 5 (prestin) (SLC26A5), transcript variant d, mRNA. | solute carrier family 26, member 5 (prestin) | SLC26A5 | 117 |

(continued)

| Probe Set ID | Transcript ID | Transcript Description | Gene Title | Gene Symbol | |
|---|---|---|---|---|---|
| 7934 731 | ENST0000031 8965 /// ENST0000037 2288 /// ENST0000037 2287 /// ENST0000040 5868 /// XM_ 00172365 3 /// XM_926017 | cdna:pseudogene chromosome: NCBI36:10:32840 249: 32840671:-1 gene: ENSG00000181993 /// cdna: pseudogene chromosome: NCBI36:10:81774 473: 81774898:-1 gene: ENSG00000204042 /// cdna: pseudogene chromosome: NCBI36:10:81781 704: 81782129:-1 gene: ENSG00000204041 /// cdna: pseudogene chromosome: NCBI36:10:81790 363: 81790779:-1 gene: ENSG00000217279 /// PREDICTED: Homo sapiens similar to hCG1791993 (LOC642538), mRNA. /// PREDICTED: Homo sapiens similar to hCG1791993 (LOC642521), mRNA. | similar to hCG1791993 /// similar to hCG1791993 | LOC6425 38 /// LOC6425 21 | 118 |
| 7921 449 | ENST0000025 5030 /// ENST0000036 8112 /// ENST0000036 8111 /// ENST0000036 8110 /// ENST0000034 3919 /// AK289443 /// NM_000567 | Isoform 1 of C-reactive protein gene:ENSG00000132693 /// Isoform 2 of C-reactive protein gene:ENSG00000132693 /// C-reactive protein, pentraxin-related gene:ENSG00000132693 /// C-reactive protein, pentraxin-related gene:ENSG00000132693 /// C-reactive protein, pentraxin-related gene:ENSG00000132693 /// Homo sapiens cDNA FLJ78115 complete cds, highly similar to Homo sapiens C-reactive protein, pentraxin-related (CRP), mRNA. /// Homo sapiens C-reactive protein, pentraxin-related (CRP), mRNA. | C-reactive protein, pentraxin-related | CRP | 119 |
| 8157 818 | ENST0000037 3574 /// BC127949 /// NM_00104547 6 | WD repeat-containing protein 38 gene:ENSG00000136918 /// Homo sapiens WD repeat domain 38, mRNA (cDNA clone MGC: 158102 IMAGE:40132852), complete cds. /// Homo sapiens WD repeat domain 38 (WDR38), mRNA. | WD repeat domain 38 | WDR38 | 120 |

(continued)

| Probe Set ID | Transcript ID | Transcript Description | Gene Title | Gene Symbol | |
|---|---|---|---|---|---|
| 7920 264 | ENST0000035 9215 /// ENST0000036 8718 /// ENST0000036 8717 /// BC093955 /// NM_002962 | S100 calcium binding protein A5 gene:ENSG00000196420 /// S100 calcium binding protein A5 gene:ENSG00000196420 /// S100 calcium binding protein A5 gene:ENSG00000196420 /// Homo sapiens S100 calcium binding protein A5, mRNA (cDNA clone MGC:120990 IMAGE: 7939800), complete cds. /// Homo sapiens S100 calcium binding protein A5 (S100A5), mRNA. | S100 calcium binding protein A5 | S100A5 | 121 |
| 8099 362 | ENST0000041 1154 /// ENST0000038 7157 | ncrna:rRNA chromosome: NCBI36:4:972647 8:9726606:-1 gene:ENSG00000223086 /// ncrna:rRNA_pseudogene chromosome:NCBI36:4:972651 7:9726606:-1 gene: ENSG00000209892 | --- | --- | 122 |
| 8095 412 | ENST0000038 1066 /// ENST0000035 4865 /// ENST0000024 6891 /// BC128227 /// NM_00102510 4 /// NM_001890 | Casein gene: ENSG00000126545 /// Isoform 3 of Alpha-S1-casein gene: ENSG00000126545 /// Isoform 1 of Alpha-S1-casein gene: ENSG00000126545 /// Homo sapiens casein alpha s1, mRNA (cDNA clone MGC:149367 IMAGE:40114618), complete cds. /// Homo sapiens casein alpha s1 (CSN1S1), transcript variant 2, mRNA. /// Homo sapiens casein alpha s1 (CSN1S1), transcript variant 1, mRNA. | casein alpha s1 | CSN1S1 | 123 |
| 8010 622 | ENST0000038 4294 | ncrna:misc_RNA chromosome: NCBI36:17:77150 953: 77151065:1 qene: ENSG00000207021 | --- | --- | 124 |
| 8107 421 | ENST0000031 6788 /// BC012614 /// NM_001284 | AP-3 complex subunit sigma-1 gene:ENSG00000177879 /// Homo sapiens adaptor-related protein complex 3, sigma 1 subunit, mRNA (cDNA clone IMAGE:4281620). /// Homo sapiens adaptor-related protein complex 3, sigma 1 subunit (AP3S1), mRNA. | adaptor-related protein complex 3, sigma 1 subunit | AP3S1 | 125 |

(continued)

| Probe Set ID | Transcript ID | Transcript Description | Gene Title | Gene Symbol | |
|---|---|---|---|---|---|
| 8126 750 | ENST0000023 0565 /// ENST0000037 1383 /// BX647968 /// NM_021572 | Ectonucleotide pyrophosphatase/phosphodiest erase family member 5 gene: ENSG00000112796 /// Ectonucleotide pyrophosphatase/phosphodiest erase family member 5 gene: ENSG00000112796 /// Homo sapiens mRNA; cDNA DKFZp686E1552 (from clone DKFZp686E1552). /// Homo sapiens ectonucleotide pyrophosphatase/phosphodiest erase 5 (putative function) (ENPP5), mRNA. | ectonucleotide pyrophosphatase/pho sphodiesterase 5 (putative function) | ENPP5 | 126 |
| 8027 770 | ENST0000027 0310 /// BC018619 /// NM_022006 | FXYD domain-containing ion transport regulator 7 gene: ENSG00000126258 /// Homo sapiens FXYD domain containing ion transport regulator 7, mRNA (cDNA clone MGC:31815 IMAGE:3626060), complete cds. /// Homo sapiens FXYD domain containing ion transport regulator 7 (FXYD7), mRNA. | FXYD domain containing ion transport regulator 7 | FXYD7 | 127 |
| 8088 491 | ENST0000038 3710 /// ENST0000038 3709 /// ENST0000028 3269 /// ENST0000035 7948 /// ENST0000036 0097 /// AF458662 /// NM_ 183393 /// NM_ 003716 /// NM_ 183394 | Isoform 1 of Calcium-dependent secretion activator 1 gene: ENSG00000163618 /// Isoform 4 of Calcium-dependent secretion activator 1 gene: ENSG00000163618 /// Isoform 3 of Calcium-dependent secretion activator 1 gene: ENSG00000163618 /// Isoform 2 of Calcium-dependent secretion activator 1 gene: ENSG00000163618 /// Isoform 5 of Calcium-dependent secretion activator 1 gene: ENSG00000163618 /// Homo sapiens calcium-dependent activator protein for secretion protein mRNA, complete cds. /// Homo sapiens Ca++-dependent secretion activator (CADPS), transcript variant 3, mRNA. /// Homo sapiens Ca++-dependent secretion activator (CADPS), transcript variant 1, mRNA. /// Homo sapiens Ca++-dependent secretion activator (CADPS), transcript variant 2, mRNA. | Ca++-dependent secretion activator | CADPS | 128 |

(continued)

| Probe Set ID | Transcript ID | Transcript Description | Gene Title | Gene Symbol | |
|---|---|---|---|---|---|
| 8161 192 | ENST0000037 7877 /// ENST0000035 7058 /// ENST0000035 0199 /// ENST0000037 7885 /// ENST0000037 7876 /// ENST0000035 3739 /// ENST0000025 9605 /// ENST0000037 7870 /// AF394047 /// NM_022781 /// NM_194328 /// NM_194329 /// NM_194330 /// NM_194332 | Ring finger protein 38 gene: ENSG00000137075 /// ring finger protein 38 isoform 2 gene: ENSG00000137075 /// ring finger protein 38 isoform 2 gene: ENSG00000137075 /// ring finger protein 38 isoform 2 gene: ENSG00000137075 /// ring finger protein 38 isoform 2 gene: ENSG00000137075 /// Isoform 2 of RING finger protein 38 gene: ENSG00000137075 /// Isoform 1 of RING finger protein 38 gene: ENSG00000137075 /// Ring finger protein 38 gene: ENSG00000137075 /// Homo sapiens RING finger protein 38 (RNF38) mRNA, complete cds. /// Homo sapiens ring finger protein 38 (RNF38), transcript variant 1, mRNA. /// Homo sapiens ring finger protein 38 (RNF38), transcript variant 2, mRNA. /// Homo sapiens ring finger protein 38 (RNF38), transcript variant 3, mRNA. /// Homo sapiens ring finger protein 38 (RNF38), transcript variant 5, mRNA. /// Homo sapiens ring finger protein 38 (RNF38), transcript variant 6, mRNA. | ring finger protein 38 | RNF38 | 129 |
| 8124 634 | ENST0000040 4200 /// ENST0000040 1594 /// ENST0000036 6307 | cdna:pseudogene chromosome: NCBI36:c6_COX: 29249580: 29250516:-1 gene: ENSG00000219452 /// cdna: pseudogene chromosome: NCBI36:c6_QBL: 29248513: 29249449:-1 gene: ENSG00000216296 /// cdna: pseudogene chromosome: NCBI36:6:292136 26:29214572:-1 gene:ENSG00000203492 | --- | --- | 130 |
| 8007 584 | ENST0000029 3414 /// BC075088 /// NM_080863 | Ankyrin repeat and SOCS box protein 16 gene: ENSG00000161664 /// Homo sapiens ankyrin repeat and SOCS box-containing 16, mRNA (cDNA clone MGC:103981 IMAGE: 30915388), complete cds. /// Homo sapiens ankyrin repeat and SOCS box-containing 16 (ASB16), mRNA. | ankyrin repeat and SOCS box-containing 16 | ASB16 | 131 |

(continued)

| Probe Set ID | Transcript ID | Transcript Description | Gene Title | Gene Symbol | |
|---|---|---|---|---|---|
| 7964 660 | ENST0000029 9178 /// AY322550 /// NM_000706 | Vasopressin V1a receptor gene: ENSG00000166148 /// Homo sapiens arginine vasopressin receptor 1A mRNA, complete cds. /// Homo sapiens arginine vasopressin receptor 1A (AVPR1A), mRNA. | arginine vasopressin receptor 1A | AVPR1A | 132 |
| 7980 003 | ENST0000038 7477 | ncrna:sn RNA_pseudogene chromosome:NCBI36:14:72783 233:72783340:-1 gene: ENSG00000210212 | --- | --- | 133 |
| 8135 458 | ENST0000022 2597 /// AK026762 /// NM_024814 /// NR_024199 | E3 ubiquitin-protein ligase Hakai gene:ENSG00000105879 /// Homo sapiens cDNA: FLJ23109 fis, clone LNG07754. /// Homo sapiens Cas-Br-M (murine) ecotropic retroviral transforming sequence-like 1 (CBLL1), transcript variant 1, mRNA. /// Homo sapiens Cas-Br-M (murine) ecotropic retroviral transforming sequence-like 1 (CBLL1), transcript variant 2, transcribed RNA. | Cas-Br-M (murine) ecotropic retroviral transforming sequence-like 1 | CBLL1 | 134 |
| 7991 512 | ENST0000035 2519 /// ENST0000034 1853 /// AK302717 /// NR_003260 | Uncharacterized protein C15orf51 (Fragment) gene: ENSG00000182397 /// UPF0621 protein C15orf51 gene: ENSG00000182397 /// Homo sapiens cDNA FLJ54911 complete cds. /// Homo sapiens chromosome 15 open reading frame 51 (C15orf51), non-coding RNA. | chromosome 15 open reading frame 51 | C15orf51 | 135 |
| 8029 693 | ENST0000035 3609 /// BC036724 /// NM_006732 /// NM_00111417 1 | Protein fosB gene: ENSG00000125740 /// Homo sapiens FBJ murine osteosarcoma viral oncogene homolog B, mRNA (cDNA clone MGC:39968 IMAGE:5212854), complete cds. /// Homo sapiens FBJ murine osteosarcoma viral oncogene homolog B (FOSB), transcript variant 1, mRNA. /// Homo sapiens FBJ murine osteosarcoma viral oncogene homolog B (FOSB), transcript variant 2, mRNA. | FBJ murine osteosarcoma viral oncogene homolog B | FOSB | 136 |

(continued)

| Probe Set ID | Transcript ID | Transcript Description | Gene Title | Gene Symbol | |
|---|---|---|---|---|---|
| 7981 773 | ENST0000038 4559 | ncrna:snRNA chromosome: NCBI36:15:19204 036: 19204142:1 qene: ENSG00000207289 | --- | --- | 137 |
| 7934 729 | GENSCAN000 00036525 /// ENST0000031 8965 /// ENST0000037 2288 /// ENST0000037 2287 /// ENST0000040 5868 /// XM_ 00172365 3 /// XM_926017 | cdna:Genscan chromosome: NCBI36:10:81732 033: 81798683:-1 /// cdna: pseudogene chromosome: NCBI36:10:32840 249: 32840671:-1 gene: ENSG00000181993 /// cdna: pseudogene chromosome: NCBI36:10:81774 473: 81774898:-1 gene: ENSG00000204042 /// cdna: pseudogene chromosome: NCBI36:10:81781 704: 81782129:-1 gene: ENSG00000204041 /// cdna: pseudogene chromosome: NCBI36:10:81790 363: 81790779:-1 gene: ENSG00000217279 /// PREDICTED: Homo sapiens similar to hCG1791993 (LOC642538), mRNA. /// PREDICTED: Homo sapiens similar to hCG1791993 (LOC642521), mRNA. | similar to hCG1791993 /// similar to hCG1791993 | LOC6425 38 /// LOC6425 21 | 138 |
| 7936 996 | ENST0000035 6858 /// ENST0000028 4694 /// ENST0000036 8674 /// ENST0000039 2694 /// AK297577 /// NM_00100429 8 | Novel protein gene: ENSG00000154493 /// cDNA FLJ60307 gene: ENSG00000154493 /// Novel protein gene: ENSG00000154493 /// Novel protein gene: ENSG00000154493 /// Homo sapiens cDNA FLJ60307 complete cds. /// Homo sapiens chromosome 10 open reading frame 90 (C10orf90), mRNA. | chromosome 10 open reading frame 90 | C10orf90 | 139 |

(continued)

| Probe Set ID | Transcript ID | Transcript Description | Gene Title | Gene Symbol | |
|---|---|---|---|---|---|
| 7906 205 | ENST0000032 9117 /// ENST0000036 1588 /// ENST0000025 5029 /// AY358372 /// NM_021948 /// NM_198427 | Isoform 1 of Brevican core protein gene:ENSG00000132692 /// Isoform 2 of Brevican core protein gene:ENSG00000132692 /// Hyaluronan binding protein (Fragment) gene: ENSG00000132692 /// Homo sapiens clone DNA98565 Brevican Core Pro (UNQ2525) mRNA, complete cds. /// Homo sapiens brevican (BCAN), transcript variant 1, mRNA. /// Homo sapiens brevican (BCAN), transcript variant 2, mRNA. | brevican | BCAN | 140 |
| 8100 990 | BC132938 /// L10403 | Homo sapiens pro-platelet basic protein-like 2, mRNA (cDNA clone MGC:164569 IMAGE: 40146960), complete cds. /// Homo sapiens DNA binding protein for surfactant protein B mRNA, complete cds. | pro-platelet basic protein-like 2 | PPBPL2 | 141 |
| 8156 846 | GENSCAN000 00020848 /// ENST0000040 9669 /// ENST0000041 0082 /// ENST0000040 9686 | cdna:Genscan chromosome: NCBI36:9:101107 215: 101108714:1 /// cdna: pseudogene chromosome: NCBI36:9:101107 215: 101108714:1 gene: ENSG00000222026 /// cdna: pseudogene chromosome: NCBI36:9:101107 215: 101108714:1 gene: ENSG00000222034 /// cdna: pseudogene chromosome: NCBI36:9:101107 215: 101108714:1 gene: ENSG00000222039 | --- | --- | 142 |

(continued)

| Probe Set ID | Transcript ID | Transcript Description | Gene Title | Gene Symbol | |
|---|---|---|---|---|---|
| 8044 563 | ENST0000034 1010 /// ENST0000033 7569 /// ENST0000039 3197 /// AY029413 /// NM_032556 /// NM_173161 | Isoform 1 of Interleukin-1 family member 10 gene: ENSG00000136697 /// Isoform 2 of Interleukin-1 family member 10 gene:ENSG00000136697 /// Isoform 1 of Interleukin-1 family member 10 gene: ENSG00000136697 /// Homo sapiens interleukin-1 receptor antagonist-like FIL1 theta (FIL1-theta) mRNA, complete cds. /// Homo sapiens interleukin 1 family, member 10 (theta) (IL1F10), transcript variant 1, mRNA. /// Homo sapiens interleukin 1 family, member 10 (theta) (IL1F10), transcript variant 2, mRNA. | interleukin 1 family, member 10 (theta) | IL1F10 | 143 |
| 7932 964 | ENST0000035 5848 /// ENST0000041 0067 /// BC005235 /// NM_006333 /// NM_173177 | Nuclear nucleic acid-binding protein C1D gene: ENSG00000197223 /// cdna: known chromosome:NCBI36:2: 681232 92:68143661:-1 gene: ENSG00000197223 /// Homo sapiens nuclear DNA-binding protein, mRNA (cDNA clone MGC:12261 IMAGE:3930648), complete cds. /// Homo sapiens nuclear DNA-binding protein (C1D), transcript variant 1, mRNA. /// Homo sapiens nuclear DNA-binding protein (C1D), transcript variant 2, mRNA. | nuclear DNA-binding protein | C1D | 144 |
| 7912 606 | ENST0000036 1079 /// ENST0000033 0881 /// ENST0000035 7367 /// NM_ 00101227 7 /// NM_00101227 6 | PRAME family member 7 gene: ENSG00000204510 /// PRAME family member 7 gene: ENSG00000204510 /// PRAME family member 8 gene: ENSG00000182330 /// Homo sapiens PRAME family member 7 (PRAMEF7), mRNA. /// Homo sapiens PRAME family member 8 (PRAMEF8), mRNA. | PRAME family member 7 /// PRAME family member 8 | PRAMEF7 /// PRAMEF8 | 145 |
| 8099 713 | ENST0000038 7283 | ncrna:Mt_tRNA_pseudogene chromosome: NCB I36:4:253286 70:25328727:-1 qene: ENSG00000210018 | --- | --- | 146 |

(continued)

| Probe Set ID | Transcript ID | Transcript Description | Gene Title | Gene Symbol | |
|---|---|---|---|---|---|
| 8139 723 | ENST0000032 4256 /// BC011872 /// NR_003949 | Putative FK506-binding protein 9-like protein gene: ENSG00000176826 /// Homo sapiens FK506 binding protein 9-like, mRNA (cDNA clone MGC: 20531 IMAGE:3028515), complete cds. /// Homo sapiens FK506 binding protein 9-like (FKBP9L), non-coding RNA. | FK506 binding protein 9-like | FKBP9L | 147 |
| 8109 159 | hsa-mir-145 /// hsa-mir-145 /// AK093957 | MI0000461 Homo sapiens miR-145 stem-loop /// MI0000461 Homo sapiens miR-145 stem-loop /// Homo sapiens cDNA FLJ36638 fis, clone TRACH2018950. | hypothetical protein LOC728264 | LOC7282 64 | 1 4 8 |
| 7906 197 | ENST0000025 5039 /// AB049054 /// NM_021817 | Hyaluronan and proteoglycan link protein 2 gene: ENSG00000132702 /// Homo sapiens BRAL1 mRNA for brain link protein-1, complete cds. /// Homo sapiens hyaluronan and proteoglycan link protein 2 (HAPLN2), mRNA. | hyaluronan and proteoglycan link protein 2 | HAPLN2 | 149 |
| 7912 591 | ENST0000036 1079 /// ENST0000033 0881 /// ENST0000035 7367 /// NM_ 00101227 7 /// NM_00101227 6 | PRAME family member 7 gene: ENSG00000204510 /// PRAME family member 7 gene: ENSG00000204510 /// PRAME family member 8 gene: ENSG00000182330 /// Homo sapiens PRAME family member 7 (PRAMEF7), mRNA. /// Homo sapiens PRAME family member 8 (PRAMEF8), mRNA. | PRAME family member 7 /// PRAME family member 8 | PRAMEF7 /// PRAMEF8 | 150 |
| 7986 291 | A Y358254 | Homo sapiens clone DNA172197 IFMQ9370 (UNQ9370) mRNA, complete cds. | IFMQ9370 | UNQ9370 | 151 |
| 7925 504 | ENST0000035 7246 /// BC132986 /// NM_00100434 3 | Microtubule-associated proteins 1A/1B light chain 3C gene: ENSG00000197769 /// Homo sapiens microtubule-associated protein 1 light chain 3 gamma, mRNA (cDNA clone MGC: 164617 IMAGE:40147008), complete cds. /// Homo sapiens microtubule-associated protein 1 light chain 3 gamma (MAP1LC3C), mRNA. | microtubule-associated protein 1 light chain 3 gamma | MAP1LC3 C | 152 |

(continued)

| Probe Set ID | Transcript ID | Transcript Description | Gene Title | Gene Symbol | |
|---|---|---|---|---|---|
| 7897 991 | ENST0000036 1079 /// ENST0000033 0881 /// ENST0000035 7367 /// NM_ 00101227 7 /// NM_00101227 6 | PRAME family member 7 gene: ENSG00000204510 /// PRAME family member 7 gene: ENSG00000204510 /// PRAME family member 8 gene: ENSG00000182330 /// Homo sapiens PRAME family member 7 (PRAMEF7), mRNA. /// Homo sapiens PRAME family member 8 (PRAMEF8), mRNA. | PRAME family member 7 /// PRAME family member 8 | PRAMEF7 /// PRAMEF8 | 153 |
| 7972 461 | ENST0000037 6503 /// ENST0000031 3260 /// ENST0000037 6494 /// U21936 /// NM_005073 | Solute carrier family 15 member 1 gene:ENSG00000088386 /// pH-sensing regulatory factor of peptide transporter gene: ENSG00000088386 /// Solute carrier family 15 (Oligopeptide transporter), member 1 gene: ENSG00000088386 /// Human peptide transporter (HPEPT1) mRNA, complete cds. /// Homo sapiens solute carrier family 15 (oligopeptide transporter), member 1 (SLC15A1), mRNA. | solute carrier family 15 (oligopeptide transporter), member 1 | SLC15A1 | 154 |
| 8146 857 | ENST0000038 8545 | ncrna:rRNA_pseudogene chromosome:NCBI36:8:697704 42:69770521:1 gene: ENSG00000211280 | --- | --- | 155 |
| 7925 846 | AF220183 /// BC104155 | Homo sapiens uncharacterized hypothalamus protein HT009 mRNA, complete cds. /// Homo sapiens chromosome 10 open reading frame 110, mRNA (cDNA clone IMAGE:40029412), complete cds. | chromosome 10 open reading frame 110 | C10orf110 | 156 |
| 8059 026 | hsa-mir-375 /// hsa-mir-375 | MI0000783 Homo sapiens miR-375 stem-loop /// MI0000783 Homo sapiens miR-375 stem-loop | --- | --- | 157 |
| 8071 274 | L20860 | Human glycoprotein Ib beta mRNA, complete cds. | glycoprotein Ib (platelet), beta polypeptide | GP1BB | 158 |
| 8050 113 | AK125905 | Homo sapiens cDNA FLJ43917 fis, clone TESTI4011505. | hypothetical LOC100129581 | LOC1001 29581 | 159 |
| 8170 159 | ENST0000037 0648 /// L08893 /// NM_001727 | Bombesin receptor subtype-3 gene:ENSG00000102239 /// Human bombesin receptor subtype-3 mRNA, complete cds. /// Homo sapiens bombesin-like receptor 3 (BRS3), mRNA. | bombesin-like receptor 3 | BRS3 | 160 |

(continued)

| Probe Set ID | Transcript ID | Transcript Description | Gene Title | Gene Symbol | |
|---|---|---|---|---|---|
| 7958 711 | ENST0000030 8208 /// BC044815 /// NM_152591 | Coiled-coil domain-containing protein 63 gene: ENSG00000173093 /// Homo sapiens coiled-coil domain containing 63, mRNA (cDNA clone IMAGE:5166270), complete cds. /// Homo sapiens coiled-coil domain containing 63 (CCDC63), mRNA. | coiled-coil domain containing 63 | CCDC63 | 1 6 1 |
| 8021 357 | ENST0000026 2095 /// NM_ 004852 | one cut domain, family member 2 gene:ENSG00000119547 /// Homo sapiens one cut homeobox 2 (ONECUT2), mRNA. | one cut homeobox 2 | ONECUT2 | 1 6 2 |
| 8095 161 | ENST0000038 7825 | ncrna:snRNA_pseudogene chromosome:NCBI36:4:563595 61:56359653:1 gene: ENSG00000210560 | --- | --- | 1 6 3 |
| 7960 861 | ENST0000036 4793 | ncrna:misc_RNA chromosome: NCBI36:12:78945 80:7894681 :- 1 gene:ENSG00000201663 | --- | --- | 1 6 4 |
| 8127 932 | ENST0000033 0469 /// ENST0000036 9663 /// BC132715 /// NM_00108050 8 | TBX18 protein (Fragment) gene: ENSG00000112837 /// T-box transcription factor TBX18 gene: ENSG00000112837 /// Homo sapiens T-box 18, mRNA (cDNA clone MGC:164346 IMAGE: 40146737), complete cds. /// Homo sapiens T-box 18 (TBX18), mRNA. | T-box 18 | TBX18 | 1 6 5 |
| 8116 607 | ENST0000039 9551 /// AK123663 /// BC108683 | hypothetical protein gene: ENSG00000215076 /// Homo sapiens cDNA FLJ41669 fis, clone FEBRA2028618. /// Homo sapiens cDNA clone IMAGE: 5212284. | hypothetical LOC401232 | DKFZP68 6115217 | 1 6 6 |
| 8164 766 | ENST0000029 8545 /// BC050576 /// NM_152572 | Isoform 1 of Putative adenylate kinase-like protein C9orf98 gene: ENSG00000165695 /// Homo sapiens chromosome 9 open reading frame 98, mRNA (cDNA clone MGC:57797 IMAGE: 5744517), complete cds. /// Homo sapiens chromosome 9 open reading frame 98 (C9orf98), mRNA. | chromosome 9 open reading frame 98 | C9orf98 | 1 6 7 |

(continued)

| Probe Set ID | Transcript ID | Transcript Description | Gene Title | Gene Symbol | |
|---|---|---|---|---|---|
| 8012 726 | ENST0000037 9814 /// ENST0000022 6207 /// AF111785 /// NM_005963 | MYH1 protein (Fragment) gene: ENSG00000109061 /// Myosin-1 gene:ENSG00000109061 /// Homo sapiens myosin heavy chain IIx/d mRNA, complete cds. /// Homo sapiens myosin, heavy chain 1, skeletal muscle, adult (MYH1), mRNA. | myosin, heavy chain 1, skeletal muscle, adult | MYH1 | 1 6 8 |
| 7906 552 | ENST0000036 8078 /// ENST0000036 8079 /// BC022289 /// NM_001231 | Calsequestrin gene: ENSG00000143318 /// Calsequestrin-1 gene: ENSG00000143318 /// Homo sapiens calsequestrin 1 (fast-twitch, skeletal muscle), mRNA (cDNA clone MGC:22462 IMAGE:4338020), complete cds. /// Homo sapiens calsequestrin 1 (fast-twitch, skeletal muscle) (CASQ1), nuclear gene encoding mitochondrial protein, mRNA. | calsequestrin 1 (fast-twitch, skeletal muscle) | CASQ1 | 1 6 9 |
| 8115 831 | ENST0000023 9223 /// BC022463 /// NM_004417 | Dual specificity protein phosphatase 1 gene: ENSG00000120129 /// Homo sapiens dual specificity phosphatase 1, mRNA (cDNA clone MGC:26153 IMAGE: 4794895), complete cds. /// Homo sapiens dual specificity phosphatase 1 (DUSP1), mRNA. | dual specificity phosphatase 1 | DUSP1 | 1 7 0 |
| 8143 708 | AK125575 | Homo sapiens cDNA FLJ43587 fis, clone SKNMC2009450. | --- | --- | 1 7 1 |
| 8036 430 | ENST0000035 8582 /// BC108687 /// NM_152605 | Isoform 2 of Zinc finger protein 781 gene:ENSG00000196381 /// Homo sapiens zinc finger protein 781, mRNA (cDNA clone MGC: 131783 IMAGE:6148649), complete cds. /// Homo sapiens zinc finger protein 781 (ZNF781), mRNA. | zinc finger protein 781 | ZNF781 | 1 7 2 |
| 7937 975 | ENST0000035 4690 | cdna:pseudogene chromosome: NCBI36:11:48099 75:4810860:1 gene:ENSG00000197984 | --- | --- | 1 7 3 |
| 7973 054 | ENST0000036 3355 | ncrna:rRNA chromosome: NCBI36:14:19952 986: 19953103:1 gene: ENSG00000200225 | --- | --- | 1 7 4 |

(continued)

| Probe Set ID | Transcript ID | Transcript Description | Gene Title | Gene Symbol | |
|---|---|---|---|---|---|
| 8052 698 | ENST0000035 5848 /// ENST0000040 7324 /// ENST0000041 0067 /// ENST0000040 9302 /// BC005235 /// NM_006333 /// NM_173177 | Nuclear nucleic acid-binding protein C1D gene: ENSG00000197223 /// 20 kDa protein gene: ENSG00000197223 /// cdna: known chromosome:NCBI36:2: 681232 92:68143661:-1 gene: ENSG00000197223 /// cdna: known chromosome:NCBI36:2: 681233 13:68143645:-1 gene: ENSG00000197223 /// Homo sapiens nuclear DNA-binding protein, mRNA (cDNA clone MGC:12261 IMAGE:3930648), complete cds. /// Homo sapiens nuclear DNA-binding protein (C1D), transcript variant 1, mRNA. /// Homo sapiens nuclear DNA-binding protein (C1D), transcript variant 2, mRNA. | nuclear DNA-binding protein | C1D | 1 7 5 |
| 8113 352 | ENST0000038 8656 | ncrna:Mt_tRNA_pseudogene chromosome:NCBI36:5:994146 67:99414734:-1 gene: ENSG00000211391 | --- | --- | 1 7 6 |
| 8077 499 | AF086709 /// AK054898 /// BC016278 | Homo sapiens NAG-7 protein (NAG-7) mRNA, complete cds. /// Homo sapiens cDNA FLJ30336 fis, clone BRACE2007358, moderately similar to Homo sapiens NAG-7 protein (NAG-7) mRNA. /// Homo sapiens loss of heterozygosity, 3, chromosomal region 2, gene A, mRNA (cDNA clone MGC:8781 IMAGE: 3915957), complete cds. | loss of heterozygosity, 3, chromosomal region 2, gene A | LOH3CR2 A | 1 7 7 |
| 8104 074 | ENST0000030 7161 /// BC126297 /// NM_005958 | Melatonin receptor type 1A gene: ENSG00000168412 /// Homo sapiens melatonin receptor 1A, mRNA (cDNA clone MGC: 161575 IMAGE:8992013), complete cds. /// Homo sapiens melatonin receptor 1A (MTNR1A), mRNA. | melatonin receptor 1A | MTNR1A | 1 7 8 |
| 7951 701 | ENST0000038 8431 /// ENST0000038 8445 | ncrna:scRNA_pseudogene chromosome:NCBI36:11:11205 3855:112053924:-1 gene: ENSG00000211166 /// ncrna: scRNA _ pseudogene chromosome:NCBI36:11:11298 6610:112986679:1 qene: ENSG00000211180 | --- | --- | 1 7 9 |

(continued)

| Probe Set ID | Transcript ID | Transcript Description | Gene Title | Gene Symbol | |
|---|---|---|---|---|---|
| 8157 092 | ENST0000037 4692 /// ENST0000037 4689 /// ENST0000037 4688 /// BC000049 /// NM_018112 | Trimeric intracellular cation channel type B gene: ENSG00000095209 /// Putative uncharacterized protein TMEM38B gene: ENSG00000095209 /// 26 kDa protein gene: ENSG00000095209 /// Homo sapiens transmembrane protein 38B, mRNA (cDNA clone MGC: 960 IMAGE:3506969), complete cds. /// Homo sapiens transmembrane protein 38B (TMEM38B), mRNA. | transmembrane protein 38B | TMEM38B | 1 8 0 |
| 7942 814 | ENST0000038 7816 | ncrna:Mt_tRNA_pseudogene chromosome:NCBI36:11:80940 435:80940502:1 gene: ENSG00000210551 | --- | --- | 1 8 1 |
| 8090 366 | ENST0000029 0868 /// ENST0000038 3579 /// BC115405 /// NM_144639 | Probable urocanate hydratase gene:ENSG00000159650 /// Urocanase family protein gene: ENSG00000159650 /// Homo sapiens urocanase domain containing 1, mRNA (cDNA clone MGC:135007 IMAGE: 40076084), complete cds. /// Homo sapiens urocanase domain containing 1 (UROC1), mRNA. | urocanase domain containing 1 | UROC1 | 1 8 2 |
| 8036 300 | ENST0000036 3309 | ncrna:misc_RNA chromosome: NCBI36:19:41386 089: 41386201:-1 gene: ENSG00000200179 | --- | --- | 1 8 3 |
| 8036 403 | ENST0000031 6807 | Putative uncharacterized protein LOC400692 gene: ENSG00000180458 | --- | --- | 1 8 4 |

(continued)

| Probe Set ID | Transcript ID | Transcript Description | Gene Title | Gene Symbol | |
|---|---|---|---|---|---|
| 7971 644 | ENST0000037 8195 /// ENST0000036 1840 /// AF334405 /// NM_020456 /// NM_00112748 2 /// NR_023351 | Isoform 2 of Chronic lymphocytic leukemia deletion region gene 6 protein gene: ENSG00000123178 /// Isoform 1 of Chronic lymphocytic leukemia deletion region gene 6 protein gene:ENSG00000123178 /// Homo sapiens CLLL6 protein (CLLD6) mRNA, complete cds. /// Homo sapiens chromosome 13 open reading frame 1 (C13orf1), transcript variant 1, mRNA. /// Homo sapiens chromosome 13 open reading frame 1 (C13orf1), transcript variant 2, mRNA. /// Homo sapiens chromosome 13 open reading frame 1 (C13orf1), transcript variant 3, transcribed RNA. | chromosome 13 open reading frame 1 | C13orf1 | 1 8 5 |
| 8157 216 | ENST0000037 4279 /// BC038711 /// NM_003358 | Ceramide glucosyltransferase gene:ENSG00000148154 /// Homo sapiens UDP-glucose ceramide glucosyltransferase, mRNA (cDNA clone MGC:33797 IMAGE:5295561), complete cds. /// Homo sapiens UDP-glucose ceramide glucosyltransferase (UGCG), mRNA. | UDP-glucose ceram ide glucosyltransferase | UGCG | 1 8 6 |
| 8074 714 | ENST0000034 2005 /// ENST0000032 9949 /// ENST0000040 2027 /// ENST0000024 8992 /// AK292412 /// AK302597 /// AY358961 /// NR_003714 | cDNA FLJ60978, weakly similar to Nuclear envelope pore membrane protein POM 121 gene:ENSG00000182356 /// Putative uncharacterized protein ENSP00000383394 gene: ENSG00000217261 /// POM121-like 1 protein gene: ENSG00000183169 /// POM121-like gene:ENSG00000128262 /// Homo sapiens cDNA FLJ76724 complete cds. /// Homo sapiens cDNA FLJ60978 complete cds, weakly similar to Nuclear envelope pore membrane protein POM 121. /// Homo sapiens clone DNA107786 POM121-like (UNQ2565) mRNA, complete cds. /// Homo sapiens POM121-like protein (DKFZP434P211), non-coding RNA. | POM121 membrane glycoprotein-like 1 (rat) /// POM121 membrane glycoprotein-like 1 pseudogene /// POM121 membrane glycoprotein-like 1 pseudogene | POM121L 1 /// DKFZp43 4K191 /// DKFZP43 4P211 | 1 8 7 |
| 8065 758 | AK096092 | Homo sapiens cDNA FLJ38773 fis, clone KIDNE2018071. | hypothetical protein FLJ38773 | FLJ38773 | 1 8 8 |

(continued)

| Probe Set ID | Transcript ID | Transcript Description | Gene Title | Gene Symbol | |
|---|---|---|---|---|---|
| 8049 530 | ENST0000030 8482 /// NM_ 00113755 0 | leucine rich repeat (in FLII) interacting protein 1 isoform 1 gene:ENSG00000124831 /// Homo sapiens leucine rich repeat (in FLII) interacting protein 1 (LRRFIP1), transcript variant 1, mRNA. | leucine rich repeat (in FLII) interacting protein 1 | LRRFIP1 | 1 8 9 |
| 7906 475 | ENST0000032 1935 /// ENST0000036 8106 /// ENST0000033 9348 /// ENST0000039 2235 /// AK131201 /// NM_00100431 0 | Isoform 2 of Fc receptor-like protein 6 gene: ENSG00000181036 /// Isoform 1 of Fc receptor-like protein 6 gene: ENSG00000181036 /// Isoform 3 of Fc receptor-like protein 6 gene: ENSG00000181036 /// Isoform 4 of Fc receptor-like protein 6 gene: ENSG00000181036 /// Homo sapiens cDNA FLJ16056 fis, clone SPLEN2010588, weakly similar to CELL SURFACE GLYCOPROTEIN GP42 PRECURSOR. /// Homo sapiens Fc receptor-like 6 (FCRL6), mRNA. | Fc receptor-like 6 | FCRL6 | 1 9 0 |
| 7989 476 | ENST0000030 4813 /// ENST0000029 9125 /// AK096042 | cdna:known chromosome: NCBI36:15:60322 074: 60323649:-1 gene: ENSG00000220356 /// Isoform 1 of Golgin subfamily A member 2-like protein 4 gene: ENSG00000166104 /// Homo sapiens cDNA FLJ38723 fis, clone KIDNE2010137, weakly similar to GOLGIN-95. | hypothetical FLJ38723 | FLJ38723 | 1 9 1 |
| 8103 722 | ENST0000032 5407 /// A Y956762 | Heat shock protein 90Af gene: ENSG00000181359 /// Homo sapiens heat shock protein 90Af (HSP90Af) mRNA, complete cds. | heat shock protein 90kDa alpha (cytosolic), class A member 6 (pseudogene) | HSP90AA 6P | 1 9 2 |
| 8035 146 | ENST0000040 9035 /// ENST0000026 9881 /// BC014595 /// NM_145046 | cdna:known chromosome: NCBI36:19:16450 878: 16600015:-1 gene: ENSG00000141979 /// Calreticulin-3 gene: ENSG00000141979 /// Homo sapiens calreticulin 3, mRNA (cDNA clone MGC:26577 IMAGE:4822010), complete cds. /// Homo sapiens calreticulin 3 (CALR3), mRNA. | calreticulin 3 | CALR3 | 1 9 3 |

(continued)

| Probe Set ID | Transcript ID | Transcript Description | Gene Title | Gene Symbol | |
|---|---|---|---|---|---|
| 8150 722 | ENST0000027 6480 /// AB011107 /// NM_014682 | Suppression of tumorigenicity protein 18 gene: ENSG00000147488 /// Homo sapiens mRNA for KIAA0535 protein, complete cds. /// Homo sapiens suppression of tumorigenicity 18 (breast carcinoma) (zinc finqer protein) (ST18), mRNA. | suppression of tumorigenicity 18 (breast carcinoma) (zinc finger protein) | ST18 | 1 9 4 |
| 8053 715 | GENSCAN000 00025928 /// ENST0000031 2946 /// ENST0000040 2897 | cdna:Genscan chromosome: NCBI36:2:892092 83:89223706:-1 /// cdna:pseudogene chromosome:NCBI36:2:892093 04:89210080:-1 gene: ENSG00000204732 /// cdna: pseudogene chromosome: NCBI36:2:897287 32:89729523: 1 gene:ENSG00000218041 | --- | --- | 1 9 5 |
| 7922 976 | ENST0000036 7468 /// AY151286 /// NM_000963 | Prostaglandin G/H synthase 2 gene:ENSG00000073756 /// Homo sapiens cyclooxygenase 2b mRNA, complete cds; alternatively spliced. /// Homo sapiens prostaglandin-endoperoxide synthase 2 (prostaglandin G/H synthase and cyclooxygenase) (PTGS2), mRNA. | prostaglandin-endoperoxide synthase 2 (prostaglandin G/H synthase and cyclooxygenase) | PTGS2 | 1 9 6 |
| 8087 433 | ENST0000027 3588 /// ENST0000039 5338 /// ENST0000027 3598 /// AF538150 /// D13811 /// NM_ 000481 /// NM_ 032316 | Aminomethyltransferase, mitochondrial gene: ENSG00000145020 /// Aminomethyltransferase gene: ENSG00000145020 /// Isoform 1 of Nicolin-1 gene: ENSG00000145029 /// Homo sapiens NPCEDRGP (NPCEDRG) mRNA, NPCEDRG-s allele, complete cds. /// Homo sapiens mRNA for glycine cleavage system T-protein, complete cds. /// Homo sapiens aminomethyltransferase (AMT), mRNA. /// Homo sapiens nicolin 1 (NICN1), mRNA. | nicolin 1 /// aminomethyltransfera se | NICN1 /// AMT | 1 9 7 |

(continued)

| Probe Set ID | Transcript ID | Transcript Description | Gene Title | Gene Symbol | |
|---|---|---|---|---|---|
| 8075 142 | ENST0000039 7906 /// ENST0000026 6082 /// AB028966 /// BC016465 | Tetratricopeptide repeat protein 28 gene:ENSG00000100154 /// Tetratricopeptide repeat protein 28 gene:ENSG00000100154 /// Homo sapiens mRNA for KIAA1043 protein, partial cds. /// Homo sapiens tetratricopeptide repeat domain 28, mRNA (cDNA clone MGC:18145 IMAGE: 4154050), complete cds. | tetratricopeptide repeat domain 28 | TTC28 | 1 9 8 |
| 7919 751 | ENST0000030 7940 /// ENST0000036 9026 /// BC017197 /// NM_182763 /// NM_021960 | Isoform 2 of Induced myeloid leukemia cell differentiation protein Mcl-1 gene: ENSG00000143384 /// Isoform 1 of Induced myeloid leukemia cell differentiation protein Mcl-1 gene: ENSG00000143384 /// Homo sapiens myeloid cell leukemia sequence 1 (BCL2-related), mRNA (cDNA clone MGC:1839 IMAGE:3138465), complete cds. /// Homo sapiens myeloid cell leukemia sequence 1 (BCL2-related) (MCL1), transcript variant 2, mRNA. /// Homo sapiens myeloid cell leukemia sequence 1 (BCL2-related) (MCL1), transcript variant 1, mRNA. | myeloid cell leukemia sequence 1 (BCL2-related) | MCL1 | 1 9 9 |

(continued)

| Probe Set ID | Transcript ID | Transcript Description | Gene Title | Gene Symbol | |
|---|---|---|---|---|---|
| 8000 590 | ENST0000031 4752 /// ENST0000039 5609 /// ENST0000039 5607 /// ENST0000035 0842 /// AB209149 /// NM_177529 /// NM_177530 /// NM_177536 /// NM_001055/// NM_177534 | Sulfotransferase 1A1 gene: ENSG00000196502 /// Sulfotransferase 1A1 gene: ENSG00000196502 /// Sulfotransferase 1A1 gene: ENSG00000196502 /// sulfotransferase family, cytosolic, 1A, phenol-preferring, member 1 isoform b gene: ENSG00000196502 /// Homo sapiens mRNA for Phenol-sulfating phenol sulfotransferase 1 variant protein. /// Homo sapiens sulfotransferase family, cytosolic, 1A, phenol-preferring, member 1 (SULT1A1), transcript variant 2, mRNA. /// Homo sapiens sulfotransferase family, cytosolic, 1A, phenol-preferring, member 1 (SULT1A1), transcript variant 3, mRNA. /// Homo sapiens sulfotransferase family, cytosolic, 1A, phenol-preferring, member 1 (SULT1A1), transcript variant 5, mRNA. /// Homo sapiens sulfotransferase family, cytosolic, 1A, phenol-preferring, member 1 (SULT1A1), transcript variant 1, mRNA. /// Homo sapiens sulfotransferase family, cytosolic, 1A, phenol-preferring, member 1 (SULT1A1), transcript variant 4, mRNA. | sulfotransferase family, cytosolic, 1A, phenol-preferring, member 1 | SULT1A1 | 2 0 0 |
| 7973 444 | ENST0000038 6719 /// ENST0000041 0999 | ncrna:snRNA_pseudogene chromosome:NCBI36:14:23330 971:23331182:1 gene: ENSG00000209454 /// ncrna: misc_RNA chromosome: NCBI36:14:23330 979: 23331267:1 gene: ENSG00000222931 | --- | --- | 2 0 1 |
| 8072 004 | ENST0000038 2738 /// NM_ 00101361 8 | immunoglobulin lambda-like polypeptide 3 gene: ENSG00000206066 /// Homo sapiens immunoglobulin lambda-like polypeptide 3 (IGLL3), mRNA. | immunoglobulin lambda-like polypeptide 3 | IGLL3 | 2 0 2 |

(continued)

| Probe Set ID | Transcript ID | Transcript Description | Gene Title | Gene Symbol | |
|---|---|---|---|---|---|
| 7982 574 | ENST0000039 7609 /// ENST0000030 5752 /// AK095745 /// NM_173611 /// NM_00104242 9 | family with sequence similarity 98, member B isoform 1 gene: ENSG00000171262 /// Protein FAM98B gene: ENSG00000171262 /// Homo sapiens cDNA FLJ38426 fis, clone FEBRA2012507. /// Homo sapiens family with sequence similarity 98, member B (FAM98B), transcript variant 1, mRNA. /// Homo sapiens family with sequence similarity 98, member B (FAM98B), transcript variant 2, mRNA. | family with sequence similarity 98, member B | FAM98B | 2 0 3 |
| 8135 436 | ENST0000026 5715 /// AF030880 /// NM_000441 | Pendrin gene: ENSG00000091137 /// Homo sapiens pendrin (PDS) mRNA, complete cds. /// Homo sapiens solute carrier family 26, member 4 (SLC26A4), mRNA. | solute carrier family 26, member 4 | SLC26A4 | 2 0 4 |
| 8180 281 | --- | --- | --- | --- | 2 0 5 |
| 8080 676 | ENST0000031 1180 /// AK300374 /// NM_177966 | Isoform 1 of 2',5'-phosphodiesterase 12 gene: ENSG00000174840 /// Homo sapiens cDNA FLJ54489 complete cds, highly similar to Homo sapiens 2'-phosphodiesterase (2'-PDE), mRNA. /// Homo sapiens phosphodiesterase 12 (PDE12), mRNA. | phosphodiesterase 12 | PDE12 | 2 0 6 |
| 8154 135 | ENST0000026 2352 /// ENST0000038 1910 /// BC033040 /// NM_004170 | Excitatory amino acid transporter 3 gene:ENSG00000106688 /// Solute carrier family 1 (Neuronal/ epithelial high affinity glutamate transporter, system member 1 gene:ENSG00000106688 /// Homo sapiens solute carrier family 1 (neuronal/epithelial high affinity glutamate transporter, system Xag), member 1, mRNA (cDNA clone MGC:33786 IMAGE:5261168), complete cds. /// Homo sapiens solute carrier family 1 (neuronal/epithelial high affinity glutamate transporter, system Xag), member 1 (SLC1A1), mRNA. | solute carrier family 1 (neuronal/epithelial high affinity glutamate transporter, system Xag), member 1 | SLC1A1 | 2 0 7 |

(continued)

| Probe Set ID | Transcript ID | Transcript Description | Gene Title | Gene Symbol | |
|---|---|---|---|---|---|
| 8091 306 | ENST0000035 4952 /// ENST0000038 3083 /// AF199023 /// NM_00112830 5 /// NM_020353 /// NM_00112830 4 /// NM_ 00112830 6 | Phospholipid scramblase 4 gene: ENSG00000114698 /// phospholipid scramblase 4 isoform b gene: ENSG00000114698 /// Homo sapiens phospholipid scramblase 4 mRNA, complete cds. /// Homo sapiens phospholipid scramblase 4 (PLSCR4), transcript variant 3, mRNA. /// Homo sapiens phospholipid scramblase 4 (PLSCR4), transcript variant 2, mRNA. /// Homo sapiens phospholipid scramblase 4 (PLSCR4), transcript variant 1, mRNA. /// Homo sapiens phospholipid scramblase 4 (PLSCR4), transcript variant 4, mRNA. | phospholipid scramblase 4 | PLSCR4 | 2 0 8 |
| 7971 375 | ENST0000037 9056 /// ENST0000037 9060 /// ENST0000037 9055 /// ENST0000030 9246 /// BC040008 /// NM_003295 | Tumor protein, translationally-controlled 1, isoform CRA_a gene:ENSG00000133112 /// Translationally-controlled tumor protein gene: ENSG00000133112 /// Tumor protein, translationally-controlled 1, isoform CRA_a gene: ENSG00000133112 /// Tumor protein, translationally-controlled 1 gene:ENSG00000133112 /// Homo sapiens tumor protein, translationally-controlled 1, mRNA (cDNA clone IMAGE: 5219529), complete cds. /// Homo sapiens tumor protein, translationally-controlled 1 (TPT1), mRNA. | tumor protein, translationally-controlled 1 | TPT1 | 2 0 9 |
| 7981 998 | NR_003339 | Homo sapiens small nucleolar RNA, C/D box 116-25 (SNORD116-25), non-coding RNA. | small nuclear ribonucleoprotein polypeptide N /// small nucleolar RNA, C/D box 116-25 | SNRPN /// SNORD11 6-25 | 2 1 0 |
| 7905 077 | BC068044 | Homo sapiens cDNA clone IMAGE:6380649, containing frame-shift errors. | --- | --- | 2 1 1 |

(continued)

| Probe Set ID | Transcript ID | Transcript Description | Gene Title | Gene Symbol | |
|---|---|---|---|---|---|
| 7912 349 | ENST0000037 7008 /// ENST0000037 7004 /// AK094437 /// AK095152 /// BC126349 | Isoform 1 of Uncharacterized protein C1orf127 gene: ENSG00000175262 /// Isoform 2 of Uncharacterized protein C1orf127 gene: ENSG00000175262 /// Homo sapiens cDNA FLJ37118 fis, clone BRACE2022328. /// Homo sapiens cDNA FLJ37833 fis, clone BRSSN2009702. /// Homo sapiens chromosome 1 open reading frame 127, mRNA (cDNA clone MGC:161627 IMAGE: 8992065), complete cds. | chromosome 1 open reading frame 127 | C1orf127 | 2 1 2 |
| 8091 097 | XR_040865 | PREDICTED: Homo sapiens misc_RNA (FLJ11827), miscRNA. | hypothetical protein FLJ11827 | FLJ11827 | 2 1 3 |
| 7904 287 | ENST0000036 94787// ENST0000036 9477 /// BC033583 /// NM_001767 | T-cell surface antigen CD2 gene: ENSG00000116824 /// CD2 molecule gene: ENSG00000116824 /// Homo sapiens CD2 molecule, mRNA (cDNA clone MGC:34621 IMAGE:5227138), complete cds. /// Homo sapiens CD2 molecule (CD2), mRNA. | CD2 molecule | CD2 | 2 1 4 |
| 8043 100 | ENST0000023 3143 /// BC1 07889 /// NM_ 021103 | Thymosin beta-10 gene: ENSG00000034510 /// Homo sapiens cDNA clone IMAGE: 6651898. /// Homo sapiens thymosin beta 10 (TMSB10), mRNA. | thymosin beta 10 | TMSB10 | 2 1 5 |

(continued)

| Probe Set ID | Transcript ID | Transcript Description | Gene Title | Gene Symbol | |
|---|---|---|---|---|---|
| 8177 323 | ENST0000030 3804/// ENST0000034 1740 /// ENST0000033 8793 /// ENST0000030 3728 /// ENST0000034 3584 /// ENST0000030 3593 /// ENST0000030 6589 /// ENST0000033 8673 /// AF517635 /// NM_00100275 8 /// NM_004676 | Isoform 1 of PTPN13-like protein, Y-linked gene: ENSG00000169807 /// Isoform 2 of PTPN13-like protein, Y-linked gene:ENSG00000169807 /// Isoform 2 of PTPN13-like protein, Y-linked gene: ENSG00000169789 /// Isoform 1 of PTPN13-like protein, Y-linked gene:ENSG00000169789 /// Isoform 1 of PTPN13-like protein, Y-linked gene: ENSG00000169763 /// Isoform 2 of PTPN13-like protein, Y-linked gene:ENSG00000169763 /// Isoform 1 of PTPN13-like protein, Y-linked gene: ENSG00000172283 /// Isoform 2 of PTPN13-like protein, Y-linked gene:ENSG00000172283 /// Homo sapiens testis-specific PTP-BL related Y protein mRNA, complete cds, alternatively spliced. /// Homo sapiens PTPN13-like, Y-linked 2 (PRY2), mRNA. /// Homo sapiens PTPN13-like, Y-linked (PRY), mRNA. | PTPN13-like, Y-linked /// PTPN13-like, Y-linked 2 | PRY /// PRY2 | 2 1 6 |
| 7920 633 | ENST0000036 8399 /// ENST0000034 1298 /// ENST0000036 8400 /// AK293625 /// NM_153741 /// NM_018973 | cDNA FLJ60436, highly similar to Homo sapiens dolichyl-phosphate mannosyltransferase polypeptide 3, transcript variant 1, mRNA gene:ENSG00000179085 /// Isoform 1 of Dolichol-phosphate mannosyltransferase subunit 3 gene: ENSG00000179085 /// Isoform 1 of Dolichol-phosphate mannosyltransferase subunit 3 gene:ENSG00000179085 /// Homo sapiens cDNA FLJ60436 complete cds, highly similar to Homo sapiens dolichyl-phosphate mannosyltransferase polypeptide 3, transcript variant 1, mRNA. /// Homo sapiens dolichyl-phosphate mannosyltransferase polypeptide 3 (DPM3), transcript variant 2, mRNA. /// Homo sapiens dolichyl-phosphate mannosyltransferase polypeptide 3 (DPM3), transcript variant 1, mRNA. | dolichyl-phosphate mannosyltransferase polypeptide 3 | DPM3 | 2 1 7 |

(continued)

| Probe Set ID | Transcript ID | Transcript Description | Gene Title | Gene Symbol | |
|---|---|---|---|---|---|
| 8110 916 | ENST0000038 2550 /// AK124118 | cDNA FLJ42124 fis, clone TESTI2009477, weakly similar to TRICHOHYALIN gene: ENSG00000205976 /// Homo sapiens cDNA FLJ42124 fis, clone TESTI2009477, weakly similar to TRICHOHYALIN. | similar to hypothetical protein FLJ36144 | LOC4421 32 | 2 1 8 |
| 7943 051 | ENST0000032 1955 /// ENST0000037 5944 /// BC096316 /// NM_005467 | N-acetylated-alpha-linked acidic dipeptidase 2 gene: ENSG00000077616 /// NAALAD2 protein gene: ENSG00000077616 /// Homo sapiens N-acetylated alpha-linked acidic dipeptidase 2, mRNA (cDNA clone MGC:116994 IMAGE:40007638), complete cds. /// Homo sapiens N-acetylated alpha-linked acidic dipeptidase 2 (NAALAD2), mRNA. | N-acetylated alpha-linked acidic dipeptidase 2 | NAALAD2 | 2 1 9 |
| 7938 951 | ENST0000032 4559 /// AL833271 /// NM_213599 | Anoctamin-5 gene: ENSG00000171714 /// Homo sapiens mRNA; cDNA DKFZp451A148 (from clone DKFZp451A148). /// Homo sapiens anoctamin 5 (ANO5), mRNA. | anoctamin 5 | ANO5 | 2 2 0 |
| 8083 839 | ENST0000040 2305 /// ENST0000035 5897 /// BC000181 /// NM_014373 | Probable G-protein coupled receptor 160 gene: ENSG00000173890 /// Probable G-protein coupled receptor 160 gene:ENSG00000173890 /// Homo sapiens G protein-coupled receptor 160, mRNA (cDNA clone MGC:5003 IMAGE:3048193), complete cds. /// Homo sapiens G protein-coupled receptor 160 (GPR160), mRNA. | G protein-coupled receptor 160 | GPR160 | 2 2 1 |

(continued)

| Probe Set ID | Transcript ID | Transcript Description | Gene Title | Gene Symbol | |
|---|---|---|---|---|---|
| 8046 020 | ENST0000037 5437 /// ENST0000035 7398 /// ENST0000028 3256 /// ENST0000037 5427 /// AB208888 /// NM_021007 /// NM_00104014 2/// NM_ 00104014 3 | Isoform 1 of Sodium channel protein type 2 subunit alpha gene: ENSG00000136531 /// Isoform 2 of Sodium channel protein type 2 subunit alpha gene: ENSG00000136531 /// Isoform 1 of Sodium channel protein type 2 subunit alpha gene: ENSG00000136531 /// Isoform 2 of Sodium channel protein type 2 subunit alpha gene: ENSG00000136531 /// Homo sapiens mRNA for Sodium channel protein type II alpha subunit variant protein. /// Homo sapiens sodium channel, voltage-gated, type II, alpha subunit (SCN2A), transcript variant 1, mRNA. /// Homo sapiens sodium channel, voltage-gated, type II, alpha subunit (SCN2A), transcript variant 2, mRNA. /// Homo sapiens sodium channel, voltage-gated, type II, alpha subunit (SCN2A), transcript variant 3, mRNA. | sodium channel, voltage-gated, type II, alpha subunit | SCN2A | 2 2 2 |
| 8127 658 | ENST0000036 4421 | ncrna:snRNA chromosome: NCBI36:6: 762401 74: 76240330:-1 gene: ENSG00000201291 | --- | --- | 2 2 3 |
| 8129 097 | AK096882 | Homo sapiens cDNA FLJ39563 fis, clone SKMUS2001164. | TSPY-like 1 | TSPYL1 | 2 2 4 |
| 7903 079 | ENST0000037 0272 /// ENST0000037 0267 /// BC035507 /// NM_001938 | Protein Dr1 gene: ENSG00000117505 /// Protein Dr1 gene:ENSG00000117505 /// Homo sapiens down-regulator of transcription 1, TBP-binding (negative cofactor 2), mRNA (cDNA clone MGC:29766 IMAGE:4555131), complete cds. /// Homo sapiens down-regulator of transcription 1, TBP-binding (negative cofactor 2) (DR1), mRNA. | down-regulator of transcription 1, TBP-binding (negative cofactor 2) | DR1 | 2 2 5 |
| 8110 104 | ENST0000041 0179 | ncrna:misc_RNA chromosome: NCBI36:5:174987 797: 174988108:1 gene: ENSG00000222111 | --- | --- | 2 2 6 |

(continued)

| Probe Set ID | Transcript ID | Transcript Description | Gene Title | Gene Symbol | |
|---|---|---|---|---|---|
| 8127 145 | ENST0000030 4434 /// ENST0000037 0918 /// AF338241 /// NM_021814 | Elongation of very long chain fatty acids protein 5 gene: ENSG00000012660 /// 35 kDa protein gene: ENSG00000012660 /// Homo sapiens elongation of very long chain fatty acids protein-like protein 2 (ELOVL2) mRNA, complete cds. /// Homo sapiens ELOVL family member 5, elongation of long chain fatty acids (FEN1/Elo2, SUR4/Elo3-like, yeast) (ELOVL5), mRNA. | ELOVL family member 5, elongation of long chain fatty acids (FEN1/Elo2, SUR4/Elo3-like, yeast) | ELOVL5 | 2 2 7 |
| 8175 299 | ENST0000039 1440 /// BC086860 /// NM_00107817 3 | Protein FAM127C gene: ENSG00000212747 /// Homo sapiens cDNA clone IMAGE: 6153002. /// Homo sapiens family with sequence similarity 127, member C (FAM127C), mRNA. | family with sequence similarity 127, member C | FAM127C | 2 2 8 |
| 8033 248 | ENST0000024 5912 /// AF064090 /// NM_172014 /// NM 003807 | tumor necrosis factor ligand superfamily, member 14 isoform 1 precursor gene: ENSG00000125735 /// Homo sapiens ligand for herpesvirus entry mediator (HVEM-L) mRNA, complete cds. /// Homo sapiens tumor necrosis factor (ligand) superfamily, member 14 (TNFSF14), transcript variant 2, mRNA. /// Homo sapiens tumor necrosis factor (ligand) superfamily, member 14 (TNFSF14), transcript variant 1, mRNA. | tumor necrosis factor (ligand) superfamily, member 14 | TNFSF14 | 2 2 9 |
| 7933 075 | ENST0000037 4694 /// AB043703 /// NM_031866 | Frizzled-8 gene: ENSG00000177283 /// Homo sapiens FZD8 mRNA for seven-transmembrane receptor Frizzled-8, complete cds. /// Homo sapiens frizzled homolog 8 (Drosophila) (FZD8), mRNA. | frizzled homolog 8 (Drosophila) | FZD8 | 2 3 0 |

(continued)

| Probe Set ID | Transcript ID | Transcript Description | Gene Title | Gene Symbol | |
|---|---|---|---|---|---|
| 7915 870 | ENST0000037 1937 /// ENST0000032 9231 /// BC008498 /// NM_022745 /// NM_00104254 6 | ATP synthase mitochondrial F1 complex assembly factor 1 gene: ENSG00000123472 /// ATP synthase mitochondrial F1 complex assembly factor 1 isoform 2 precursor gene: ENSG00000123472 /// Homo sapiens ATP synthase mitochondrial F1 complex assembly factor 1, mRNA (cDNA clone MGC:14830 IMAGE: 4281102), complete cds. /// Homo sapiens ATP synthase mitochondrial F1 complex assembly factor 1 (ATPAF1), nuclear gene encoding mitochondrial protein, transcript variant 1, mRNA. /// Homo sapiens ATP synthase mitochondrial F1 complex assembly factor 1 (ATPAF1), nuclear gene encoding mitochondrial protein, transcript variant 2, mRNA. | ATP synthase mitochondrial F1 complex assembly factor 1 | ATPAF1 | 2 3 1 |
| 8121 064 | ENST0000038 8700 /// ENST0000041 1294 | ncrna:scRNA_pseudogene chromosome:NCBI36:6:886120 87:88612384:1 gene: ENSG00000211435 /// ncrna: misc_RNA chromosome: NCBI36:6:886120 87:88612382: 1 qene:ENSG00000223226 | --- | - | 2 3 2 |
| 8161 437 | GENSCAN000 00015556 /// ENST0000040 7551 /// XM_ 00171438 0 | cdna:Genscan chromosome: NCBI36:9:460071 63:46060142:- 1 /// cdna:pseudogene chromosome: NCBI36:9:462770 08:46277218:1 gene: ENSG00000216653 /// PREDICTED: Homo sapiens similar to hCG1656091 (LOC100132357), mRNA. | similar to hCG1656091 | LOC1001 32357 | 2 3 3 |
| 8008 980 | ENST0000033 5108 /// BC046200 | Putative uncharacterized protein C17orf82 gene: ENSG00000187013 /// Homo sapiens chromosome 17 open reading frame 82, mRNA (cDNA clone MGC:57831 IMAGE: 6152618), complete cds. | chromosome 17 open reading frame 82 | C17orf82 | 2 3 4 |

(continued)

| Probe Set ID | Transcript ID | Transcript Description | Gene Title | Gene Symbol | |
|---|---|---|---|---|---|
| 8154 563 | ENST0000034 0967 /// ENST0000038 0376 /// BC092487 /// NM_00101088 7 | Isoform 1 of Alkaline ceramidase 2 gene:ENSG00000177076 /// Isoform 3 of Alkaline ceramidase 2 gene:ENSG00000177076 /// Homo sapiens N-acylsphingosine amidohydrolase 3-like, mRNA (cDNA clone MGC:104688 IMAGE:30528463), complete cds. /// Homo sapiens N-acylsphingosine amidohydrolase 3-like (ASAH3L), mRNA. | alkaline ceramidase 2 | ACER2 | 2 3 5 |
| 8161 426 | GENSCAN000 00015556 /// ENST0000040 7551 /// XM_ 00171438 0 | cdna:Genscan chromosome: NCBI36:9:460071 63:46060142:-1 /// cdna:pseudogene chromosome: NCBI36:9:462770 08:46277218:1 gene: ENSG00000216653 /// PREDICTED: Homo sapiens similar to hCG1656091 (LOC100132357), mRNA. | similar to hCG1656091 | LOC1001 32357 | 2 3 6 |
| 8083 223 | ENST0000031 5691 /// BC037293 /// NM_173552 /// NM_00113447 0 | UPF0672 protein C3orf58 gene: ENSG00000181744 /// Homo sapiens chromosome 3 open reading frame 58, mRNA (cDNA clone MGC:33365 IMAGE: 5267770), complete cds. /// Homo sapiens chromosome 3 open reading frame 58 (C3orf58), transcript variant 1, mRNA. /// Homo sapiens chromosome 3 open reading frame 58 (C3orf58), transcript variant 2, mRNA. | chromosome 3 open reading frame 58 | C3orf58 | 2 3 7 |
| 8001 329 | ENST0000021 9197 /// M58583 /// NM_004352 | Cerebellin-1 gene: ENSG00000102924 /// Human precerebellin and cerebellin mRNA, complete cds. /// Homo sapiens cerebellin 1 precursor (CBLN1), mRNA. | cerebellin 1 precursor | CBLN1 | 2 3 8 |

(continued)

| Probe Set ID | Transcript ID | Transcript Description | Gene Title | Gene Symbol | |
|---|---|---|---|---|---|
| 8165 575 | ENST0000037 1457 /// ENST0000027 7531 /// ENST0000040 6427 /// ENST0000037 1451 /// ENST0000037 1450 /// ENST0000037 1446/// AK297623 /// NM_152286 /// NM_00109853 7 | cDNA FLJ45411 fis, clone BRHIP3032374, moderately similar to Homo sapiens neuropathy target esterase gene: ENSG00000130653 /// Isoform 1 of Patatin-like phospholipase domain-containing protein 7 gene:ENSG00000130653 /// patatin-like phospholipase domain containing 7 isoform a gene:ENSG00000130653 /// Putative uncharacterized protein PNPLA7 gene: ENSG00000130653 /// Isoform 2 of Patatin-like phospholipase domain-containing protein 7 gene:ENSG00000130653 /// 23 kDa protein gene: ENSG00000130653 /// Homo sapiens cDNA FLJ55553 complete cds. /// Homo sapiens patatin-like phospholipase domain containing 7 (PNPLA7), transcript variant 2, mRNA. /// Homo sapiens patatin-like phospholipase domain containing 7 (PNPLA7), transcript variant 1, mRNA. | patatin-like phospholipase domain containing 7 | PNPLA7 | 2 3 9 |
| 7971 731 | ENST0000040 0366 /// ENST0000034 4297 /// ENST0000024 2839 /// ENST0000040 0370 /// U11700 /// NM_000053 /// NM_00100591 8 | Isoform 3 of Coppertransporting ATPase 2 gene: ENSG00000123191 /// Isoform 2 of Coppertransporting ATPase 2 gene:ENSG00000123191 /// Isoform 1 of Coppertransporting ATPase 2 gene: ENSG00000123191 /// ATP7B protein gene: ENSG00000123191 /// Human copper transporting ATPase mRNA, complete cds. /// Homo sapiens ATPase, Cu++ transporting, beta polypeptide (ATP7B), transcript variant 1, mRNA. /// Homo sapiens ATPase, Cu++ transporting, beta polypeptide (ATP7B), transcript variant 2, mRNA. | ATPase, Cu++ transporting, beta polypeptide | ATP7B | 2 4 0 |

(continued)

| Probe Set ID | Transcript ID | Transcript Description | Gene Title | Gene Symbol | |
|---|---|---|---|---|---|
| 8028 600 | ENST0000033 9852 /// BC092493 /// NM_00100141 4 | FBA domain-containing protein LOC342897 gene: ENSG00000188505 /// Homo sapiens similar to F-box only protein 2, mRNA (cDNA clone MGC:104713 IMAGE: 30337042), complete cds. /// Homo sapiens nonspecific cytotoxic cell receptor protein 1 homolog zebrafish (NCCRP1), mRNA. | non-specific cytotoxic cell receptor protein 1 homolog (zebrafish) | NCCRP1 | 2 4 1 |
| 8155 569 | ENST0000040 7551 /// XM_ 00171438 0 | cdna:pseudogene chromosome: NCBI36:9:462770 08:46277218: 1 gene:ENSG00000216653 /// PREDICTED: Homo sapiens similar to hCG1656091 (LOC100132357), mRNA. | similar to hCG1656091 | LOC1001 32357 | 2 4 2 |
| 8039 605 | ENST0000034 2088 /// AK023017 /// NM_00100585 0 | Zinc finger protein 835 gene: ENSG00000127903 /// Homo sapiens cDNA FLJ12955 fis, clone NT2RP2005496, moderately similar to ZINC FINGER PROTEIN 135. /// Homo sapiens zinc finger protein 835 (ZNF835), mRNA. | zinc finger protein 835 | ZNF835 | 2 4 3 |
| 7937 971 | ENST0000032 2493 | cdna:pseudogene chromosome: NCBI36:11:47645 60:4765496:1 gene:ENSG00000176951 | --- | --- | 2 4 4 |
| 8137 925 | ENST0000038 4168 | ncrna:misc_RNA chromosome: NCBI36:7:481734 5:4817446:-1 gene:ENSG00000206895 | --- | --- | 2 4 5 |
| 7915 408 | ENST0000037 2572 /// ENST0000037 2571 /// ENST0000037 2573 /// ENST0000036 1346/// ENST0000036 1776 /// BC152441 /// NM_014947 | Isoform 1 of Forkhead box protein J3 gene:ENSG00000198815 /// 15 kDa protein gene: ENSG00000198815 /// Isoform 1 of Forkhead box protein J3 gene: ENSG00000198815 /// Isoform 1 of Forkhead box protein J3 gene: ENSG00000198815 /// Isoform 2 of Forkhead box protein J3 gene: ENSG00000198815 /// Homo sapiens forkhead box J3, mRNA (cDNA clone MGC:176686 IMAGE:8862565), complete cds. /// Homo sapiens forkhead box J3 (FOXJ3), mRNA. | forkhead box J3 | FOXJ3 | 2 4 6 |

(continued)

| Probe Set ID | Transcript ID | Transcript Description | Gene Title | Gene Symbol | |
|---|---|---|---|---|---|
| 8014 891 | ENST0000039 4189 /// ENST0000037 7944 /// ENST0000034 8427 /// ENST0000034 6872 /// ENST0000037 7958 /// ENST0000029 3068 /// ENST0000035 1680 /// ENST0000035 0532 /// ENST0000037 7945 /// ENST0000034 6243 /// ENST0000037 7952 /// AY377981 /// NM_183230 /// NM_183231 /// NM _183232 /// NM_012481 /// NM _183228 /// NM_183229 | Aiolos isoform hAio-del gene: ENSG00000161405 /// Aiolos isoform hAio-del gene: ENSG00000161405 /// Isoform 2 of Zinc finger protein Aiolos gene: ENSG00000161405 /// Isoform 5 of Zinc finger protein Aiolos gene: ENSG00000161405 /// Aiolos isoform hAio-del gene: ENSG00000161405 /// Isoform 1 of Zinc finger protein Aiolos gene: ENSG00000161405 /// Isoform 3 of Zinc finger protein Aiolos gene: ENSG00000161405 /// Isoform 4 of Zinc finger protein Aiolos gene: ENSG00000161405 /// Aiolos isoform hAio-del gene: ENSG00000161405 /// Isoform 6 of Zinc finger protein Aiolos gene: ENSG00000161405 /// Aiolos isoform hAio-del gene: ENSG00000161405 /// Homo sapiens aiolos isoform hAio-ALT (ZNFN1A3) mRNA, complete cds, alternatively spliced. /// Homo sapiens IKAROS family zinc finger 3 (Aiolos) (IKZF3), transcript variant 4, mRNA. /// Homo sapiens IKAROS family zinc finger 3 (Aiolos) (IKZF3), transcript variant 5, mRNA. /// Homo sapiens IKAROS family zinc finger 3 (Aiolos) (IKZF3), transcript variant 6, mRNA. /// Homo sapiens IKAROS family zinc finger 3 (Aiolos) (IKZF3), transcript variant 1, mRNA. /// Homo sapiens IKAROS family zinc finger 3 (Aiolos) (IKZF3), transcript variant 2, mRNA. /// Homo sapiens IKAROS family zinc finger 3 (Aiolos) (IKZF3), transcript variant 3, mRNA. | IKAROS family zinc finger 3 (Aiolos) | IKZF3 | 2 4 7 |
| 7999 406 | ENST0000038 6866 | ncrna:Mt_tRNA_pseudogene chromosome:NCBI36:16: 10722 924:10722982:-1 qene: ENSG00000209601 | --- | --- | 2 4 8 |

(continued)

| Probe Set ID | Transcript ID | Transcript Description | Gene Title | Gene Symbol | |
|---|---|---|---|---|---|
| 8128 087 | ENST0000036 9451 /// BC130344 /// NM_002042 | gamma-aminobutyric acid (GABA) receptor, rho 1 gene: ENSG00000146276 /// Homo sapiens gamma-aminobutyric acid (GABA) receptor, rho 1, mRNA (cDNA clone MGC: 163216 IMAGE:40146375), complete cds. /// Homo sapiens gamma-aminobutyric acid (GABA) receptor, rho 1 (GABRR1), mRNA. | gamma-aminobutyric acid (GABA) receptor, rho 1 | GABRR1 | 2 4 9 |
| 8011 968 | ENST0000022 5728 /// AF151883 /// NM_016060 | Mediator of RNA polymerase II transcription subunit 31 gene: ENSG00000108590 /// Homo sapiens CGI-125 protein mRNA, complete cds. /// Homo sapiens mediator complex subunit 31 (MED31), mRNA. | mediator complex subunit 31 | MED31 | 2 5 0 |
| 7950 555 | ENST0000040 4995 /// ENST0000040 7242 /// ENST0000026 0061 /// BC070079 /// NM_005512 /// NM_00112892 2 | Leucine-rich repeat-containing protein 32 gene: ENSG00000137507 /// Leucine-rich repeat-containing protein 32 gene:ENSG00000137507 /// Leucine-rich repeat-containing protein 32 gene: ENSG00000137507 /// Homo sapiens leucine rich repeat containing 32, mRNA (cDNA clone MGC:87399 IMAGE: 30344529), complete cds. /// Homo sapiens leucine rich repeat containing 32 (LRRC32), transcript variant 1, mRNA. /// Homo sapiens leucine rich repeat containing 32 (LRRC32), transcript variant 2, mRNA. | leucine rich repeat containing 32 | LRRC32 | 2 5 1 |
| 8012 535 | ENST0000032 9805 /// AY129026 /// NM_152599 | UPF0537 transmembrane protein gene:ENSG00000185156 /// Homo sapiens clone FP7072 unknown mRNA. /// Homo sapiens major facilitator superfamily domain containing 6-like (MFSD6L), mRNA. | major facilitator superfamily domain containing 6-like | MFSD6L | 2 5 2 |

(continued)

| Probe Set ID | Transcript ID | Transcript Description | Gene Title | Gene Symbol | |
|---|---|---|---|---|---|
| 7988 767 | ENST0000039 6402 /// ENST0000039 6404 /// ENST0000026 0433 /// ENST0000040 5913 /// AK291778 /// NM_031226 /// NM_000103 | Cytochrome P450 19A1 gene: ENSG00000137869 /// Cytochrome P450 19A1 gene: ENSG00000137869 /// Cytochrome P450 19A1 gene: ENSG00000137869 /// CYP19A1 protein gene: ENSG00000137869 /// Homo sapiens cDNA FLJ75846 complete cds, highly similar to Homo sapiens cytochrome P450, family 19, subfamily A, polypeptide 1 (CYP19A1), transcript variant 1, mRNA. /// Homo sapiens cytochrome P450, family 19, subfamily A, polypeptide 1 (CYP19A1), transcript variant 2, mRNA. /// Homo sapiens cytochrome P450, family 19, subfamily A, polypeptide 1 (CYP19A1), transcript variant 1, mRNA. | cytochrome P450, family 19, subfamily A, polypeptide 1 | CYP19A1 | 2 5 3 |
| 8036 291 | ENST0000035 5114/// ENST0000039 2173 /// ENST0000030 4116 /// BC068453 /// NM_00104247 4 /// NM_152477 | cDNA FLJ32728 fis, clone TESTI2001049, highly similar to Zinc finger protein 565 gene: ENSG00000196357 /// Zinc finger protein 565 gene: ENSG00000196357 /// Zinc finger protein 565 gene: ENSG00000196357 /// Homo sapiens zinc finger protein 565, mRNA (cDNA clone IMAGE: 30343899). /// Homo sapiens zinc finger protein 565 (ZNF565), transcript variant 1, mRNA. /// Homo sapiens zinc finger protein 565 (ZNF565), transcript variant 2, mRNA. | zinc finger protein 565 | ZNF565 | 2 5 4 |

(continued)

| Probe Set ID | Transcript ID | Transcript Description | Gene Title | Gene Symbol | |
|---|---|---|---|---|---|
| 8064 351 | ENST0000040 0227 /// ENST0000040 0217 /// ENST0000036 1797 /// ENST0000021 7244 /// ENST0000034 9736 /// ENST0000038 1973 /// AY112721 /// BC053532 /// NM_001895 /// NR_002207 /// NM_177559 /// NM_177560 | Casein kinase 2 alpha isoform gene:ENSG00000101266 /// casein kinase II alpha 1 subunit isoform b gene: ENSG00000101266 /// casein kinase II alpha 1 subunit isoform b gene:ENSG00000101266 /// Casein kinase II subunit alpha gene:ENSG00000101266 /// Casein kinase II subunit alpha gene:ENSG00000101266 /// CSNK2A1 protein gene: ENSG00000101266 /// Homo sapiens casein kinase II alpha subunit mRNA, complete cds. /// Homo sapiens casein kinase 2, alpha 1 polypeptide, mRNA (cDNA clone MGC:61540 IMAGE:3908058), complete cds. /// Homo sapiens casein kinase 2, alpha 1 polypeptide (CSNK2A1), transcript variant 2, mRNA. /// Homo sapiens casein kinase 2, alpha 1 polypeptide pseudogene (CSNK2A1 P), non-coding RNA. /// Homo sapiens casein kinase 2, alpha 1 polypeptide (CSNK2A1), transcript variant 1, mRNA. /// Homo sapiens casein kinase 2, alpha 1 polypeptide (CSNK2A1), transcript variant 3, mRNA. | casein kinase 2, alpha 1 polypeptide pseudogene /// casein kinase 2, alpha 1 polypeptide | CSNK2A1 P /// CSNK2A1 | 2 5 5 |
| 8131 719 | ENST0000040 9508 /// ENST0000032 8843 /// AK095018 /// NM_003777 | cdna:known chromosome: NCBI36:7:215493 58:21907711: 1 gene:ENSG00000105877 /// Dynein heavy chain 11, axonemal gene:ENSG00000105877 /// Homo sapiens cDNA FLJ37699 fis, clone BRHIP2016788. /// Homo sapiens dynein, axonemal, heavy chain 11 (DNAH11), mRNA. | dynein, axonemal, heavy chain 11 | DNAH11 | 2 5 6 |
| 8095 331 | ENST0000036 5299 | ncrna:misc_RNA chromosome: NCBI36:4:624546 26:62454721: 1 gene:ENSG00000202169 | --- | --- | 2 5 7 |
| 8180 232 | --- | --- | --- | --- | 2 5 8 |

(continued)

| Probe Set ID | Transcript ID | Transcript Description | Gene Title | Gene Symbol | |
|---|---|---|---|---|---|
| 8105 040 | ENST0000027 4276 /// U60805 /// NM_003999 | Isoform 1 of Oncostatin-M specific receptor subunit beta gene: ENSG00000145623 /// Human oncostatin-M specific receptor beta subunit (OSMRB) mRNA, complete cds. /// Homo sapiens oncostatin M receptor (OSMR), mRNA. | oncostatin M receptor | OSMR | 259 |
| 7924 495 | ENST0000039 1137 | ncrna:rRNA chromosome: NCBI36:1 :220936 485: 220936584:-1 pene: ENSG00000212439 | --- | --- | 260 |
| 8122 684 | ENST0000032 6669 /// AY340238 /// NM_00100225 5 | SMT3 suppressor of mif two 3 homolog 4 gene: ENSG00000177688 /// Homo sapiens small ubiquitinlike protein 4 mRNA, complete cds. /// Homo sapiens SMT3 suppressor of mif two 3 homolog 4 (S. cerevisiae) (SUMO4), mRNA. | SMT3 suppressor of mif two 3 homolog 4 (S. cerevisiae) | SUMO4 | 261 |
| 7940 622 | ENST0000030 6238 /// BC062693 /// NM_006552 | Secretoglobin family 1D member 1 gene:ENSG00000168515 /// Homo sapiens secretoglobin, family 1D, member 1, mRNA (cDNA clone MGC:71958 IMAGE:30327780), complete cds. /// Homo sapiens secretoglobin, family 1D, member 1 (SCGB1D1), mRNA. | secretoglobin, family 1D, member 1 | SCGB1D1 | 262 |
| 8092 654 | ENST0000029 6277 /// BC012328 /// NM_052969 | 60S ribosomal protein L39-like gene:ENSG00000163923 /// Homo sapiens ribosomal protein L39-like, mRNA (cDNA clone MGC:20168 IMAGE:4555759), complete cds. /// Homo sapiens ribosomal protein L39-like (RPL39L), mRNA. | ribosomal protein L39-like | RPL39L | 263 |
| 7944 867 | ENST0000036 3408 | ncrna:rRNA chromosome: NCBI36:11 :12401 1565: 124011685:1 qene: ENSG00000200278 | --- | --- | 264 |
| 7925 087 | ENST0000038 4108 | ncrna:snRNA chromosome: NCBl36:1:231034 386: 231034556:-1 gene: ENSG00000206835 | --- | --- | 265 |

(continued)

| Probe Set ID | Transcript ID | Transcript Description | Gene Title | Gene Symbol | |
|---|---|---|---|---|---|
| 7985 920 | ENST0000034 1735 /// BC111413 /// NM_00103995 8 _ | mesoderm posterior 2 homolog gene:ENSG00000188095 /// Homo sapiens mesoderm posterior 2 homolog (mouse), mRNA (cDNA clone MGC: 133018 IMAGE:40004357), complete cds. /// Homo sapiens mesoderm posterior 2 homolog (mouse) (MESP2), mRNA. | mesoderm posterior 2 homolog (mouse) | MESP2 | 2 6 6 |
| 7939 314 | ENST0000025 7831 /// AF203977 /// NM_012153 | Isoform 1 of ETS homologous factor gene:ENSG00000135373 /// Homo sapiens ETS-family transcription factor EHF (EHF) mRNA, complete cds. /// Homo sapiens ets homologous factor (EHF), mRNA. | ets homologous factor | EHF | 2 6 7 |
| 7979 179 | ENST0000039 5686 /// ENST0000035 9133 /// AF081886 /// NM_014584 | Putative uncharacterized protein ER01 L gene: ENSG00000197930 /// ER01-like protein alpha gene: ENSG00000197930 /// Homo sapiens ER01-like protein (ER01-L) mRNA, complete cds. /// Homo sapiens ER01-like (S. cerevisiae) (ER01L), mRNA. | ER01-like (S. cerevisiae) | ER01 L | 2 6 8 |
| 8123 819 | ENST0000037 9715 /// BC005291 /// NM_004280 /// NM_00113565 0 | Eukaryotic translation elongation factor 1 epsilon-1 gene: ENSG00000124802 /// Homo sapiens eukaryotic translation elongation factor 1 epsilon 1, mRNA (cDNA clone MGC:12352 IMAGE:3685030), complete cds. /// Homo sapiens eukaryotic translation elongation factor 1 epsilon 1 (EEF1 E1), transcript variant 1, mRNA. /// Homo sapiens eukaryotic translation elongation factor 1 epsilon 1 (EEF1 E1), transcript variant 2, mRNA. | eukaryotic translation elongation factor 1 epsilon 1 | EEF1E1 | 2 6 9 |

(continued)

| Probe Set ID | Transcript ID | Transcript Description | Gene Title | Gene Symbol | |
|---|---|---|---|---|---|
| 8173 414 | ENST0000029 8085 /// ENST0000037 4299 /// BC033816 /// NM 032803 /// NM_ 00104816 4 | Cationic amino acid transporter 3 gene:ENSG00000165349 /// Cationic amino acid transporter 3 gene:ENSG00000165349 /// Homo sapiens solute carrier family 7 (cationic amino acid transporter, y+ system), member 3, mRNA (cDNA clone MGC: 44839 IMAGE:5206252), complete cds. /// Homo sapiens solute carrier family 7 (cationic amino acid transporter, y+ system), member 3 (SLC7A3), transcript variant 1, mRNA. /// Homo sapiens solute carrier family 7 (cationic amino acid transporter, y+ system), member 3 (SLC7A3), transcript variant 2, mRNA. | solute carrier family 7 (cationic amino acid transporter, y+ system), member 3 | SLC7A3 | 270 |
| 7952 797 | ENST0000029 9140 /// BC058039 /// NM_174927 | Spermatogenesis-associated protein 19, mitochondrial gene: ENSG00000166118 /// Homo sapiens spermatogenesis associated 19, mRNA (cDNA clone MGC:62071 IMAGE: 6619434), complete cds. /// Homo sapiens spermatogenesis associated 19 (SPATA19), mRNA. | spermatogenesis associated 19 | SPATA19 | 271 |
| 8031 387 | ENST0000029 1890 /// ENST0000033 8835 /// ENST0000035 0790 /// ENST0000035 7397 /// BC064806 /// NM_004829 | Isoform 1 of Natural cytotoxicity triggering receptor 1 gene: ENSG00000189430 /// Isoform 2 of Natural cytotoxicity triggering receptor 1 gene: ENSG00000189430 /// Isoform 3 of Natural cytotoxicity triggering receptor 1 gene: ENSG00000189430 /// Isoform 5 of Natural cytotoxicity triggering receptor 1 gene: ENSG00000189430 /// Homo sapiens natural cytotoxicity triggering receptor 1, mRNA (cDNA clone MGC:65100 IMAGE:5218848), complete cds. /// Homo sapiens natural cytotoxicity triggering receptor 1 (NCR1), mRNA. | natural cytotoxicity triggering receptor 1 | NCR1 | 272 |

(continued)

| Probe Set ID | Transcript ID | Transcript Description | Gene Title | Gene Symbol | |
|---|---|---|---|---|---|
| 8038 655 | ENST0000032 4041 /// AF113140 /// NM_004917 | Kallikrein-4 gene: ENSG00000167749 /// Homo sapiens serine protease prostase mRNA, complete cds. /// Homo sapiens kallikrein-related peptidase 4 (KLK4), mRNA. | kallikrein-related peptidase 4 | KLK4 | 2 7 3 |
| 8074 106 | ENST0000025 2783 /// ENST0000039 5676 /// BC009980 /// NM_138433 | Kelch domain-containing protein 7B gene:ENSG00000130487 /// kelch domain containing 7B gene: ENSG00000130487 /// Homo sapiens kelch domain containing 7B, mRNA (cDNA clone MGC: 16635 IMAGE:4121528), complete cds. /// Homo sapiens kelch domain containing 7B (KLHDC7B), mRNA. | kelch domain containing 7B | KLHDC7B | 2 7 4 |
| 8166 382 | ENST0000037 9484 /// ENST0000036 5779 /// AK292933 /// NM_015884 | Membrane-bound transcription factor site-2 protease gene: ENSG00000012174 /// 36 kDa protein gene: ENSG00000012174 /// Homo sapiens cDNA FLJ75833 complete cds, highly similar to Homo sapiens membrane-bound transcription factor peptidase, site 2 (MBTPS2), mRNA. /// Homo sapiens membrane-bound transcription factor peptidase, site 2 (MBTPS2), mRNA. | membrane-bound transcription factor peptidase, site 2 | MBTPS2 | 2 7 5 |
| 7965 884 | ENST0000030 7000 /// U49897 /// NM_000277 | Phenylalanine-4-hydroxylase gene:ENSG00000171759 /// Homo sapiens phenylalanine hydroxylase (PAH) mRNA, complete cds. /// Homo sapiens phenylalanine hydroxylase (PAH), mRNA. | phenylalanine hydroxylase | PAH | 2 7 6 |
| 8103 684 | ENST0000026 1511 /// BC010367 /// NM_017867 | UPF0609 protein C4orf27 gene: ENSG00000056050 /// Homo sapiens chromosome 4 open reading frame 27, mRNA (cDNA clone MGC:13432 IMAGE: 4334172), complete cds. /// Homo sapiens chromosome 4 open reading frame 27 (C4orf27), mRNA. | chromosome 4 open reading frame 27 | C4orf27 | 2 7 7 |

(continued)

| Probe Set ID | Transcript ID | Transcript Description | Gene Title | Gene Symbol | |
|---|---|---|---|---|---|
| 8139 592 | ENST0000025 8774 /// AF076844 /// NM_004507 | Checkpoint protein HUS1 gene: ENSG00000136273 /// Homo sapiens Hus1-like protein (HUS1) mRNA, complete cds. /// Homo sapiens HUS1 checkpoint homolog (S. pombe) (HUS1), mRNA. | HUS1 checkpoint homolog (S. pombe) | HUS1 | 2 7 8 |
| 8004 957 | ENST0000036 1801 /// ENST0000026 2442 /// ENST0000039 6001 /// AJ404468 /// NM_001372 /// _ NM_004662 | dynein, axonemal, heavy chain 9 isoform 1 gene: ENSG00000007174 /// Isoform 1 of Dynein heavy chain 9, axonemal gene: ENSG00000007174 /// dynein, axonemal, heavy chain 9 isoform 1 gene:ENSG00000007174 /// Homo sapiens mRNA for dynein heavy chain 9 (DNAH9 gene). /// Homo sapiens dynein, axonemal, heavy chain 9 (DNAH9), transcript variant 2, mRNA. /// Homo sapiens dynein, axonemal, heavy chain 9 (DNAH9), transcript variant 1, mRNA. | dynein, axonemal, heavy chain 9 | DNAH9 | 2 7 9 |
| 8130 553 | AK130765 | Homo sapiens cDNA FLJ27255 fis, clone SYN09519. | hypothetical LOC401281 | FLJ27255 | 2 8 0 |
| 8094 830 | ENST0000026 4452 /// AY659966 /// NM_018126 | Transmembrane protein 33 gene: ENSG00000109133 /// Homo sapiens SHINC3 (SHINC3) mRNA, complete cds. /// Homo sapiens transmembrane protein 33 (TMEM33), mRNA. | transmembrane protein 33 | TMEM33 | 2 8 1 |
| 8149 438 | ENST0000032 9135 /// ENST0000038 2080 /// AY028700 /// NM_139167 | Zeta-sarcoglycan gene: ENSG00000185053 /// sarcoglycan zeta gene: ENSG00000185053 /// Homo sapiens zeta-sarcoglycan mRNA, complete cds. /// Homo sapiens sarcoglycan zeta (SGCZ), mRNA. | sarcoglycan zeta | SGCZ | 2 8 2 |

(continued)

| Probe Set ID | Transcript ID | Transcript Description | Gene Title | Gene Symbol | |
|---|---|---|---|---|---|
| 8180 029 | ENST0000039 9426 /// ENST0000039 9424 /// ENST0000039 9427 /// ENST0000038 3099 /// ENST0000038 3245 /// ENST0000032 3109 /// ENST0000039 9658 /// ENST0000039 9661 /// ENST0000032 3143 /// ENST0000039 9053 /// ENST0000037 4931 /// ENST0000037 4934 /// AK098007 /// NR_003937 | Major histocompatibility complex, class II, DQ beta 2 gene: ENSG00000215008 /// Major histocompatibility complex, class II, DQ beta 2 gene: ENSG00000215008 /// Major histocompatibility complex, class II, DQ beta 2 gene: ENSG00000215008 /// Major histocompatibility complex, class II, DQ beta 2 gene: ENSG00000215008 /// Major histocompatibility complex, class II, DQ beta 2 gene: ENSG00000196610 /// Major histocompatibility complex, class II, DQ beta 2 gene: ENSG00000196610 /// Major histocompatibility complex, class II, DQ beta 2 gene: ENSG00000196610 /// cdna: known chromosome:NCBI36: c6_QBL: 32795451:32802909:-1 gene:ENSG00000196610 /// Major histocompatibility complex, class II, DQ beta 2 gene: ENSG00000204275 /// Major histocompatibility complex, class II, DQ beta 2 gene: ENSG00000204275 /// Major histocompatibility complex, class II, DQ beta 2 gene: ENSG00000204275 /// Homo sapiens cDNA FLJ40688 fis, clone THYMU2024185, highly similar to HLA class II histocompatibility antigen, DX beta chain precursor. /// Homo sapiens major histocompatibility complex, class II, DQ beta 2 (HLA-DQB2), non-codinp RNA. | major histocompatibility complex, class II, DQ beta 2 | HLA-DQB2 | 2 8 3 |
| 8015 060 | ENST0000026 4651 /// AK000268 /// NM_019016 | Keratin, type I cytoskeletal 24 gene:ENSG00000167916 /// Homo sapiens cDNA FLJ20261 fis, clone COLF7630. /// Homo sapiens keratin 24 (KRT24), mRNA. | keratin 24 | KRT24 | 2 8 4 |

(continued)

| Probe Set ID | Transcript ID | Transcript Description | Gene Title | Gene Symbol | |
|---|---|---|---|---|---|
| 8066 384 | ENST0000037 3005 /// ENST0000037 3003 /// BC040049 /// NM_176791 /// NM_00100890 1 | gametocyte specific factor 1-like isoform 2 gene: ENSG00000124196 /// Gametocyte-specific factor 1-like gene:ENSG00000124196 /// Homo sapiens gametocyte specific factor 1-like, mRNA (cDNA clone MGC:50820 IMAGE:5744556), complete cds. /// Homo sapiens gametocyte specific factor 1-like (GTSF1L), transcript variant 1, mRNA. /// Homo sapiens gametocyte specific factor 1-like (GTSF1L), transcript variant 2, mRNA. | gametocyte specific factor 1-like | GTSF1L | 2 8 5 |
| 8001 197 | ENST0000030 3155 /// AY358718 /// NM_018092 | Isoform 1 of Neuropilin and tolloid-like protein 2 gene: ENSG00000171208 /// Homo sapiens clone DNA84912 Neto2 (UNQ1926) mRNA, complete cds. /// Homo sapiens neuropilin (NRP) and tolloid (TLL)-like 2 (NETO2), mRNA. | neuropilin (NRP) and tolloid (TLL)-like 2 | NETO2 | 2 8 6 |
| 8177 195 | ENST0000025 3323 /// ENST0000025 3325 /// AF332238 /// NR_001530 /// NR_002159 | Putative transcript Y 9 protein gene:ENSG00000131007 /// Putative transcript Y 9 protein gene:ENSG00000131009 /// Homo sapiens testis transcript Y 9 (TTY9) mRNA, complete cds. /// Homo sapiens testis-specific transcript, Y-linked 9A (TTTY9A), non-coding RNA. /// Homo sapiens testis-specific transcript, Y-linked 9B (TTTY9B), non-coding RNA. | testis-specific transcript, Y-linked 9A /// testis-specific transcript, Y-linked 9B | TTTY9A /// TTTY9B | 2 8 7 |
| 8008 885 | hsa-mir-21 /// hsa-mir-21 /// A Y699265 | MI0000077 Homo sapiens miR-21 stem-loop /// MI0000077 Homo sapiens miR-21 stem-loop /// Homo sapiens microRNA pri-miR-21, complete sequence. | microRNA 21 | MIR21 | 2 8 8 |

(continued)

| Probe Set ID | Transcript ID | Transcript Description | Gene Title | Gene Symbol | |
|---|---|---|---|---|---|
| 8176 692 | ENST0000025 3323 /// ENST0000025 3325 /// AF332238 /// NR_001530 /// NR_002159 | Putative transcript Y 9 protein gene:ENSG00000131007 /// Putative transcript Y 9 protein gene:ENSG00000131009 /// Homo sapiens testis transcript Y 9 (TTY9) mRNA, complete cds. /// Homo sapiens testis-specific transcript, Y-linked 9A (TTTY9A), non-coding RNA. /// Homo sapiens testis-specific transcript, Y-linked 9B (TTTY9B), non-coding RNA. | testis-specific transcript, Y-linked 9A /// testis-specific transcript, Y-linked 9B | TTTY9A /// TTTY9B | 289 |
| 8056 959 | ENST0000030 8618 /// NM_ 00108045 8 | Homeobox even-skipped homolog protein 2 gene: ENSG00000174279 /// Homo sapiens even-skipped homeobox 2 (EVX2, mRNA. | even-skipped homeobox 2 | EVX2 | 290 |
| 7974 562 | ENST0000036 3948 | ncrna:snRNA chromosome: NCBI36:14:56361 457: 56361563:1 qene: ENSG00000200818 | --- | --- | 291 |
| 8136 557 | ENST0000033 6425 /// ENST0000026 3552 /// BC014117 /// NM_00113096 6 ///NM_001061 /// NM_030984 | thromboxane A synthase 1 isoform TXS-I gene: ENSG00000059377 /// thromboxane A synthase 1 isoform TXS-II gene: ENSG00000059377 /// Homo sapiens thromboxane A synthase 1 (platelet), mRNA (cDNA clone MGC:20885 IMAGE:4548935), complete cds. /// Homo sapiens thromboxane A synthase 1 (platelet) (TBXAS1), transcript variant TXS-III, mRNA. /// Homo sapiens thromboxane A synthase 1 (platelet) (TBXAS1), transcript variant TXS-I, mRNA. /// Homo sapiens thromboxane A synthase 1 (platelet) (TBXAS1), transcript variant TXS-II, mRNA. | thromboxane A synthase 1 (platelet) | TBXAS1 | 292 |

(continued)

| Probe Set ID | Transcript ID | Transcript Description | Gene Title | Gene Symbol | |
|---|---|---|---|---|---|
| 7936 968 | ENST0000036 8679 /// ENST0000036 8683 /// ENST0000036 8676 /// AF023476 /// NM_003474 /// NM_021641 | Isoform 1 of ADAM 12 gene: ENSG00000148848 /// Isoform 4 of ADAM 12 gene: ENSG00000148848 /// Isoform 2 of ADAM 12 gene: ENSG00000148848 /// Homo sapiens meltrin-L precursor (ADAM12) mRNA, complete cds, alternatively spliced. /// Homo sapiens ADAM metallopeptidase domain 12 (ADAM12), transcript variant 1, mRNA. /// Homo sapiens ADAM metallopeptidase domain 12 (ADAM12), transcript variant 2, mRNA. | ADAM metallopeptidase domain 12 | ADAM12 | 2 9 3 |
| 8019 478 | ENST0000031 2648 /// AY935535 /// NM_006137 | T-cell antigen CD7 gene: ENSG00000173762 /// Homo sapiens clone 14 CD7 antigen mRNA, complete cds. Homo sapiens CD7 molecule (CD7), mRNA. | CD7 molecule | CD7 | 2 9 4 |
| 8073 799 | ENST0000025 2934 /// ENST0000039 6011 /// ENST0000038 1061 /// ENST0000040 2380 /// BC007508 /// NM_013236 | Ataxin-10 gene: ENSG00000130638 /// HUMEEP gene:ENSG00000130638 /// Putative uncharacterized protein ATXN10 gene: ENSG00000130638 /// Ataxin 10 gene:ENSG00000130638 /// Homo sapiens ataxin 10, mRNA (cDNA clone MGC:4152 IMAGE: 3030062), complete cds. /// Homo sapiens ataxin 10 (ATXN10), mRNA. | ataxin 10 | ATXN10 | 2 9 5 |
| 8035 789 | ENST0000035 8224 /// BC016785 /// NM_00103988 4 | Zinc finger protein 826 gene: ENSG00000178604 /// Homo sapiens zinc finger protein 826, mRNA (cDNA clone IMAGE: 4096414), complete cds. /// Homo sapiens zinc finger protein 826 (ZNF826), mRNA. | zinc finger protein 826 | ZNF826 | 2 9 6 |
| 7951 485 | ENST0000026 5836 /// ENST0000037 5682 /// AK128062 /// NM_017515 | Isoform 1 of Solute carrier family 35 member F2 gene: ENSG00000110660 /// Putative uncharacterized protein SLC35F2 (Fragment) gene: ENSG00000110660 /// Homo sapiens cDNA FLJ46182 fis, clone TESTI4004539. /// Homo sapiens solute carrier family 35, member F2 (SLC35F2), mRNA. | solute carrier family 35, member F2 | SLC35F2 | 2 9 7 |

(continued)

| Probe Set ID | Transcript ID | Transcript Description | Gene Title | Gene Symbol | |
|---|---|---|---|---|---|
| 8114 805 | ENST0000035 9370 /// ENST0000037 8046 /// ENST0000033 7706 /// ENST0000036 0966 /// ENST0000040 7758 /// ENST0000040 5304 /// ENST0000040 3113/// ENST0000037 8047 /// ENST0000039 4496 /// ENST0000039 4493 /// BC032697 /// NM_033136 /// NM_033137 /// NM_000800 | Heparin-binding growth factor 1 gene:ENSG00000113578 /// Heparin-binding growth factor 1 gene:ENSG00000113578 /// Heparin-binding growth factor 1 gene:ENSG00000113578 /// fibroblast growth factor 1 (acidic) isoform 2 precursor gene: ENSG00000113578 /// Putative uncharacterized protein FGF1 gene:ENSG00000113578 /// fibroblast growth factor 1 (acidic) isoform 2 precursor gene: ENSG00000113578 /// Heparin-binding growth factor 1 gene: ENSG00000113578 /// Heparin-binding growth factor 1 gene: ENSG00000113578 /// Heparin-binding growth factor 1 gene: ENSG00000113578 /// fibroblast growth factor 1 (acidic) isoform 3 precursor gene: ENSG00000113578 /// Homo sapiens fibroblast growth factor 1 (acidic), mRNA (cDNA clone MGC:44867 IMAGE:5403677), complete cds. /// Homo sapiens fibroblast growth factor 1 (acidic) (FGF1), transcript variant 2, mRNA. /// Homo sapiens fibroblast growth factor 1 (acidic) (FGF1), transcript variant 3, mRNA. /// Homo sapiens fibroblast growth factor 1 (acidic) (FGF1), transcript variant 1, mRNA. | fibroblast growth factor 1 (acidic) | FGF1 | 2 9 8 |
| 8095 680 | ENST0000040 1931 /// ENST0000030 7407 /// ENST0000039 5775 /// M17017 /// NM_000584 | 11 kDa protein gene: ENSG00000169429 /// Isoform 1 of Interleukin-8 gene: ENSG00000169429 ///15 kDa protein gene: ENSG00000169429 /// Human beta-thromboglobulin-like protein mRNA, complete cds. /// Homo sapiens interleukin 8 (IL8), mRNA. | interleukin 8 | IL8 | 2 9 9 |
| 8017 827 | ENST0000036 4677 | ncrna:misc_RNA chromosome: NCBI36: 17:62835 352: 62835459:-1 qene: ENSG00000201547 | --- | --- | 3 0 0 |

(continued)

| Probe Set ID | Transcript ID | Transcript Description | Gene Title | Gene Symbol | |
|---|---|---|---|---|---|
| 8083 034 | ENST0000033 2210/// AY753303 /// NM_022131 | Calsyntenin-2 gene: ENSG00000158258 /// Homo sapiens alcadein gamma mRNA, complete cds. /// Homo sapiens calsyntenin 2 (CLSTN2), mRNA. | calsyntenin 2 | CLSTN2 | 3 0 1 |
| 8131 140 | ENST0000031 3156 /// AK024457 | FLJ00049 protein (Fragment) gene:ENSG00000175873 /// Homo sapiens mRNA for FLJ00049 protein, partial cds. | FLJ00049 protein | FLJ00049 | 3 0 2 |
| 7977 003 | ENST0000026 2241 /// AF155595 /// NM_015156 | REST corepressor 1 gene: ENSG00000089902 /// Homo sapiens CoREST protein (COREST) mRNA, complete cds. /// Homo sapiens REST corepressor 1 (RCOR1), mRNA. | REST corepressor 1 | RCOR1 | 3 0 3 |
| 8144 774 | ENST0000032 4815 /// ENST0000032 4849 /// BC067754 /// NM_152415 | Isoform 3 of Vacuolar protein sorting-associated protein 37A gene:ENSG00000155975 /// Isoform 1 of Vacuolar protein sorting-associated protein 37A gene:ENSG00000155975 /// sapiens vacuolar protein sorting 37 homolog A (S. cerevisiae), mRNA (cDNA clone MGC:87029 IMAGE:5275060), complete cds. /// Homo sapiens vacuolar protein sorting 37 homolog A (S. cerevisiae) (VPS37A), mRNA. | vacuolar protein sorting 37 homolog A (S. cerevisiae) | VPS37A | 3 0 4 |
| 8172 266 | hsa-mir-221 /// hsa-mir-221 | MI0000298 Homo sapiens miR-221 stem-loop /// MI0000298 Homo sapiens miR-221 stem-loop | --- | --- | 3 0 5 |
| 8091 186 | --- | --- | --- | --- | 3 0 6 |
| 8076 298 | ENST0000038 6767 | ncrna:scRNA_pseudogene chromosome: NCBI36:22:39776 868:39776953:-1 gene: ENSG00000209502 | --- | --- | 3 0 7 |

(continued)

| Probe Set ID | Transcript ID | Transcript Description | Gene Title | Gene Symbol | |
|---|---|---|---|---|---|
| 8094 704 | ENST0000029 5963 /// ENST0000026 1427 /// U58522 /// NM_ 00111111 2 /// NM_00111111 3 /// NM_005339 | Isoform 2 of Ubiquitin-conjugating enzyme E2 K gene: ENSG00000078140 /// Isoform 1 of Ubiquitin-conjugating enzyme E2 K gene:ENSG00000078140 /// Human huntingtin interacting protein (HIP2) mRNA, complete cds. /// Homo sapiens ubiquitin-conjugating enzyme E2K (UBC1 homolog, yeast) (UBE2K), transcript variant 2, mRNA. /// Homo sapiens ubiquitin-conjugating enzyme E2K (UBC1 homolog, yeast) (UBE2K), transcript variant 3, mRNA. /// Homo sapiens ubiquitin-conjugating enzyme E2K (UBC1 homolog, yeast) (UBE2K), transcript variant 1, mRNA. | ubiquitin-conjugating enzyme E2K (UBC1 homolog, yeast) | UBE2K | 3 0 8 |
| 8089 329 | ENST0000027 3353 /// AK126801 /// NM_014981 | Myosin-15 gene: ENSG00000144821 /// Homo sapiens cDNA FLJ44851 fis, clone BRACE3051819, moderately similar to Myosin heavy chain, cardiac muscle alpha isoform. /// Homo sapiens myosin, heavy chain 15 (MYH15), mRNA. | myosin, heavy chain 15 | MYH15 | 3 0 9 |
| 8116 548 | ENST0000034 4450 /// BC022847 /// NM_020185 | Isoform 1 of Dual specificity protein phosphatase 22 gene: ENSG00000112679 /// Homo sapiens dual specificity phosphatase 22, mRNA (cDNA clone MGC:15090 IMAGE: 3942055), complete cds. /// Homo sapiens dual specificity phosphatase 22 (DUSP22), mRNA. | dual specificity phosphatase 22 | DUSP22 | 3 1 0 |
| 7903 294 | ENST0000037 0152 /// AK057172 /// NM_033055 | Hippocampus abundant transcript 1 protein gene: ENSG00000156875 /// Homo sapiens cDNA FLJ32610 fis, clone STOMA2000055, highly similar to Mouse mRNA for tetracycline transporter-like protein. /// Homo sapiens hippocampus abundant transcript 1 (HIAT1), mRNA. | hippocampus abundant transcript 1 | HIAT1 | 3 1 1 |

(continued)

| Probe Set ID | Transcript ID | Transcript Description | Gene Title | Gene Symbol | |
|---|---|---|---|---|---|
| 7977 567 | ENST0000034 4581 /// BC146951 | similar to hCG2036672 gene: ENSG00000185271 /// Homo sapiens kelch-like 33 (Drosophila), mRNA (cDNA clone MGC:182054 IMAGE:9056879), complete cds. | kelch-like 33 (Drosophila) | KLHL33 | 3 1 2 |
| 7902 685 | ENST0000041 1322 | ncrna:misc_RNA chromosome: NCBI36:1:855025 67:85502673: 1 pene:ENSG00000223254 | --- | --- | 3 1 3 |
| 8137 219 | ENST0000034 3855 /// AL832660 /// NM_138434 | Uncharacterized protein C7orf29 gene:ENSG00000188707 /// Homo sapiens mRNA; cDNA DKFZp313D2012 (from clone DKFZp313D2012). /// Homo sapiens chromosome 7 open reading frame 29 (C7orf29), mRNA. | chromosome 7 open reading frame 29 | C7orf29 | 3 1 4 |
| 7927 153 | ENST0000038 7115 /// ENST0000040 8541 | ncrna:sn RNA_pseudogene chromosome:NCBI36:10:43157 240:43157363:1 gene: ENSG00000209850 /// ncrna: snRNA chromosome:NCBI36:10: 43157 240:43157364:1 gene: ENSG00000221468 | --- | --- | 3 1 5 |
| 7919 572 | ENST0000038 6002 | ncrna:tRNA_pseudogene chromosome:NCBI36:1 :147478 572:147478645:-1 gene: ENSG00000208737 | --- | --- | 3 1 6 |
| 8073 544 | hsa-mir-33a /// hsa-mir-33a | MI0000091 Homo sapiens miR-33a stem-loop /// M10000091 Homo sapiens miR-33a stem-loop | --- | --- | 3 1 7 |
| 8090 091 | ENST0000038 3657 /// BC049369 /// NM_198402 | Protein-tyrosine phosphatase-like member B gene: ENSG00000206527 /// Homo sapiens protein tyrosine phosphatase-like (proline instead of catalytic arginine), member b, mRNA (cDNA clone MGC:57203 IMAGE:5286864), complete cds. /// Homo sapiens protein tyrosine phosphatase-like (proline instead of catalytic arginine), member b (PTPLB), mRNA. | protein tyrosine phosphatase-like (proline instead of catalytic arginine), member b | PTPLB | 3 1 8 |

(continued)

| Probe Set ID | Transcript ID | Transcript Description | Gene Title | Gene Symbol | |
|---|---|---|---|---|---|
| 8045 889 | ENST0000026 3635 /// AK128859 /// NM_033394 | Isoform 1 of Protein TANC1 gene: ENSG00000115183 /// Homo sapiens cDNA FLJ46667 fis, clone TRACH3007689. /// sapiens tetratricopeptide repeat, ankyrin repeat and coiled-coil containing 1 (TANC1), mRNA. | tetratricopeptide repeat, ankyrin repeat and coiled-coil containing 1 | TANC1 | 3 1 9 |
| 7974 689 | ENST0000039 5151 /// ENST0000033 5867 /// ENST0000039 5153 /// AF251079 /// NM 001079520 /// NM_016651 | 60 kDa protein gene: ENSG00000165617 /// Dapper homolog 1 gene: ENSG00000165617 /// dapper 1 isoform 2 gene: ENSG00000165617 /// Homo sapiens heptacellular carcinoma novel pene-3 protein mRNA, complete cds. /// Homo sapiens dapper, antagonist of beta-catenin, homolog 1 (Xenopus laevis) (DACT1), transcript variant 2, mRNA. /// Homo sapiens dapper, antagonist of beta-catenin, homolog 1 (Xenopus laevis) (DACT1), transcript variant 1, mRNA. | dapper, antagonist of beta-catenin, homolog 1 (Xenopus laevis) | DACT1 | 3 2 0 |

(continued)

| Probe Set ID | Transcript ID | Transcript Description | Gene Title | Gene Symbol | |
|---|---|---|---|---|---|
| 8160 040 | ENST0000034 6816 /// ENST0000038 1196 /// ENST0000035 6435 III ENST0000035 8503 III ENST0000036 0074 /// ENST0000039 7617 /// ENST0000039 7611 /// ENST0000035 5233 /// ENST0000039 7606 /// AB211400 /// NM_130393 /// NM_130392 /// NM_130391 /// NM_002839 /// NM_00104071 2 | Isoform 3 of Receptor-type tyrosine-protein phosphatase delta gene:ENSG00000153707 /// Isoform 1 of Receptor-type tyrosine-protein phosphatase delta gene:ENSG00000153707 /// Isoform 1 of Receptor-type tyrosine-protein phosphatase delta gene:ENSG00000153707 /// 214 kDa protein gene: ENSG00000153707 /// Protein tyrosine phosphatase receptor type D gene:ENSG00000153707 /// 215 kDa protein gene: ENSG00000153707 /// 170 kDa protein gene: ENSG00000153707 /// PTPRD protein gene: ENSG00000153707 /// PTPRD protein gene: ENSG00000153707 /// Homo sapiens PTPRD mRNA for protein tyrosine phosphatase receptor type D, complete cds. /// Homo sapiens protein tyrosine phosphatase, receptor type, D (PTPRD), transcript variant 4, mRNA. /// Homo sapiens protein tyrosine phosphatase, receptor type, D (PTPRD), transcript variant 3, mRNA. /// Homo sapiens protein tyrosine phosphatase, receptor type, D (PTPRD), transcript variant 2, mRNA. /// Homo sapiens protein tyrosine phosphatase, receptor type, D (PTPRD), transcript variant 1, mRNA. /// Homo sapiens protein tyrosine phosphatase, receptor type, D (PTPRD), transcript variant 5, mRNA. | protein tyrosine phosphatase, receptor type, D | PTPRD | 3 2 1 |
| 8142 878 | AF503918 | Homo sapiens CDC26 subunit of anaphase promoting complex (CDC26) mRNA, complete cds. | cell division cycle 26 homolog (S. cerevisiae) | CDC26 | 3 2 2 |
| 8065 603 | BC101556 /// NR_002781 | Homo sapiens TSPY-like 3 (pseudogene), mRNA (cDNA clone MGC:126605 IMAGE: 8069062), complete cds. /// Homo sapiens TSPY-like 3 (pseudogene) (TSPYL3), non-coding RNA. | TSPY-like 3 (pseudogene) | TSPYL3 | 3 2 3 |

(continued)

| Probe Set ID | Transcript ID | Transcript Description | Gene Title | Gene Symbol | |
|---|---|---|---|---|---|
| 8104 781 | ENST0000033 0120 /// D88437 /// NM_016568 | Relaxin-3 receptor 1 gene: ENSG00000182631 /// Homo sapiens mRNA for G-protein coupled receptor SALPR, complete cds. /// Homo sapiens relaxin/insulin-like family peptide receptor 3 (RXFP3), mRNA. | relaxin/insulin-like family peptide receptor 3 | RXFP3 | 3 2 4 |
| 7959 012 | ENST0000041 0526 /// ENST0000038 6460 | ncrna:misc_RNA chromosome: NCBI36:12:11463 9906: 114640199:1 gene: ENSG00000222458 /// ncrna: scRNA_pseudogene chromosome:NCBI36:12:11463 9907:114640200:1 gene: ENSG00000209195 | --- | --- | 3 2 5 |
| 7954 692 | ENST0000031 3737 /// AK023286 | Putative uncharacterized protein FLJ13224 gene: ENSG00000177340 /// Homo sapiens cDNA FLJ13224 fis, clone OVARC1000008. | hypothetical protein FLJ13224 | FLJ13224 | 3 2 6 |
| 7951 038 | AK128061 /// NR_002973 | Homo sapiens cDNA FLJ46181 fis, clone TESTI4004210. /// Homo sapiens small nucleolar RNA, H/ACA box 40 (SNORA40), non-coding RNA. | TATA box binding protein (TBP)-associated factor, RNA polymerase I, D, 41kDa /// small nucleolar RNA, H/ACA box 40 | TAF1D/// SNORA40 | 3 2 7 |
| 8169 984 | ENST0000037 0796 /// ENST0000029 8556 /// M31642 /// NM_000194 | Putative uncharacterized protein HPRT1 gene: ENSG00000165704 /// Hypoxanthine-guanine phosphoribosyltransferase gene: ENSG00000165704 /// Homo sapiens hypoxanthine phosphoribosyltransferase 1 (HPRT1) mRNA, complete cds. /// Homo sapiens hypoxanthine phosphoribosyltransferase 1 (HPRT1), mRNA. | hypoxanthine phosphoribosyltransfe rase 1 | HPRT1 | 3 2 8 |
| 7933 204 | ENST0000029 8295 /// AB022718 /// NM_007021 | Protein DEPP gene: ENSG00000165507 /// Homo sapiens mRNA for DEPP (decidual protein induced by progesterone), complete cds. /// Homo sapiens chromosome 10 open reading frame 10 (C10orf10), mRNA. | chromosome 10 open reading frame 10 | C10orf10 | 3 2 9 |

(continued)

| Probe Set ID | Transcript ID | Transcript Description | Gene Title | Gene Symbol | |
|---|---|---|---|---|---|
| 7953 747 | ENST0000036 4910 | ncrna:snRNA chromosome: NCBI36:12:85286 09:8528715:1 qene:ENSG00000201780 | --- | --- | 3 3 0 |
| 8175 256 | uc004exm.1 /// GENSCAN000 00003290 /// BC007360 /// XM_00171578 7 ///XM_00112841 9 /// XM_ 00171587 2 | /// cdna:Genscan chromosome: NCBI36:X:133504 408: 133568038:-1 /// Homo sapiens hypothetical protein MGC16121, mRNA (cDNA clone IMAGE: 3627113), complete cds. /// PREDICTED: Homo sapiens hypothetical protein MGC16121 (MGC16121), mRNA. /// PREDICTED: Homo sapiens hypothetical protein MGC16121 (MGC16121), mRNA. /// PREDICTED: Homo sapiens hypothetical protein MGC16121 (MGC16121), mRNA. | hypothetical protein MGC16121 | MGC1612 1 | 3 3 1 |
| 8180 344 | --- | --- | --- | --- | 3 3 2 |
| 8013 517 | ENST0000038 7217 | ncrna:Mt_tRNA_pseudogene chromosome:NCBI36:17:21942 958:21943026:-1 gene: ENSG00000209952 | --- | --- | 3 3 3 |
| 8155 802 | ENST0000037 6993 /// ENST0000037 6989 /// ENST0000035 8399 /// ENST0000023 8018 /// AF019638 /// NM_004293 | Guanine deaminase gene: ENSG00000119125 /// Guanine deaminase gene: ENSG00000119125 /// Guanine aminohydrolase gene: ENSG00000 119125 /// cDNA FLJ60569, highly similar to Guanine deaminase gene: ENSG00000119125 /// Homo sapiens nedasin s-form mRNA, complete cds. /// Homo sapiens guanine deaminase (GDA), mRNA. | guanine deaminase | GDA | 3 3 4 |
| 8114 468 | NR_002913 | Homo sapiens small nucleolar RNA, C/D box 63 (SNORD63), non-coding RNA. | small nucleolar RNA, C/D box 63 | SNORD63 | 3 3 5 |

(continued)

| Probe Set ID | Transcript ID | Transcript Description | Gene Title | Gene Symbol | |
|---|---|---|---|---|---|
| 8140 398 | ENST0000030 7630 /// BC020963 /// NM_012479 | 14-3-3 protein gamma gene: ENSG00000170027 /// Homo sapiens tyrosine 3-monooxygenase/tryptophan 5-monooxygenase activation protein, gamma polypeptide, mRNA (cDNA clone MGC:8908 IMAGE:3915246), complete cds. //1 Homo sapiens tyrosine 3-monooxygenase/tryptophan 5-monooxygenase activation protein, gamma polypeptide (YWHAG), mRNA. | tyrosine 3-monooxygenase/trypt ophan 5-monooxygenase activation protein, gamma polypeptide | YWHAG | 3 3 6 |
| 7982 000 | NR_003340 | Homo sapiens small nucleolar RNA, C/D box 116-26 (SNORD116-26), non-coding RNA. | small nuclear ribonucleoprotein polypeptide N /// small nucleolar RNA, C/D box 116-26 | SNRPN /// SNORD11 6-26 | 3 3 7 |
| 8141 150 | ENST0000039 4309 /// ENST0000017 5506 /// ENST0000039 4308 /// BC008723 /// NM_133436/// NM_001673 /// NM_183356 | Asparagine synthetase [glutamine-hydrolyzing] gene: ENSG00000070669 /// Asparagine synthetase [glutamine-hydrolyzing] gene: ENSG00000070669 /// Asparagine synthetase [glutamine-hydrolyzing] gene: ENSG00000070669 /// Homo sapiens asparagine synthetase, mRNA (cDNA clone MGC:8639 IMAGE:2961551), complete cds. /// Homo sapiens asparagine synthetase (ASNS), transcript variant 1, mRNA. /// Homo sapiens asparagine synthetase (ASNS), transcript variant 2, mRNA. /// Homo sapiens asparagine synthetase (ASNS), transcript variant 3, mRNA. | asparagine synthetase | ASNS | 3 3 8 |
| 8055 265 | ENST0000038 5636 | ncrna:Mt_tRNA_pseudogene chromosome:NCBI36:2: 131846 208:131846265:-1 gene: ENSG00000208371 | --- | --- | 3 3 9 |
| 8105 607 | ENST0000038 9074 | UPF0514 membrane protein FAM159B qene: ENSG00000145642 | --- | --- | 3 4 0 |
| 8099 130 | ENST0000036 3891 | ncrna:misc_RNA chromosome: NCBI36:4:497338 1:4973692:-1 gene: ENSG00000200761 | --- | --- | 3 4 1 |

(continued)

| Probe Set ID | Transcript ID | Transcript Description | Gene Title | Gene Symbol | |
|---|---|---|---|---|---|
| 8015 456 | ENST0000031 9121 /// BC034470 /// NM_018143 | Kelch-like protein 11 gene: ENSG00000178502 /// Homo sapiens kelch-like 11 (Drosophila), mRNA (cDNA clone MGC:26174 IMAGE:4822768), complete cds. /// Homo sapiens kelch-like 11 (Drosophila) (KLHL11), mRNA. | kelch-like 11 (Drosophila) | KLHL11 | 3 4 2 |
| 8028 332 | ENST0000026 3372 /// AF117708 /// NM_004823 | Isoform 1 of Potassium channel subfamily K member 6 gene: ENSG00000099337 /// Homo sapiens tandem pore domain potassium channel TWIK-2 (KCNK6) mRNA, complete cds. /// Homo sapiens potassium channel, subfamily K, member 6 (KCNK6), mRNA. | potassium channel, subfamily K, member 6 | KCNK6 | 3 4 3 |
| 7947 147 | ENST0000035 4193 /// AL833119 /// NM_148893 | Small VCP/p97-interacting protein gene: ENSG00000198168 /// Homo sapiens mRNA; cDNA DKFZp313A2432 (from clone DKFZp313A2432). /// Homo sapiens small VCP/p97-interacting protein (SVIP), mRNA. | small VCP/p97-interacting protein | SVIP | 3 4 4 |
| 7961 198 | ENST0000002 3165 /// ENST0000034 1141 /// AK296103 /// NM_006611 | killer cell lectin-like receptor subfamily A, member 1 gene: ENSG00000021602 /// KLRA1 gene:ENSG00000021602 /// Homo sapiens cDNA FLJ59270 complete cds, highly similar to Homo sapiens killer cell lectin-like receptor subfamily A, member 1 (KLRA1), mRNA. /// Homo sapiens killer cell lectin-like receptor subfamily A, member 1 (KLRA1), mRNA. | killer cell lectin-like receptor subfamily A, member 1 | KLRA1 | 3 4 5 |
| 7957 008 | ENST0000026 6679 /// ENST0000035 1671 /// BC000714 /// NM_007007 | Isoform 2 of Cleavage and polyadenylation specificity factor subunit 6 gene: ENSG00000111605 /// Isoform 1 of Cleavage and polyadenylation specificity factor subunit 6 gene: ENSG00000111605 /// Homo sapiens cleavage and polyadenylation specific factor 6, 68kDa, mRNA (cDNA clone MGC: 1242 IMAGE:3506481), complete cds. /// Homo sapiens cleavage and polyadenylation specific factor 6, 68kDa (CPSF6), mRNA. | cleavage and polyadenylation specific factor 6, 68kDa | CPSF6 | 3 4 6 |

(continued)

| Probe Set ID | Transcript ID | Transcript Description | Gene Title | Gene Symbol | |
|---|---|---|---|---|---|
| 8046 790 | ENST0000038 4449 | ncrna:snRNA chromosome: NCBI36:2: 183446 814: 183446920:1 gene: ENSG00000207178 | --- | - | 3 4 7 |
| 8049 375 | ENST0000038 9758 /// ENST0000039 6517 /// ENST0000032 7506 | similar to hCG2012694 gene: ENSG00000185038 /// similar to hCG2012694 gene: ENSG00000185038 /// similar to hCG2012694 gene: ENSG00000185038 | --- | --- | 3 4 8 |
| 8122 732 | GENSCAN000 00041083 /// ENST0000030 9074 | cdna:Genscan chromosome: NCBI36:6: 150340 754: 150341242:1 /// cdna: pseudogene chromosome: NCBI36:6: 150340 754: 150341242:1 gene: ENSG00000173909 | --- | --- | 3 4 9 |
| 7907 024 | ENST0000036 7876 /// ENST0000036 7875 /// AB040946 /// NM_017542 | Pogo transposable element with KRAB domain gene: ENSG00000143157 /// Pogo transposable element with KRAB domain gene: ENSG00000143157 /// Homo sapiens mRNA for KIAA1513 protein, partial cds. /// Homo sapiens pogo transposable element with KRAB domain (POGK), mRNA. | pogo transposable element with KRAB domain | POGK | 3 5 0 |

(continued)

| Probe Set ID | Transcript ID | Transcript Description | Gene Title | Gene Symbol | |
|---|---|---|---|---|---|
| 8161 774 | ENST0000037 6870 /// ENST0000036 0774 /// ENST0000036 1255 /// ENST0000037 6872 /// ENST0000037 6871 /// ENST0000037 6864 /// ENST0000031 2449 /// ENST0000035 9047 /// AF350881 /// NM_017662 | Isoform M6-kinase 3 of Transient receptor potential cation channel subfamily M member 6 gene: ENSG00000119121 /// Isoform TRPM6a of Transient receptor potential cation channel subfamily M member 6 gene: ENSG00000119121 /// Putative uncharacterized protein TRPM6 gene:ENSG00000119121 /// Isoform M6-kinase 1 of Transient receptor potential cation channel subfamily M member 6 gene: ENSG00000119121 /// Isoform M6-kinase 2 of Transient receptor potential cation channel subfamily M member 6 gene: ENSG00000119121 /// Isoform TRPM6t of Transient receptor potential cation channel subfamily M member 6 gene: ENSG00000119121 /// Isoform TRPM6t of Transient receptor potential cation channel subfamily M member 6 gene: ENSG00000119121 /// Transient receptor potential cation channel, subfamily M, member 6, isoform CRA_j gene:ENSG00000119121 /// Homo sapiens channel kinase 2 (CHAK2) mRNA, complete cds. /// Homo sapiens transient receptor potential cation channel, subfamily M, member 6 (TRPM6), mRNA. | transient receptor potential cation channel, subfamily M, member 6 | TRPM6 | 3 5 1 |
| 8157 144 | ENST0000032 2940 /// ENST0000037 4621 /// ENST0000037 4624 /// BC015795 /// NM_017832 | UPF0436 protein C9orf6 gene: ENSG00000119328 /// Putative uncharacterized protein C9orf6 (Fragment) gene: ENSG00000119328 /// 13 kDa protein gene: ENSG00000119328 /// Homo sapiens chromosome 9 open reading frame 6, mRNA (cDNA clone MGC:8859 IMAGE: 3910513), complete cds. /// Homo sapiens chromosome 9 open reading frame 6 (C9orf6), mRNA. | chromosome 9 open reading frame 6 | C9orf6 | 3 5 2 |

(continued)

| Probe Set ID | Transcript ID | Transcript Description | Gene Title | Gene Symbol | |
|---|---|---|---|---|---|
| 8018 377 | ENST0000041 1285/// ENST0000038 8598 | ncrna:snRNA chromosome: NCBI36:17:70916 762: 70916860:-1 gene: ENSG00000223217 /// ncrna : snRNA_pseudogene chromosome:NCBI36:17:70916 768:70916864:-1 qene: ENSG00000211333 | --- | --- | 3 5 3 |
| 8146 564 | ENST0000026 2646 /// ENST0000039 6697 /// ENST0000039 6696 /// BC008929 /// NM 002865 | Ras-related protein Rab-2A gene: ENSG00000104388 /// 24 kDa protein gene: ENSG00000104388 /// Putative uncharacterized protein RAB2A gene:ENSG00000104388 /// Homo sapiens RAB2A, member RAS oncogene family, mRNA (cDNA clone MGC:1656 IMAGE: 2966694), complete cds. /// Homo sapiens RAB2A, member RAS oncogene family (RAB2A), mRNA. | RAB2A, member RAS oncogene family | RAB2A | 3 5 4 |
| 7957 298 | ENST0000026 6692 /// ENST0000022 8327 /// ENST0000039 7909 /// ENST0000037 8640 /// BC017667 /// NM_014903 | Isoform 3 of Neuron navigator 3 gene:ENSG00000067798 /// Isoform 1 of Neuron navigator 3 gene:ENSG00000067798 /// Isoform 2 of Neuron navigator 3 gene:ENSG00000067798 /// 253 kDa protein gene: ENSG00000067798 /// Homo sapiens neuron navigator 3, mRNA (cDNA clone IMAGE: 3914378), partial cds. /// Homo sapiens neuron naviqator 3 (NAV3), mRNA. | neuron navigator 3 | NAV3 | 3 5 5 |
| 8091 118 | --- | --- | --- | --- | 3 5 6 |
| 8107 909 | ENST0000020 0652 /// BC028313 /// NM_003059 | Solute carrier family 22 member 4 gene:ENSG00000197208 /// Homo sapiens solute carrier family 22 (organic cation/ ergothioneine transporter), member 4, mRNA (cDNA clone MGC:34546 IMAGE:5186192), complete cds. /// Homo sapiens solute carrier family 22 (organic cation/ergothioneine transporter), member 4 (SLC22A4), mRNA. | solute carrier family 22 (organic cation/ ergothioneine transporter), member 4 | SLC22A4 | 3 5 7 |

(continued)

| Probe Set ID | Transcript ID | Transcript Description | Gene Title | Gene Symbol | |
|---|---|---|---|---|---|
| 8101 723 | ENST0000032 3061 /// BC104883 /// NM-1 53757 | Nucleosome assembly protein 1-like 5 gene:ENSG00000177432 /// Homo sapiens nucleosome assembly protein 1-like 5, mRNA (cDNA clone MGC:132543 IMAGE:8143886), complete cds. /// Homo sapiens nucleosome assembly protein 1-like 5 (NAP1L5), mRNA. | nucleosome assembly protein 1-like 5 | NAP1L5 | 3 5 8 |
| 8053 311 | ENST0000036 3618 | ncrna:misc_RNA chromosome: NCBI36:2:765257 13:76526044:-1 gene: ENSG00000200488 | --- | --- | 3 5 9 |
| 8054 364 | ENST0000039 3359 /// ENST0000025 8449 /// BC020548 /// NM_004257 | Transforming growth factor-beta receptor-associated protein 1 gene:ENSG00000135966 /// Transforming growth factor-beta receptor-associated protein 1 gene:ENSG00000135966 /// Homo sapiens transforming growth factor, beta receptor associated protein 1, mRNA (cDNA clone MGC:21319 IMAGE:4420120), complete cds. /// Homo sapiens transforming growth factor, beta receptor associated protein 1 (TGFBRAP1), mRNA. | transforming growth factor, beta receptor associated protein 1 | TGFBRAP 1 | 3 6 0 |
| 7938 295 | ENST0000031 4138 /// NM_ 000990 | 60S ribosomal protein L27a gene: ENSG00000166441 /// Homo sapiens ribosomal protein L27a (RPL27A), mRNA. | ribosomal protein L27a | RPL27A | 3 6 1 |

(continued)

| Probe Set ID | Transcript ID | Transcript Description | Gene Title | Gene Symbol | |
|---|---|---|---|---|---|
| 7995 267 | ENST0000031 5486 /// ENST0000034 1305 /// ENST0000039 8682 /// ENST0000035 4614 /// ENST0000039 8680 /// ENST0000039 8667 /// ENST0000039 8666 /// ENST0000036 0260 /// ENST0000039 8664 /// ENST0000038 0147 /// ENST0000038 0148 /// AB023508 /// NM_016212 /// NM_00109968 7 | Isoform 1 of TP53-target gene 3 protein gene: ENSG00000180598 /// Isoform 2 of TP53-target gene 3 protein gene:ENSG00000180598 /// Isoform 2 of TP53-target gene 3 protein gene: ENSG00000183632 /// Isoform 3 of TP53-target gene 3 protein gene:ENSG00000183632 /// Isoform 1 of TP53-target gene 3 protein gene: ENSG00000183632 /// Isoform 2 of TP53-target gene 3 protein gene:ENSG00000205457 /// Isoform 1 of TP53-target gene 3 protein gene: ENSG00000205457 /// Isoform 3 of TP53-target gene 3 protein gene:ENSG00000205457 /// Isoform 2 of TP53-target gene 3 protein gene: ENSG00000205456 /// Isoform 1 of TP53-target gene 3 protein gene:ENSG00000205456 /// Isoform 3 of TP53-target gene 3 protein gene: ENSG00000205456 /// Homo sapiens mRNA for TP53TG3b, complete cds. /// Homo sapiens TP53 target 3 (TP53TG3), mRNA. /// Homo sapiens similar to TP53TG3 protein (LOC729355), mRNA. | TP53 target 3 /// similar to TP53TG3 protein | TP53TG3 /// LOC7293 55 | 3 6 2 |
| 8032 465 | ENST0000030 0961 /// BC021201 /// NM_144616 | Junctional sarcoplasmic reticulum protein 1 gene: ENSG00000167476 /// Homo sapiens junctional sarcoplasmic reticulum protein 1, mRNA (cDNA clone MGC:13120 IMAGE: 4106867), complete cds. /// Homo sapiens junctional sarcoplasmic reticulum protein 1 (JSRP1), mRNA. | junctional sarcoplasmic reticulum protein 1 | JSRP1 | 3 6 3 |
| 8167 953 | ENST0000041 1174 /// ENST0000038 8411 | ncrna:misc_RNA chromosome: NCBI36:X:633472 96:63347591: 1 gene:ENSG00000223106 /// ncrna:scRNA_pseudogene chromosome: NCBI36:X:633472 96:63347593: 1 gene: ENSG00000211146 | --- | --- | 3 6 4 |

(continued)

| Probe Set ID | Transcript ID | Transcript Description | Gene Title | Gene Symbol | |
|---|---|---|---|---|---|
| 8102 643 | ENST0000027 4026 /// AK291931 /// NM_001237 | Cyclin-A2 gene: ENSG00000145386 /// Homo sapiens cDNA FLJ77347 complete cds, highly similar to sapiens cyclin A2 (CCNA2), mRNA. /// Homo sapiens cyclin A2 (CCNA2), mRNA. | cyclin A2 | CCNA2 | 3 6 5 |
| 7897 960 | ENST0000033 2530 /// ENST0000035 9318 /// NM_ 00110316 9 /// NM_00110317 0 | arylacetamide deacetylase-like 3 isoform 2 gene: ENSG00000188984/// Arylacetamide deacetylase-like 3 gene:ENSG00000188984 /// Homo sapiens arylacetamide deacetylase-like 3 (AADACL3), transcript variant 2, mRNA. /// Homo sapiens arylacetamide deacetylase-like 3 (AADACL3), transcript variant 1, mRNA. | aryl acetamide deacetylase-like 3 | AADACL3 | 3 6 6 |
| 8055 424 | ENST0000036 3794 | ncrna:misc_RNA chromosome: NCB)36:2:136284 209: 136284319:-1 gene: ENSG00000200664 | --- | --- | 3 6 7 |
| 8097 704 | ENST0000029 6582 /// BC046128 /// NM_018241 | Isoform 1 of Transmembrane protein 184C gene: ENSG00000164168 /// Homo sapiens transmembrane protein 184C, mRNA (cDNA clone MGC: 57601 IMAGE:5750613), complete cds. /// Homo sapiens transmembrane protein 184C (TMEM184C), mRNA. | transmembrane protein 184C | TMEM184 C | 3 6 8 |
| 8106 820 | ENST0000039 9107/// AK295434 /// NM_006467 | DNA-directed RNA polymerase /// subunit G gene: ENSG00000113356 /// Homo sapiens cDNA FLJ60555 complete cds, highly similar to DNA-directed RNA polymerase /// subunit G (EC2.7.7.6). /// Homo sapiens polymerase (RNA) III (DNA directed) polypeptide G (32kD) (POLR3G), mRNA. | polymerase (RNA) /// (DNA directed) polypeptide G (32kD) | POLR3G | 3 6 9 |
| 7992 782 | ENST0000039 6927 /// AK091002 /// AY390431 /// NM_020982 | Claudin-9 gene: ENSG00000213937 /// Homo sapiens cDNA FLJ33683 fis, clone BRAWH2002623, highly similar to CLAUDIN-9. /// Homo sapiens HCTP4-binding protein mRNA, complete cds. /// Homo sapiens claudin 9 (CLDN9), mRNA. | claudin 9 /// HCTP4-binding protein | CLDN9 /// LOC1001 34406 | 3 7 0 |

(continued)

| Probe Set ID | Transcript ID | Transcript Description | Gene Title | Gene Symbol | |
|---|---|---|---|---|---|
| 8089 830 | ENST0000029 5628 /// NM_ 00109967 8 | Leucine-rich repeat-containing protein 58 gene: ENSG00000163428 /// Homo sapiens leucine rich repeat containing 58 (LRRC58), mRNA. | leucine rich repeat containing 58 | LRRC58 | 3 7 1 |
| 7998 629 | GENSCAN000 00001581 | cdna:Genscan chromosome: NCBI36:16:18318 18:1893847:-1 | --- | --- | 3 7 2 |
| 7943 736 | hsa-mir-34b /// hsa-mir-34b | MI0000742 Homo sapiens miR-34b stem-loop /// MI0000742 Homo sapiens miR-34b stem-loop | --- | --- | 3 7 3 |
| 7921 356 | ENST0000039 2265 /// ENST0000031 4902 /// NM_ 00100447 6 | Seven transmembrane helix receptor gene: ENSG00000180708 /// Olfactory receptor 10K2 gene: ENSG00000180708 /// Homo sapiens olfactory receptor, family 10, subfamily K, member 2 (OR10K2), mRNA. | olfactory receptor, family 10, subfamily K, member 2 | OR10K2 | 3 7 4 |
| 8052 413 | GENSCAN000 00048378 /// ENST0000040 4638 | cdna:Genscan chromosome: NCBI36:2:608155 29:60816357:-1 /// cdna:pseudogene chromosome:NCBI36:2:608155 29:60816366:-1 gene: ENSG00000217407 | - | - | 3 7 5 |
| 7961 865 | ENST0000025 6078 /// ENST0000031 1936 /// ENST0000039 5977 /// M54968 /// NM_004985 /// NM_033360 | Isoform 2A of GTPase KRas gene:ENSG00000133703 /// Isoform 2B of GTPase KRas gene:ENSG00000133703 /// Isoform 2A of GTPase KRas gene:ENSG00000133703 /// Homo sapiens K-ras oncogene protein (KRAS) mRNA, complete cds. /// Homo sapiens v-Ki-ras2 Kirsten rat sarcoma viral oncogene homolog (KRAS), transcript variant b, mRNA. /// Homo sapiens v-Ki-ras2 Kirsten rat sarcoma viral oncogene homolog (KRAS), transcript variant a, mRNA. | v-Ki-ras2 Kirsten rat sarcoma viral oncogene homolog | KRAS | 3 7 6 |

(continued)

| Probe Set ID | Transcript ID | Transcript Description | Gene Title | Gene Symbol | |
|---|---|---|---|---|---|
| 8142 019 | ENST0000029 7431 /// AK302122 /// NM_002553 /// NM_181747 | Origin recognition complex subunit 5 gene: ENSG00000164815 /// Homo sapiens cDNA FLJ53851 complete cds, highly similar to Origin recognition complex subunit 5. /// Homo sapiens origin recognition complex, subunit 5-like (yeast) (ORC5L), transcript variant 1, mRNA. /// Homo sapiens origin recognition complex, subunit 5-like (yeast) (ORC5L), transcript variant 2, mRNA. | origin recognition complex, subunit 5-like (yeast) | ORC5L | 3 7 7 |
| 8009 873 | ENST0000032 5720 /// AF418286 /// NR_003587 | cdna:known chromosome: NCBI36: 17:71097 148: 71101765:1 gene: ENSG00000204326 /// Homo sapiens clone 1 myosin XVBP (MY015B) pseudogene, partial sequence. /// Homo sapiens myosin XVB pseudogene (MY015B) on chromosome 17. | myosin XVB pseudogene | MY015B | 3 7 8 |
| 8149 749 | ENST0000031 2584 /// AY358285 /// NM_003840 | Tumor necrosis factor receptor superfamily member 10D gene: ENSG00000173530 /// Homo sapiens clone DNA35663 DcR2-TNFR (UNQ251) mRNA, complete cds. /// Homo sapiens tumor necrosis factor receptor superfamily, member 10d, decoy with truncated death domain (TNFRSF10D), mRNA. | tumor necrosis factor receptor superfamily, member 10d, decoy with truncated death domain | TNFRSF1 0D | 3 7 9 |

(continued)

| Probe Set ID | Transcript ID | Transcript Description | Gene Title | Gene Symbol | |
|---|---|---|---|---|---|
| 7986 665 | ENST0000033 7451 /// ENST0000035 9727 /// ENST0000039 8014 /// ENST0000039 8013 /// BC011775 /// NM_030922 /// NM_00100889 2 /// NM_ 00100889 4 /// NM_00100886 0 | Non-imprinted in Prader-Willi/ Angelman syndrome region protein 2 gene: ENSG00000140157 /// non imprinted in Prader-Willi/ Angelman syndrome 2 isoform b gene:ENSG00000140157 /// Non-imprinted in Prader-Willi/ Angelman syndrome region protein 2 gene: ENSG00000140157 /// Non- imprinted in Prader-Willi/ Angelman syndrome region protein 2 gene: ENSG00000140157 /// Homo sapiens non imprinted in Prader- Willi/Angelman syndrome 2, mRNA (cDNA clone MGC:19609 IMAGE:3640970), complete cds. /// Homo sapiens non imprinted in Prader-Willi/Angelman syndrome 2 (NIPA2), transcript variant 1, mRNA. /// Homo sapiens non imprinted in Prader-Willi/ Angelman syndrome 2 (NIPA2), transcript variant 3, mRNA. /// Homo sapiens non imprinted in Prader-Willi/Angelman syndrome 2 (NIPA2), transcript variant 4, mRNA. /// Homo sapiens non imprinted in Prader-Willi/ Anqelman syndrome 2 (NIPA2), transcript variant 2, mRNA. | non imprinted in Prader-Willi/Angelman syndrome 2 | NIPA2 | 3 8 0 |
| 8163 015 | ENST0000038 6231 /// ENST0000041 1190 | ncrna:scRNA_pseudogene chromosome:NCBI36:9:109613 716:109614016:-1 gene: ENSG00000208966 /// ncrna: misc_RNA chromosome: NCBI36:9:109613 719: 109614016:-1 qene: ENSG00000223122 | --- | --- | 3 8 1 |
| 8007 548 | AF143236 | Homo sapiens apoptosis related protein APR-2 mRNA, complete cds. | chromosome 17 open reading frame 88 | C17orf88 | 3 8 2 |

| Probe Set ID | Transcript ID | Transcript Description | Gene Title | Gene Symbol | |
|---|---|---|---|---|---|
| 7923 659 | ENST0000036 7188 /// BC065280 /// NM_032833 | Protein phosphatase 1 regulatory subunit 15B gene: ENSG00000158615 /// Homo sapiens protein phosphatase 1, regulatory (inhibitor) subunit 15B, mRNA (cDNA clone MGC:74824 IMAGE:6172811), complete cds. /// Homo sapiens protein phosphatase 1, regulatory (inhibitor) subunit 15B (PPP1 R15B), mRNA. | protein phosphatase 1, regulatory (inhibitor) subunit 15B | PPP1 R15 B | 3 8 3 |
| 8001 666 | ENST0000026 2506 /// M55268 /// NM_001896 | Casein kinase II subunit alpha' gene:ENSG00000070770 /// Human casein kinase II alpha' subunit mRNA, complete cds. /// Homo sapiens casein kinase 2, alpha prime polypeptide (CSNK2A2), mRNA. | casein kinase 2, alpha prime polypeptide | CSNK2A2 | 3 8 4 |
| 8155 418 | ENST0000031 6269 /// AK125850 /// AL833349 | hypothetical protein gene: ENSG00000204831 /// Homo sapiens cDNA FLJ43862 fis, clone TESTI4007775. /// Homo sapiens mRNA; cDNA DKFZp686P0734 (from clone DKFZp686P0734). | hypothetical protein LOC100133036 /// family with sequence similarity 95, member B1 | LOC1001 33036 /// FAM95B1 | 3 8 5 |
| 7952 046 | ENST0000027 8937 /// BC017774 /// NM_144765 /// NM_005797 | Myelin protein zero-like protein 2 gene:ENSG00000149573 /// Homo sapiens myelin protein zero-like 2, mRNA (cDNA clone MGC:22243 IMAGE:4692569), complete cds. /// Homo sapiens myelin protein zero-like 2 (MPZL2), transcript variant 2, mRNA. /// Homo sapiens myelin protein zero-like 2 (MPZL2), transcript variant 1, mRNA. | myelin protein zero-like 2 | MPZL2 | 3 8 6 |
| 8141 843 | uc003vad.1 /// XM_00172521 8 ///XM_00171820 0 | /// PREDICTED: Homo sapiens similar to HSPC047 protein (LOC100134722), mRNA. /// PREDICTED: Homo sapiens similar to RAS p21 protein activator 4 (LOC100133005), mRNA. | similar to HSPC047 protein /// similar to RAS p21 protein activator 4 | LOC1001 34722 /// LOC1001 33005 | 3 8 7 |

(continued)

| Probe Set ID | Transcript ID | Transcript Description | Gene Title | Gene Symbol | |
|---|---|---|---|---|---|
| 8023 175 | ENST0000025 6433 /// AK027108 /// NM_016097 | Immediate early response 3-interacting protein 1 gene: ENSG00000134049 /// Homo sapiens cDNA: FLJ23455 fis, clone HSI07063, highly similar to AF125100 Homo sapiens HSPC039 protein mRNA. /// Homo sapiens immediate early response 3 interacting protein 1 (IER3IP1), mRNA. | immediate early response 3 interacting protein 1 | IER31P1 | 388 |
| 8072 153 | ENST0000024 9064 /// BC053874 /// NM_173510 | Isoform 1 of Coiled-coil domain-containing protein 117 gene: ENSG00000159873 /// Homo sapiens coiled-coil domain containing 117, mRNA (cDNA clone MGC:61737 IMAGE: 5531689), complete cds. /// Homo sapiens coiled-coil domain containing 117 (CCDC117), mRNA. | coiled-coil domain containing 117 | CCDC117 | 389 |
| 7928 308 | ENST0000030 7365 /// BC007714 /// NM_019058 | DNA-damage-inducible transcript 4 protein gene: ENSG00000168209 /// Homo sapiens DNA-damage-inducible transcript 4, mRNA (cDNA clone MGC:12610 IMAGE:4302878), complete cds. /// Homo sapiens DNA-damage-inducible transcript 4 (DDIT4), mRNA. | DNA-damage-inducible transcript 4 | DDIT4 | 390 |
| 8108 099 | ENST0000032 2887 /// ENST0000039 8844 /// ENST0000026 5341 /// AK304060 /// NM_021982 | Isoform 2 of Protein transport protein Sec24A gene: ENSG00000113615 /// Isoform 1 of Protein transport protein Sec24A gene: ENSG00000113615 /// cDNA FLJ61651, highly similar to Protein transport protein Sec24A gene:ENSG00000113615 /// Homo sapiens cDNA FLJ61651 complete cds, highly similar to Protein transport protein Sec24A. /// Homo sapiens SEC24 family, member A (S. cerevisiae) (SEC24A), mRNA. | SEC24 family, member A (S. cerevisiae) | SEC24A | 391 |

*References*

1. DeGroot, A., Patch Testing. Test Concentrations and Vehicles for 4350 Chemicals. 3rd edition ed. 2008: acdegroot publishing.
2. Luechtefeld, T., et al., Analysis of publically available skin sensitization data from REACH registrations 2008-2014.

ALTEX, 2016. 33(2): p. 135-48.

3. Boverhof, D.R., et al., Respiratory sensitization and allergy: current research approaches and needs. Toxicol Appl Pharmacol, 2008. 226(1): p. 1-13.

4. Thomas, W.R., et al., Characterization and immunobiology of house dust mite allergens. Int Arch Allergy Immunol, 2002. 129(1): p. 1-18.

5. Baur, X., Enzymes as occupational and environmental respiratory sensitisers. Int Arch Occup Environ Health, 2005. 78(4): p. 279-86.

6. Baur, X., L.T. Budnik, and G. von Kirchbach, Allergic asthma caused by exposure to bacterial alpha-amylase Termamyl(R). Am J Ind Med, 2013. 56(3): p. 378-80.

7. Budnik, L.T., et al., Sensitising effects of genetically modified enzymes used in flavour, fragrance, detergence and pharmaceutical production: cross-sectional study. Occup Environ Med, 2016.

8. http://imparas.eu/, accessed 161117.

9. McClain, S., et al., Allergic sensitization: food- and protein-related factors. Clin Transl Allergy, 2014. 4(1): p. 11.

10. North, C.M., et al., Developing a framework for assessing chemical respiratory sensitization: A workshop report. Regul Toxicol Pharmacol, 2016. 80: p. 295-309.

11. OECD, E.C.s.J.R.C., the United States Environmental Protection Agency, the US Army Engineer Research & Development Center, User's handbook supplement to the guidance document for developing and assessing AOPs. 2014.

12. Roggen, E.L., In vitro approaches for detection of chemical sensitization. Basic Clin Pharmacol Toxicol, 2014. 115(1): p. 32-40.

13. Johansson, H., et al., Genomic allergen rapid detection in-house validation--a proof of concept. Toxicol Sci, 2014.139(2): p. 362-70.

14. Schuurhuis, D.H., et al., Ins and outs of dendritic cells. Int Arch Allergy Immunol, 2006. 140(1): p. 53-72.

15. OECD, The Adverse Outcome Pathway for Skin Sensitisation Initiated by Covalent Binding to Proteins. Part 1: Scientific Evidence. 2012: p. 1-59.

16. Forreryd, A., et al., Prediction of chemical respiratory sensitizers using GARD, a novel in vitro assay based on a genomic biomarker signature. PLoS One, 2015. 10(3): p. e0118808.

17. Johansson, H., et al., A genomic biomarker signature can predict skin sensitizers using a cell-based in vitro alternative to animal tests. BMC Genomics, 2011. 12: p. 399.

18. Johansson, H., et al., The GARD assay for assessment of chemical skin sensitizers. Toxicol In Vitro, 2013. 27(3): p. 1163-9.

19. Piccolo, S.R., et al., A single-sample microarray normalization method to facilitate personalized-medicine workflows. Genomics, 2012. 100(6): p. 337-44.

20. Piccolo, S.R., et al., Multiplatform single-sample estimates of transcriptional activation. Proc Natl Acad Sci USA, 2013. 110(44): p. 17778-83.

21. Benjamini, Y. and Y. Hochberg, Controlling the False Discovery Rate: A Practical and Powerful Approach to Multiple Testing. Journal of the Royal Statistical Society. Series B (Methodological), 1995. 57(1): p. 289-300.

22. Noble, W.S., What is a support vector machine? Nat Biotech, 2006. 24(12): p. 1565-1567.

23. Carlsson, A., et al., Molecular serum portraits in patients with primary breast cancer predict the development of distant metastases. Proc Natl Acad Sci U S A, 2011. 108(34): p. 14252-7.

24. Key Pathway Advisor by Clarivate Analytics (Formerly the IP & Science business of Thomson Reuters). http://ioscience.thomsonreuters.com/product/metacore/. 2016.

25. Cortes, C. and V. Vapnik, Support-Vector Networks. Machine Learning, 1995. 20(3): p. 273-297.

26. Konig, K., et al., Cytokine profiles in nasal fluid of patients with seasonal or persistent allergic rhinitis. Allergy Asthma Clin Immunol, 2015. 11(1): p. 26.

27. King, C., et al., Dust mite proteolytic allergens induce cytokine release from cultured airway epithelium. J Immunol, 1998. 161(7): p. 3645-51.

28. *https://www.serumsourceintl.com/questions answers.html.* 2015 [cited 2016 September 5th, 2016].

29. Gough, L., et al., Proteolytic activity of the house dust mite allergen Der p 1 enhances allergenicity in a mouse inhalation model. Clin Exp Allergy, 2003. 33(8): p. 1159-63.

30. Gough, L., et al., The cysteine protease activity of the major dust mite allergen Der p 1 selectively enhances the immunoglobulin E antibody response. J Exp Med, 1999. 190(12): p. 1897-902.

31. Porter, P., et al., Link between allergic asthma and airway mucosal infection suggested by proteinase-secreting household fungi. Mucosal Immunol, 2009. 2(6): p. 504-17.

32. Corsini, E., et al., Role of oxidative stress in chemical allergens induced skin cells activation. Food Chem Toxicol, 2013. 61: p. 74-81.

33. Takai, T., et al., TSLP expression induced via Toll-like receptor pathways in human keratinocytes. Methods Enzymol, 2014. 535: p. 371-87.

34. Roggen, E.L., Breakdown of the Molecular Processes Driving the Adverse Outcome Pathways (AOPs) of Skin and Respiratory Sensitization Induction in Humans Exposed to Xenobiotics and Proteins, in Molecular Immunotoxicology, E.C.a.H.v. Loveren, Editor. 2014, Wiley-VCH Verlag GmbH & Co. KGaA: Weinheim, Germany.

35. Ather, J.L., et al., Airway epithelial NF-kappaB activation promotes allergic sensitization to an innocuous inhaled antigen. Am J Respir Cell Mol Biol, 2011. 44(5): p. 631-8.

36. Chiou, Y.L. and C.Y. Lin, Der p2 activates airway smooth muscle cells in a TLR2/MyD88-dependent manner to induce an inflammatory response. J Cell Physiol, 2009. 220(2): p. 311-8.

37. Yazdi, A.S., K. Ghoreschi, and M. Rocken, Inflammasome activation in delayed-type hypersensitivity reactions. J Invest Dermatol, 2007. 127(8): p. 1853-5.

38. Kool, M., et al., An unexpected role for uric acid as an inducer of T helper 2 cell immunity to inhaled antigens and inflammatory mediator of allergic asthma. Immunity, 2011. 34(4): p. 527-40.

39. Basketter, D.A., et al., Defining occupational and consumer exposure limits for enzyme protein respiratory allergens under REACH. Toxicology, 2010. 268(3): p. 165-70.

40. Sarlo, K., et al., Proteolytic detergent enzymes enhance the allergic antibody responses of guinea pigs to nonproteolytic detergent enzymes in a mixture: implications for occupational exposure. J Allergy Clin Immunol, 1997. 100(4): p. 480-7.

41. Scheurer, S., M. Toda, and S. Vieths, What makes an allergen? Clin Exp Allergy, 2015. 45(7): p. 1150-61.

42. Schweigert, M.K., D.P. Mackenzie, and K. Sarlo, Occupational asthma and allergy associated with the use of enzymes in the detergent industry--a review of the epidemiology, toxicology and methods of prevention. Clin Exp Allergy, 2000. 30(11): p. 1511-8.

43. Chapman, M.D., S. Wunschmann, and A. Pomes, Proteases as Th2 adjuvants. Curr Allergy Asthma Rep, 2007. 7(5): p. 363-7.

44. Straube, F., et al., Contact allergens and irritants show discrete differences in the activation of human monocyte-derived dendritic cells: consequences for in vitro detection of contact allergens. Arch Toxicol, 2005. 79(1): p. 37-46.

45. Pedersen, L.K., et al., Augmentation of skin response by exposure to a combination of allergens and irritants - a review. Contact Dermatitis, 2004. 50(5): p. 265-73.

46. Grabbe, S., et al., Dissection of antigenic and irritative effects of epicutaneously applied haptens in mice. Evidence that not the antigenic component but nonspecific proinflammatory effects of haptens determine the concentration-dependent elicitation of allergic contact dermatitis. J Clin Invest, 1996. 98(5): p. 1158-64.

47. Martin, S.F., Adaptation in the innate immune system and heterologous innate immunity. Cell Mol Life Sci, 2014. 71(21): p. 4115-30.

48. Piccolo, S.R., et al., A single-sample microarray normalization method to facilitate personalized-medicine workflows. Genomics, 2012. 100(6): p. 337-44.

49. Piccolo, S.R., et al., Multiplatform single-sample estimates of transcriptional activation. Proc Natl Acad Sci USA, 2013. 110(44): p. 17778-83.

## Claims

1. A method for identifying proteins which are allergenic in a mammal comprising or consisting of the steps of:

   (a) providing a population of dendritic cells or a population of dendritic-like cells;
   (b) exposing the cells provided in step (a) to a test protein; and
   (c) measuring in the cells of step (b) the expression of 23 or more biomarkers selected from the group defined in Table A;

   wherein the expression of the 23 or more biomarkers measured in step (c) is indicative of the allergenicity of the test protein of step (b); and
   wherein the 23 or more biomarkers selected from the group defined in Table A include the biomarkers defined by the following Probe Set ID numbers in Table A: 8104107; 7984862; 7914992; 7986229; 7896756; 7929478; 7946021; 7912863; 8143710; 8176806; 7920264; 8107421;7906205;7897991;7925846;7905077;7938951;8110104;8015060;7919572; 7953747; 7897960; 7928308.

2. The method according to any one of the preceding claims wherein step (c) comprises or consists of measuring the expression of 24 or more of the biomarkers listed in Table A, for example, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95,

96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 of the biomarkers listed in Table A.

3. The method according to any one of the preceding claims wherein step (c) comprises or consists of measuring the expression of all of the biomarkers listed in Table A.

4. The method according to any previous claim further comprising:

   d) exposing a separate population of the dendritic cells or dendritic-like cells to one or more negative control agent that is not allergenic in a mammal; and
   e) measuring in the cells of step (d) the expression of the 23 or more biomarkers measured in step (c)

   wherein the test protein is identified as allergenic in the event that the expression of the 23 or more biomarkers measured in step (e) differs from the expression of the 23 or more biomarkers measured in step (c); and/or further comprising:

   f) exposing a separate population of the dendritic cells or dendritic-like cells to one or more positive control agent that is allergenic in a mammal; and
   g) measuring in the cells of step (f) the expression of the 23 or more biomarkers measured in step (c)

   wherein the test protein is identified as allergenic in the event that the expression of the 23 or more biomarkers measured in step (f) corresponds to the expression of the 23 or more biomarkers measured in step (c).

5. The method according to any one of the preceding claims wherein step (c) comprises measuring the expression of a nucleic acid molecule of one or more of the biomarkers; optionally

   wherein the nucleic acid molecule is a cDNA molecule or an mRNA molecule; optionally wherein measuring the expression of one or more of the biomarkers in step (c) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation; optionally
   wherein measuring the expression of one or more of the biomarkers in step (c) is determined using a DNA microarray.

6. The method according to any one of the preceding claims wherein measuring the expression of 23 or more of the biomarkers in step (c) is performed using 23 or more binding moieties, each capable of binding selectively to a nucleic acid molecule encoding one of the biomarkers identified in Table A; optionally

   wherein the binding moieties each comprise or consist of a nucleic acid molecule; optionally
   wherein the binding moieties each comprise or consist of DNA, RNA, PNA, LNA, GNA, TNA or PMO; optionally
   wherein the binding moiety comprises a detectable moiety; optionally wherein the detectable moiety is selected from the group consisting of: a fluorescent moiety; a luminescent moiety; a chemiluminescent moiety; a radioactive moiety (for example, a radioactive atom); or an enzymatic moiety.

7. The method according to any one of Claims 1 to 4

wherein step (c) comprises or consists of measuring the expression of the protein of 23 or more of the biomarkers; optionally wherein measuring the expression of 23 or more of the biomarkers in step (c) is performed using 23 or more binding moieties each capable of binding selectively to one of the biomarkers identified in Table A; optionally

wherein the binding moieties comprise or consist of an antibody or an antigen-binding fragment thereof.

8. The method according to any one of the preceding claims wherein step (c) is performed using an array; optionally wherein the array is a bead-based array; optionally wherein the array is a surface-based array; and/or wherein the array is selected from the group consisting of: macroarray; microarray; nanoarray.

9. The method according to any one of the preceding claims wherein the population of dendritic cells or population of dendritic-like cells comprises or consists of immortal and/or non-naturally occurring cells; and/or

wherein the population of dendritic cells or population of dendritic-like cells is a population of dendritic-like cells; optionally
wherein the dendritic-like cells are myeloid dendritic-like cells; optionally
wherein the myeloid dendritic-like cells are derived from myeloid dendritic cells; optionally
wherein the cells derived from myeloid dendritic cells are myeloid leukaemia-derived cells such as those selected from the group consisting of KG-1, THP-1, U-937, HL-60, Monomac-6, AML-193 and MUTZ-3.

10. The method according to any one of the preceding claims for identifying proteins capable of inducing a type I hypersensitivity response in a mammal; and/or

for identifying proteins capable of inducing respiratory sensitization in a mammal; and/or
for identifying proteins capable of inducing a respiratory hypersensitivity response; and/or
wherein the hypersensitivity response is a humoral hypersensitivity response; and/or
for identifying allergenic food proteins; and/or
wherein the method is indicative of the allergenic potency of the sample to be tested.

11. The method according to any one of the preceding claims wherein the method comprises one or more of the following steps:

(i) cultivating dendritic or dendritic-like cells;
(ii) seeding cells of (i) in one or more well(s), e.g. wells of one or more multi-well assay plates;
(iii) adding to a one or more well(s) of (ii) the protein(s) to be tested;
(iv) adding to one or more separate well(s) of (ii) one or more positive control(s), e.g. Der p 1 and/or Der p 7;
(v) adding to one or more separate well(s) of (ii) one or more negative control(s), e.g. DMSO, and/or leaving one or more separate wells(s) of (ii) unstimulated to obtain a medium control;
(vi) incubating cells in wells of (iii)-(v), preferably for about 24 hours;
(vii) isolating purified total RNA from cells of (vi) and, optionally, convert mRNA into cDNA;
(viii) quantifying expression levels of individual mRNA transcripts from (vii), e.g. using an array, such as an Affymetrix Human Gene 1.0 ST array;
(ix) exporting and normalizing expression data from (viii);
(x) isolating data from (ix) originating from biomarkers of the GARD Protein Allergen Prediction Signature (i.e. the biomarkers of Table A);
(xi) applying a prediction model to data from (x), e.g. a frozen SVM model previously established and trained on historical data, e.g. data obtained in Example 1, to predict the allergenicity of tested protein(s) and negative/positive control(s).

12. An array for use in the method according to any one of Claims 1-11 the array consisting of 23 binding moieties as defined in any one of Claims 6-7

wherein the 23 binding moieties include binding moieties with specificity for each of the biomarkers defined by the following Probe Set ID numbers in Table A: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308; optionally
wherein the array comprises one or more binding moiety for each of the biomarkers defined in any one of the preceding claims.

**13.** Use of 23 or more biomarkers selected from the group defined in Table A or 23 or more binding moieties each with specificity for a biomarker selected from the group defined in Table A for determining the allergenicity of a protein; wherein the 23 or more biomarkers selected from the group defined in Table A include the biomarkers defined by the following Probe Set ID numbers in Table A: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308, or the 23 or more binding moieties each with specificity for a biomarker selected from the group defined in Table A include binding moieties with specificity for each of the biomarkers defined by the following Probe Set ID numbers in Table A: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308.

**14.** An analytical kit for use in a method according any one of Claims 1-11 comprising:

(a) an array according to Claim 12; and
(b) (optionally) one or more control agent.
(c) (optionally) instructions for performing the method as defined in any one of Claims 1-11.

**Patentansprüche**

**1.** Verfahren zum Identifizieren von Proteinen, die in einem Säugetier allergen sind, umfassend oder bestehend aus den folgenden Schritten:

(a) Bereitstellen einer Population von dendritischen Zellen oder einer Population von dendritisch-ähnlichen Zellen;
(b) Aussetzen der in Schritt (a) bereitgestellten Zellen einem Testprotein; und
(c) Messen in den Zellen von Schritt (b) der Expression von 23 oder mehr Biomarkern, die aus der Gruppe ausgewählt sind, die in Tabelle A definiert ist;

wobei die Expression der 23 oder mehr Biomarker, die in Schritt (c) gemessen werden, die Allergenität des Testproteins von Schritt (b) anzeigt; und
wobei die 23 oder mehr Biomarker, die aus der Gruppe ausgewählt sind, die in Tabelle A definiert ist, die Biomarker beinhalten, die durch die folgenden Sondensatz-ID-Nummern in Tabelle A definiert sind: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308.

**2.** Verfahren nach einem der vorhergehenden Ansprüche, wobei Schritt (c) das Messen der Expression von 24 oder mehr der Biomarker umfasst oder daraus besteht, die in Tabelle A aufgeführt sind, zum Beispiel 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, oder 391 der Biomarker, die in Tabelle A aufgeführt sind.

**3.** Verfahren nach einem der vorhergehenden Ansprüche, wobei Schritt (c) das Messen der Expression von allen der Biomarker umfasst oder daraus besteht, die in Tabelle A aufgeführt sind.

4. Verfahren nach einem der vorhergehenden Ansprüche, ferner Folgendes umfassend:

(d) Aussetzen einer separaten Population der dendritischen Zellen oder der dendritisch-ähnlichen Zellen einem oder mehreren Negativkontrollmitteln, die bei einem Säugetier nicht allergen sind; und

(e) Messen in den Zellen von Schritt (d) der Expression der 23 oder mehr Biomarker, die in Schritt (c) gemessen werden,

wobei das Testprotein als allergen identifiziert wird, falls sich die Expression der 23 oder mehr Biomarker, die in Schritt (e) gemessen werden, von der Expression der 23 oder mehr Biomarker unterscheidet, die in Schritt (c) gemessen werden; und/oder

ferner Folgendes umfassend:

(f) Aussetzen einer separaten Population der dendritischen Zellen oder der dendritisch-ähnlichen Zellen einem oder mehreren Positivkontrollmitteln, die bei einem Säugetier allergen sind; und

(g) Messen in den Zellen von Schritt (f) der Expression der 23 oder mehr Biomarker, die in Schritt (c) gemessen werden,

wobei das Testprotein als allergen identifiziert wird, falls die Expression der 23 oder mehr Biomarker, die in Schritt (f) gemessen werden, der Expression der 23 oder mehr Biomarker entspricht, die in Schritt (c) gemessen werden.

5. Verfahren nach einem der vorhergehenden Ansprüche, wobei Schritt (c) das Messen der Expression eines Nucleinsäuremoleküls von einem oder mehreren der Biomarker umfasst; optional

wobei das Nucleinsäuremolekül ein cDNA-Molekül oder ein mRNA-Molekül ist; optional wobei das Messen der Expression von einem oder mehreren der Biomarker in Schritt (c) unter Verwendung eines Verfahrens durchgeführt wird, das aus der Gruppe ausgewählt ist, die aus Folgendem besteht: Southern-Hybridisierung, Northern-Hybridisierung, Polymerasekettenreaktion (polymerase chain reaction - PCR), Reverse-Transkriptase-PCR (RT-PCR), quantitative Echtzeit-PCR (quantitative realtime PCR - qRT-PCR), Nanoarray, Microarray, Macroarray, Autoradiographie und *Insitu*-Hybridisierung; optional

wobei das Messen der Expression von einem oder mehreren der Biomarker in Schritt (c) unter Verwendung eines DNA-Microarrays bestimmt wird.

6. Verfahren nach einem der vorhergehenden Ansprüche, wobei das Messen der Expression von 23 oder mehr der Biomarker in Schritt (c) unter Verwendung von 23 oder mehr Bindungsmolekülteilen durchgeführt wird, die jeweils in der Lage sind, an ein Nucleinsäuremolekül wahlweise zu binden, das für einen der Biomarker codiert, die in Tabelle A identifiziert werden; optional

wobei die Bindungsmolekülteile jeweils ein Nucleinsäuremolekül umfassen oder daraus bestehen; optional wobei die Bindungsmolekülteile jeweils DNA, RNA, PNA, LNA, GNA, TNA oder PMO umfassen oder daraus bestehen; optional

wobei der Bindungsmolekülteil einen nachweisbaren Molekülteil umfasst; optional

wobei der nachweisbare Molekülteil aus der Gruppe ausgewählt ist, die aus Folgendem besteht: einem fluoreszierenden Molekülteil; einem lumineszierenden Molekülteil; einem chemilumineszenten Molekülteil; einem radioaktiven Molekülteil (zum Beispiel einem radioaktiven Atom); oder einem enzymatischen Molekülteil.

7. Verfahren nach einem der Ansprüche 1 bis 4, wobei Schritt (c) das Messen der Expression des Proteins von 23 oder mehr der Biomarker umfasst oder daraus besteht; optional

wobei das Messen der Expression von 23 oder mehr der Biomarker in Schritt (c) unter Verwendung von 23 oder mehr Bindungsmolekülteilen durchgeführt wird, die jeweils in der Lage sind, an einen der Biomarker wahlweise zu binden, die in Tabelle A identifiziert werden; optional

wobei die Bindungsmolekülteile einen Antikörper oder ein antigenbindendes Fragment davon umfassen oder daraus bestehen.

8. Verfahren nach einem der vorhergehenden Ansprüche, wobei Schritt (c) unter Verwendung eines Arrays durchgeführt wird; optional

wobei das Array ein perlenbasiertes Array ist; optional wobei das Array ein oberflächenbasiertes Array ist; und/oder wobei das Array aus der Gruppe ausgewählt ist, die aus Folgendem besteht: Macroarray; Microarray; Nanoarray.

9.  Verfahren nach einem der vorhergehenden Ansprüche, wobei die Population von dendritischen Zellen oder die Population von dendritisch-ähnlichen Zellen unsterbliche und/oder nicht natürlich vorkommende Zellen umfasst oder daraus besteht; und/oder

    wobei die Population von dendritischen Zellen oder die Population von dendritisch-ähnlichen Zellen eine Population von dendritisch-ähnlichen Zellen ist; optional
    wobei die dendritisch-ähnlichen Zellen myeloische dendritisch-ähnliche Zellen sind; optional
    wobei die myeloischen dendritisch-ähnlichen Zellen von myeloischen dendritischen Zellen abgeleitet sind; optional wobei die Zellen, die von myeloischen dendritischen Zellen abgeleitet sind, von myeloischer Leukämie abgeleitete Zellen sind, wie etwa diese, die aus der Gruppe ausgewählt sind, die aus KG-1, THP-1, U-937, HL-60, Monomac-6, AML-193 und MUTZ-3 besteht.

10. Verfahren nach einem der vorhergehenden Ansprüche zum Identifizieren von Proteinen, die in der Lage sind, eine Typ-I-Hypersensibilitätsreaktion bei einem Säugetier auszulösen; und/oder

    zum Identifizieren von Proteinen, die in der Lage sind, eine Sensibilisierung der Atemwege bei einem Säugetier auszulösen; und/oder
    zum Identifizieren von Proteinen, die in der Lage sind, eine Hypersensibilitätsreaktion der Atemwege auszulösen; und/oder
    wobei die Hypersensibilitätsreaktion eine humorale Hypersensibilitätsreaktion ist; und/oder
    zum Identifizieren allergener Nahrungsproteine; und/oder
    wobei das Verfahren die allergene Potenz der Probe anzeigt, die getestet werden soll.

11. Verfahren nach einem der vorhergehenden Ansprüche, wobei das Verfahren einen oder mehrere der folgenden Schritte umfasst:

    (i) Kultivieren von dendritischen oder dendritisch-ähnlichen Zellen;
    (ii) Impfen von Zellen von (i) in ein oder mehrere Wells, z. B. Wells einer oder mehrerer Multi-Well-Assay-Platten;
    (iii) Hinzufügen des Proteins, das getestet werden soll, zu einem oder mehreren Wells von (ii);
    (iv) Hinzufügen von einer oder mehreren Positivkontrollen, z. B. Der p 1 und/oder Der p 7, zu einem oder mehreren separaten Wells von (ii);
    (v) Hinzufügen von einer oder mehreren Negativkontrollen, z. B. DMSO, zu einem oder mehreren separaten Wells von (ii), und/oder Belassen eines oder mehrerer separater Wells von (ii) unstimuliert, um eine Mediumkontrolle zu erhalten;
    (vi) Inkubieren von Zellen in Wells von (iii)-(v), vorzugsweise für etwa 24 Stunden;
    (vii) Isolieren von gereinigter Gesamt-RNA aus Zellen von (vi) und, optional, Umsetzen von mRNA in cDNA;
    (viii) Quantifizieren der Expressionsniveaus von einzelnen mRNA-Transkripten aus (vii), z. B. unter Verwendung eines Arrays, wie etwa eines Affymetrix-Human-Gene-1.0-ST-Arrays;
    (ix) Exportieren und Normalisieren von Expressionsdaten aus (viii);
    (x) Isolieren von Daten aus (ix), die von Biomarkern der GARD Protein Allergen Prediction Signature (d. h. den Biomarkern von Tabelle A) stammen;
    (xi) Anwenden eines Vorhersagemodells auf Daten aus (x), z. B. eines eingefrorenen SVM-Modells, das zuvor eingerichtet und an historischen Daten trainiert wird, z. B. Daten, die in Beispiel 1 erhalten werden, um die Allergenität von getestetem(n) Protein(en) und Negativ-/Positivkontrolle(n) vorherzusagen.

12. Array zur Verwendung in dem Verfahren nach einem der Ansprüche 1-11, wobei das Array aus 23 Bindungsmolekülteilen nach einem der Ansprüche 6-7 besteht, wobei die 23 Bindungsmolekülteile Bindungsmolekülteile mit einer Spezifität für jeden der Biomarker beinhalten, die durch die folgenden Sondensatz-ID-Nummern in Tabelle A definiert sind: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308; optional
    wobei das Array ein oder mehrere Bindungsmolekülteile für jeden der Biomarker umfasst, die in einem der vorhergehenden Ansprüche definiert sind.

13. Verwendung von 23 oder mehr Biomarkern, die aus der Gruppe ausgewählt sind, die in Tabelle A definiert ist, oder 23 oder mehr Bindungsmolekülteile, jeweils mit der Spezifität für einen Biomarker, der aus der Gruppe ausgewählt ist, die in Tabelle A zum Bestimmen der Allergenität eines Proteins definiert ist; wobei die 23 oder mehr Biomarker, die aus der Gruppe ausgewählt sind, die in Tabelle A definiert ist, die Biomarker beinhalten, die durch die folgenden Sondensatz-ID-Nummern in Tabelle A definiert sind: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308, oder die 23 oder mehr Bindungsmolekülteile, jeweils mit der Spezifität für einen Biomarker, der aus der Gruppe ausgewählt ist, die in Tabelle A definiert ist, Bindungsmolekülteile mit der Spezifität für jeden der Biomarker beinhalten, die durch die folgenden Sondensatz-ID-Nummern in Tabelle A definiert sind: 8104107; 7984862; 7914992; 7986229; 7896756; 7929478; 7946021; 7912863; 8143710; 8176806; 7920264; 8107421; 7906205; 7897991; 7925846; 7905077; 7938951; 8110104; 8015060; 7919572; 7953747; 7897960; 7928308.

14. Analytisches Kit zur Verwendung in einem Verfahren nach einem der Ansprüche 1-11, Folgendes umfassend:

(a) ein Array nach Anspruch 12; und
(b) (optional) ein oder mehrere Kontrollmittel.
(c) (optional) Anweisungen zum Durchführen des Verfahrens nach einem der Ansprüche 1-11.

## Revendications

1. Procédé destiné à identifier des protéines qui sont allergènes chez un mammifère comprenant ou consistant en les étapes suivantes :

(a) la fourniture d'une population de cellules dendritiques ou d'une population de cellules de type dendritique ;
(b) l'exposition des cellules fournies à l'étape (a) à un agent de test ; et
(c) la mesure, dans les cellules de l'étape (b), de l'expression de 23 biomarqueurs ou plus choisis dans le groupe défini dans le tableau A ;

l'expression des 23 biomarqueurs ou plus mesurés à l'étape (c) indiquant l'allergénicité de la protéine test de l'étape (b) ; et
les 23 biomarqueurs ou plus choisis dans le groupe défini dans le tableau A comportant les biomarqueurs définis par les numéros d'identification d'ensemble de sondes suivants dans le tableau A : 8104107 ; 7984862 ; 7914992 ; 7986229 ; 7896756 ; 7929478 ; 7946021 ; 7912863 ; 8143710 ; 8176806 ; 7920264 ; 8107421 ; 7906205 ; 7897991 ; 7925846 ; 7905077 ; 7938951 ; 8110104 ; 8015060 ; 7919572 ; 7953747 ; 7897960 ; 7928308.

2. Procédé selon l'une quelconque des revendications précédentes, dans lequel l'étape (c) comprend ou est constituée de la mesure de l'expression de 24 biomarqueurs ou plus listés dans le tableau A, par exemple, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, ou 391 des biomarqueurs listés dans le tableau A.

3. Procédé selon l'une quelconque des revendications précédentes, dans lequel l'étape (c) comprend ou consiste en la mesure de l'expression dans l'échantillon de tous les biomarqueurs listés dans le tableau A.

4. Procédé selon l'une quelconque des revendications précédentes, comprenant en outre :

(d) l'exposition d'une population distincte des cellules dendritiques ou des cellules de type dendritique à un ou plusieurs agents de régulation négative qui ne sont pas allergènes chez un mammifère ; et

(e) la mesure, dans les cellules de l'étape (d), de l'expression des 23 biomarqueurs ou plus mesurés à l'étape (c)

la protéine test étant identifiée comme allergène dans le cas où l'expression des 23 biomarqueurs ou plus mesurés à l'étape (e) diffère de l'expression des 23 biomarqueurs ou plus mesurés à l'étape (c) ; et/ou comprenant en outre :

(f) l'exposition d'une population distincte de cellules dendritiques ou de cellules de type dendritique à un ou plusieurs agents témoins positifs qui sont allergènes chez un mammifère ; et

(g) la mesure, dans les cellules de l'étape (f), de l'expression des 23 biomarqueurs ou plus mesurés à l'étape (c)

la protéine test étant identifiée comme allergène dans le cas où l'expression des 23 biomarqueurs ou plus mesurés à l'étape (f) correspond à l'expression des 23 biomarqueurs ou plus mesurés à l'étape (c).

5. Procédé selon l'une quelconque des revendications précédentes, dans lequel l'étape (c) comprend la mesure de l'expression d'une molécule d'acide nucléique d'un ou des biomarqueurs ; éventuellement

la molécule acide nucléique étant une molécule ADNc ou une molécule ARNm ; éventuellement la mesure de l'expression du ou des biomarqueurs à l'étape (c) étant effectuée à l'aide d'un procédé choisi dans le groupe constitué d'hybridation Southern, d'hybridation Northern, d'amplification en chaîne de la polymérase (PCR), de méthode de la transcription inverse suivie de PCR (RT-PCR), de PCR quantitative en temps réel (qRT-PCR), de nanoréseau, de microréseau, de macroréseau, d'autoradiographie et d'hybridation *in situ* ; éventuellement la mesure de l'expression du ouu des biomarqueurs à l'étape (c) étant déterminée à l'aide d'un microréseau d'ADN.

6. Procédé selon l'une quelconque des revendications précédentes, dans lequel la mesure de l'expression des 23 biomarqueurs ou plus à l'étape (c) est effectuée à l'aide des 23 fragments de liaison ou plus, chacun capable de se lier sélectivement à une molécule d'acide nucléique codant un des biomarqueurs identifiés dans le tableau A ; éventuellement

les fragments de liaison, chacun comprenant ou consistant en une molécule d'acide nucléique ; éventuellement les fragments de liaison, chacun comprenant ou consistant en ADN, ARN, PNA, LNA, GNA, TNA ou PMO ; éventuellement le fragment de liaison comprenant un fragment détectable ; éventuellement le fragment détectable étant choisi dans le groupe consistant en : un fragment fluorescent ; un fragment luminescent ; un fragment chimioluminescent ; un fragment radioactif (par exemple, un atome radioactif) ; ou un fragment enzymatique.

7. Procédé selon l'une quelconque des revendications 1 à 4, dans lequel l'étape (b) comprend ou consiste en la mesure de l'expression de la protéine de 23 des biomarqueurs ou plus ; éventuellement la mesure de l'expression de 23 des biomarqueurs ou plus à l'étape (c) étant effectuée à l'aide de 23 ou plus fragments de liaison, chacun capable de se lier sélectivement à l'un des biomarqueurs identifiés dans le tableau A ; éventuellement les fragments de liaison comprenant ou consistant en un anticorps ou un de ses fragments de liaison à l'antigène.

8. Procédé selon l'une quelconque des revendications précédentes, dans lequel l'étape (c) est effectuée à l'aide d'un réseau ; éventuellement le réseau étant un réseau à base de billes ; éventuellement le réseau étant un réseau basé sur la surface ; et/ou le réseau étant choisi dans le groupe consistant en : un macroréseau ; un microréseau ; un nanoréseau.

9. Procédé selon l'une quelconque des revendications précédentes, dans lequel la population de cellules dendritiques ou la population de cellules de type dendritique comprend ou consiste en cellules immortelles et/ou non naturelles ; et/ou la population de cellules dendritiques ou la population de cellules de type dendritique étant une population de cellules de type dendritique ; éventuellement les cellules de type dendritique étant des cellules de type dendritique myéloïde ; éventuellement les cellules de type dendritique myéloïde étant dérivées à partir de cellules dendritiques myéloïdes ; éventuellement les cellules dérivées à partir de cellules dendritiques myéloïdes étant des cellules dérivées de la leucémie myéloïde telles que celles choisies dans le groupe consistant en KG-1, THP-1, U-937, HL-60, Monomac-6, AML-193 et MUTZ-3.

10. Procédé selon l'une quelconque des revendications précédentes destiné à identifier des protéines capables d'induire une réponse d'hypersensibilité de type I chez un mammifère ; et/ou

à identifier des protéines capables d'induire une sensibilisation respiratoire chez un mammifère ; et/ou
à identifier des protéines capables d'induire une sensibilisation respiratoire chez un mammifère ; et/ou
la réponse d'hypersensibilité étant une réponse d'hypersensibilité humorale ; et/ou à identifier les protéines alimentaires allergènes ; et/ou
la méthode indiquant de la puissance allergénique de l'échantillon à tester.

11. Procédé selon l'une quelconque des revendications précédentes, dans lequel le procédé comprend les étapes suivantes :

(i) la culture des cellules dendritiques ou de type dendritique ; et/ou
(ii) l'ensemencement des cellules de (i) dans un ou plusieurs puits, par exemple, des puits d'une ou plusieurs plaques d'essai à puits multiples ; et/ou
(iii) l'ajout à un ou plusieurs puits de (ii) la ou les protéines à tester ; et/ou
(iv) l'ajout à un ou plusieurs puits séparés de (ii) un ou plusieurs témoins positifs, par exemple, Der p 1 et/ou Der p 7 ; et/ou
(v) l'ajout à un ou plusieurs puits séparés de (ii) un ou plusieurs témoins négatifs, par exemple, du DMSO, et/ou le fait de laisser un ou plusieurs puits séparés de (ii) non stimulés afin d'obtenir un témoin moyen ; et/ou
(vi) l'incubation des cellules dans les puits de (iii)-(v), de préférence pendant environ 24 heures ; et/ou
(vii) l'isolement de l'ARN total purifié des cellules de (vi) et, éventuellement, la conversion de l'ARNm en ADNc ; et/ou
(viii) la quantification de niveaux d'expression des transcrits d'ARNm individuels de (vii), par exemple, à l'aide d'un réseau, tel qu'un réseau Affymetrix Human Gene 1.0 ST ; et/ou
(ix) l'export et la normalisation des données d'expression de (viii) ;
(x) l'isolement des données de (ix) provenant de biomarqueurs de la signature de prédiction d'allergènes protéiques de GARD (c'est-à-dire les biomarqueurs du tableau A) ; et/ou
(xi) l'application d'un modèle de prédiction aux données de (x), par exemple, un modèle SVM congelé précédemment établi et formé sur des données historiques, par exemple, les données obtenues dans l'exemple 1, pour prédire l'allergénicité de la ou des protéines testées et des témoins négatifs/positifs.

12. Réseau destiné à être utilisé dans le procédé selon l'une quelconque des revendications 1 à 11, le réseau consistant en 23 fragments de liaison tels que définis dans l'une quelconque des revendications 6 à 7, dans lequel les 23 fragments de liaison comportent des fragments de liaison avec une spécificité pour chacun des biomarqueurs définis par les numéros d'identification d'ensemble de sondes suivants dans le tableau A : 8104107 ; 7984862 ; 7914992 ; 7986229 ; 7896756 ; 7929478 ; 7946021 ; 7912863 ; 8143710 ; 8176806 ; 7920264 ; 8107421 ; 7906205 ; 7897991 ; 7925846 ; 7905077 ; 7938951 ; 8110104 ; 8015060 ; 7919572 ; 7953747 ; 7897960 ; 7928308 ; éventuellement
le réseau comprenant un ou plusieurs fragments de liaison pour chacun des biomarqueurs définis dans l'une quelconque des revendications précédentes.

13. Utilisation de 23 biomarqueurs ou plus choisis dans le groupe défini dans le tableau A ou de 23 fragments de liaison ou plus, chacun avec une spécificité pour un biomarqueur choisi dans le groupe défini dans le tableau A pour déterminer l'allergénicité d'une protéine ; les 23 biomarqueurs ou plus choisis dans le groupe défini dans le tableau A comportant les biomarqueurs définis par les numéros d'identification d'ensemble de sondes suivants dans le tableau A : 8104107 ; 7984862 ; 7914992 ; 7986229 ; 7896756 ; 7929478 ; 7946021 ; 7912863 ; 8143710 ; 8176806 ; 7920264 ; 8107421 ; 7906205 ; 7897991 ; 7925846 ; 7905077 ; 7938951 ; 8110104 ; 8015060 ; 7919572 ; 7953747 ; 7897960 ; 7928308, ou les 23 fragments de liaison ou plus ayant chacun une spécificité pour un biomarqueur choisi dans le groupe défini dans le tableau A comportant des fragments de liaison avec une spécificité pour chacun des biomarqueurs définis par les numéros d'identification d'ensemble de sondes suivants dans le tableau A : 8104107 ; 7984862 ; 7914992 ; 7986229 ; 7896756 ; 7929478 ; 7946021 ; 7912863 ; 8143710 ; 8176806 ; 7920264 ; 8107421 ; 7906205 ; 7897991 ; 7925846 ; 7905077 ; 7938951 ; 8110104 ; 8015060 ; 7919572 ; 7953747 ; 7897960 ; 7928308.

14. Kit analytique destiné à être utilisé dans un procédé selon l'une quelconque des revendications 1 à 11, comprenant :

(a) un réseau selon la revendication 12 ; et
(b) (éventuellement) un ou plusieurs agents de régulation.

(c) (éventuellement) des instructions destinées à effectuer le procédé tel que défini dans l'une quelconque des revendications 1 à 11.

**Figure 1**

**Figure 2**

**Figure 3**

## Figure 4

### Immune response

MIF-induced cell adhesion, migration and angiogenesis
IL-6 signaling pathway via JAK/STAT
IL-6 signaling pathway via MEK/ERK and PI3K/AKT cascades
IL-5 signaling via JAK/STAT
IL-3 signaling via JAK/STAT, p38, JNK and NF-kB
Oncostatin M signaling via MAPK in mouse cells
Oncostatin M signaling via MAPK in human cells
Gastrin in inflammatory response
IL-17 signaling pathways
IL-18 signaling
IL-15 signaling

### Development

HGF signaling pathway
Role of IL-8 in angiogenesis
GM-CSF signaling
Leptin signaling via JAK/STAT and MAPK cascades
HGF-dependent inhibition of TGF-beta-induced EMT
Mu-type opioid receptor regulation of proliferation
Angiotensin signaling via STATs

### Others

Ligand-independent activation of Androgen receptor in Prostate Cancer
Androgen receptor activation and downstream signaling in Prostate cancer
Main pathways of Schwann cells transformation in neurofibromatosis type 1
SLE genetic marker-specific pathways in T cells
Colorectal cancer (general schema)
IGF family signaling in colorectal cancer
Role of Endothelin-1 in inflammation and vasoconstriction in Sickle cell disease
G-protein signaling_Ras family GTPases in kinase cascades (schema)
Neurogenesis_NGF/TrkA MAPK-mediated signaling

0    1    2    3    4    5    6

Significance (-logP value)

## Figure 5

**Figure 6**

**Figure 6 continued**

**Figure 7**

**Figure 8**

**Figure 8 continued**

## Figure 9

relative viability

## Figure 10

GM-CSF

## Figure 11

MIP-1a

MCP-1

IL-8

# Figure 11 continued

Figure 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 39578 A
- US 5856090 A
- WO 9837186 A
- US 4376110 A, David
- US 4486530 A

**Non-patent literature cited in the description**

- **SCHERVISH, MARK J.** A Review of Multivariate Analysis. *Statistical Science,* November 1987, vol. 2 (4), 396-413
- **BURGES.** *Data Mining and Knowledge Discovery,* 1998, vol. 2, 121-167
- **FRAKER et al.** *Biochem. Biophys. Res. Comm.,* 1978, vol. 80, 49-57
- **J-F CHATAL.** Monoclonal Antibodies in Immunoscintigraphy. CRC Press, 1989
- **WINTER ; MILSTEIN.** *Nature,* 1991, vol. 349, 293-299
- **COLLETT.** *Methods,* 2005, vol. 37, 4-15
- **CLACKSON et al.** *Nature,* 1991, vol. 352, 624-628
- **MARKS et al.** *J Mol Biol,* 1991, vol. 222 (3), 581-97
- **SMITH.** *Science,* 1985, vol. 228 (4705), 1315-7
- **SANTI et al.** *J Mol Biol,* 2000, vol. 296 (2), 497-508
- **GUNNERIUSSON et al.** *Appl Environ Microbiol,* 1999, vol. 65 (9), 4134-40
- **KENAN et al.** *Methods Mol Biol,* 1999, vol. 118, 217-31
- **KIEKE et al.** *Proc Natl Acad Sci USA,* 1999, vol. 96 (10), 5651-6
- **HANES ; PLUCKTHUN.** *Proc Natl Acad Sci USA,* 1997, vol. 94 (10), 4937-42
- **HE ; TAUSSIG.** *Nucleic Acids Res,* 1997, vol. 25 (24), 5132-4
- **NEMOTO et al.** *FEBS Lett,* 1997, vol. 414 (2), 405-8
- **DAUGHERTY et al.** *Protein Eng,* 1998, vol. 11 (9), 825-32
- **DAUGHERTY et al.** *Protein Eng,* 1999, vol. 12 (7), 613-21
- **SHUSTA et al.** *J Mol Biol,* 1999, vol. 292 (5), 949-56
- **MORRISON et al.** *Proc. Natl. Acad. Sci. USA,* 1984, vol. 81, 6851-6855
- **BETTER et al.** *Science,* 1988, vol. 240, 1041
- **SKERRA et al.** *Science,* 1988, vol. 240, 1038
- **BIRD et al.** *Science,* 1988, vol. 242, 423
- **HUSTON et al.** *Proc. Natl. Acad. Sci. USA,* 1988, vol. 85, 5879
- **WARD.** *Nature,* 1989, vol. 341, 544
- **H ZOLA.** Monoclonal Antibodies: A manual of techniques. CRC Press, 1988
- **J G R HURRELL.** Monoclonal Hybridoma Antibodies: Techniques and applications. CRC Press, 1982
- **JENKINS, R.E. ; PENNINGTON, S.R.** *Proteomics,* 2001, vol. 2, 13-29
- **LAL et al.** *Drug Discov Today,* 2002, vol. 7 (18), S143-9
- **STEINMAN et al.** *Ann. Rev. Immunol.,* 1991, vol. 9, 271
- Patch Testing. **DEGROOT, A.** Test Concentrations and Vehicles for 4350 Chemicals. acdegroot publishing, 2008
- **LUECHTEFELD, T. et al.** Analysis of publically available skin sensitization data from REACH registrations 2008-2014. *ALTEX,* 2016, vol. 33 (2), 135-48
- **BOVERHOF, D.R. et al.** Respiratory sensitization and allergy: current research approaches and needs. *Toxicol Appl Pharmacol,* 2008, vol. 226 (1), 1-13
- **THOMAS, W.R. et al.** Characterization and immunobiology of house dust mite allergens. *Int Arch Allergy Immunol,* 2002, vol. 129 (1), 1-18
- **BAUR, X.** Enzymes as occupational and environmental respiratory sensitisers. *Int Arch Occup Environ Health,* 2005, vol. 78 (4), 279-86
- **BAUR, X. ; L.T. BUDNIK ; G. VON KIRCHBACH.** Allergic asthma caused by exposure to bacterial alpha-amylase Termamyl(R). *Am J Ind Med,* 2013, vol. 56 (3), 378-80
- **BUDNIK, L.T. et al.** Sensitising effects of genetically modified enzymes used in flavour, fragrance, detergence and pharmaceutical production: cross-sectional study. *Occup Environ Med,* 2016

- **MCCLAIN, S. et al.** Allergic sensitization: food- and protein-related factors. *Clin Transl Allergy,* 2014, vol. 4 (1), 11
- **NORTH, C.M. et al.** Developing a framework for assessing chemical respiratory sensitization: A workshop report. *Regul Toxicol Pharmacol,* 2016, vol. 80, 295-309
- User's handbook supplement to the guidance document for developing and assessing AOPs. US Army Engineer Research & Development Center, 2014
- **ROGGEN, E.L.** In vitro approaches for detection of chemical sensitization. *Basic Clin Pharmacol Toxicol,* 2014, vol. 115 (1), 32-40
- **JOHANSSON, H. et al.** Genomic allergen rapid detection in-house validation--a proof of concept. *Toxicol Sci,* 2014, vol. 139 (2), 362-70
- **SCHUURHUIS, D.H. et al.** Ins and outs of dendritic cells. *Int Arch Allergy Immunol,* 2006, vol. 140 (1), 53-72
- **OECD.** The Adverse Outcome Pathway for Skin Sensitisation Initiated by Covalent Binding to Proteins. Part 1. *Scientific Evidence,* 2012, 1-59
- **FORRERYD, A. et al.** Prediction of chemical respiratory sensitizers using GARD, a novel in vitro assay based on a genomic biomarker signature. *PLoS One,* 2015, vol. 10 (3), e0118808
- **JOHANSSON, H. et al.** A genomic biomarker signature can predict skin sensitizers using a cell-based in vitro alternative to animal tests. *BMC Genomics,* 2011, vol. 12, 399
- **JOHANSSON, H. et al.** The GARD assay for assessment of chemical skin sensitizers. *Toxicol In Vitro,* 2013, vol. 27 (3), 1163-9
- **PICCOLO, S.R. et al.** A single-sample microarray normalization method to facilitate personalized-medicine workflows. *Genomics,* 2012, vol. 100 (6), 337-44
- **PICCOLO, S.R. et al.** Multiplatform single-sample estimates of transcriptional activation. *Proc Natl Acad Sci USA,* 2013, vol. 110 (44), 17778-83
- **BENJAMINI, Y ; Y. HOCHBERG.** Controlling the False Discovery Rate: A Practical and Powerful Approach to Multiple Testing. *Journal of the Royal Statistical Society. Series B (Methodological),* 1995, vol. 57 (1), 289-300
- **NOBLE, W.S.** What is a support vector machine?. *Nat Biotech,* 2006, vol. 24 (12), 1565-1567
- **CARLSSON, A. et al.** Molecular serum portraits in patients with primary breast cancer predict the development of distant metastases. *Proc Natl Acad Sci U S A,* 2011, vol. 108 (34), 14252-7
- Key Pathway Advisor by Clarivate Analytics. *Formerly the IP & Science business of Thomson Reuters,* 2016, http://ioscience.thomsonreuters.com/product/metacore/
- **CORTES, C ; V. VAPNIK.** Support-Vector Networks. *Machine Learning,* 1995, vol. 20 (3), 273-297
- **KONIG, K. et al.** Cytokine profiles in nasal fluid of patients with seasonal or persistent allergic rhinitis. *Allergy Asthma Clin Immunol,* 2015, vol. 11 (1), 26
- **KING, C. et al.** Dust mite proteolytic allergens induce cytokine release from cultured airway epithelium. *J Immunol,* 1998, vol. 161 (7), 3645-51
- **GOUGH, L. et al.** Proteolytic activity of the house dust mite allergen Der p 1 enhances allergenicity in a mouse inhalation model. *Clin Exp Allergy,* 2003, vol. 33 (8), 1159-63
- **GOUGH, L. et al.** The cysteine protease activity of the major dust mite allergen Der p 1 selectively enhances the immunoglobulin E antibody response. *J Exp Med,* 1999, vol. 190 (12), 1897-902
- **PORTER, P et al.** Link between allergic asthma and airway mucosal infection suggested by proteinase-secreting household fungi. *Mucosal Immunol,* 2009, vol. 2 (6), 504-17
- **CORSINI, E et al.** Role of oxidative stress in chemical allergens induced skin cells activation. *Food Chem Toxicol,* 2013, vol. 61, 74-81
- **TAKAI, T. et al.** TSLP expression induced via Toll-like receptor pathways in human keratinocytes. *Methods Enzymol,* 2014, vol. 535, 371-87
- Breakdown of the Molecular Processes Driving the Adverse Outcome Pathways (AOPs) of Skin and Respiratory Sensitization Induction in Humans Exposed to Xenobiotics and Proteins. **ROGGEN, E.L.** Molecular Immunotoxicology. Wiley-VCH Verlag GmbH & Co. KGaA, 2014
- **ATHER, J.L. et al.** Airway epithelial NF-kappaB activation promotes allergic sensitization to an innocuous inhaled antigen. *Am J Respir Cell Mol Biol,* 2011, vol. 44 (5), 631-8
- **CHIOU, Y.L. ; C.Y. LIN.** Der p2 activates airway smooth muscle cells in a TLR2/MyD88-dependent manner to induce an inflammatory response. *J Cell Physiol,* 2009, vol. 220 (2), 311-8
- **YAZDI, A.S. ; K. GHORESCHI ; M. ROCKEN.** Inflammasome activation in delayed-type hypersensitivity reactions. *J Invest Dermatol,* 2007, vol. 127 (8), 1853-5
- **KOOL, M. et al.** An unexpected role for uric acid as an inducer of T helper 2 cell immunity to inhaled antigens and inflammatory mediator of allergic asthma. *Immunity,* 2011, vol. 34 (4), 527-40
- **BASKETTER, D.A. et al.** Defining occupational and consumer exposure limits for enzyme protein respiratory allergens under REACH. *Toxicology,* 2010, vol. 268 (3), 165-70
- **SARLO, K. et al.** Proteolytic detergent enzymes enhance the allergic antibody responses of guinea pigs to nonproteolytic detergent enzymes in a mixture: implications for occupational exposure. *J Allergy Clin Immunol,* 1997, vol. 100 (4), 480-7

- **SCHEURER, S. ; M. TODA ; S. VIETHS.** What makes an allergen?. *Clin Exp Allergy,* 2015, vol. 45 (7), 1150-61
- **SCHWEIGERT, M.K. ; D.P. MACKENZIE ; K. SARLO.** Occupational asthma and allergy associated with the use of enzymes in the detergent industry--a review of the epidemiology, toxicology and methods of prevention. *Clin Exp Allergy,* 2000, vol. 30 (11), 1511-8
- **CHAPMAN, M.D. ; S. WUNSCHMANN ; A. POMES.** Proteases as Th2 adjuvants. *Curr Allergy Asthma Rep,* 2007, vol. 7 (5), 363-7
- **STRAUBE, F. et al.** Contact allergens and irritants show discrete differences in the activation of human monocyte-derived dendritic cells: consequences for in vitro detection of contact allergens. *Arch Toxicol,* 2005, vol. 79 (1), 37-46
- **PEDERSEN, L.K. et al.** Augmentation of skin response by exposure to a combination of allergens and irritants - a review. *Contact Dermatitis,* 2004, vol. 50 (5), 265-73
- **GRABBE, S. et al.** Dissection of antigenic and irritative effects of epicutaneously applied haptens in mice. Evidence that not the antigenic component but nonspecific proinflammatory effects of haptens determine the concentration-dependent elicitation of allergic contact dermatitis. *J Clin Invest,* 1996, vol. 98 (5), 1158-64
- **MARTIN, S.F.** Adaptation in the innate immune system and heterologous innate immunity. *Cell Mol Life Sci,* 2014, vol. 71 (21), 4115-30
- **PICCOLO, S.R et al.** A single-sample microarray normalization method to facilitate personalized-medicine workflows. *Genomics,* 2012, vol. 100 (6), 337-44